# EUROPEAN PATENT SPECIFICATION

(11) **EP 2 027 264 B1**
(45) Date of publication and mention of the grant of the patent: **29.10.2014**
(21) Application number: 07718958.7
(22) Date of filing: 24.05.2007
(51) Int. Cl.: C12N 15/09, C12N 15/29, C07K 19/00, C12N 15/64

(54) **MULTI-GENE EXPRESSION VEHICLE**
MULTIGEN-EXPRESSIONSVEHIKEL
VÉHICULE D'EXPRESSION MULTIGÉNIQUE

(30) Priority: 25.05.2006 US 803206 P
(43) Date of publication of application: 25.02.2009
(73) Proprietor: Hexima Limited, Melbourne, VIC 3000 (AU)
(72) Inventor: ANDERSON, Marilyn, Anne, Keilor, Victoria 3073 (AU); HEATH, Robyn, Louise, Northcote, Victoria 3070 (AU)
(74) Representative: Dzieglewska, Hanna Eva
(86) International application number: PCT/AU2007/000712
(87) International publication number: WO 2007/137329

(56) References cited:
- WO-A-00/11175
- WO-A1-00/11175
- US-A1- 2003 129 720
- US-B2- 6 806 074
- FRANCOIS LE J A ET AL: "Processing in Arabidopsis thaliana of a heterologous polyprotein resulting in differential targeting of the individual plant defensins" PLANT SCIENCE,, vol. 166, 1 January 2004 (2004-01-01), pages 113-121, XP008110103
- DATABASE UniProt [Online] 1 October 2000 (2000-10-01), "SubName: Full=Putative C2H2-type zinc finger protein; SubName: Full=Putative uncharacterized protein At3g53820; SubName: Full=Zinc finger (C2H2 type) family protein;" XP002560732 retrieved from EBI accession no. UNIPROT:Q9M344 Database accession no. Q9M344
- DATABASE UniProt [Online] 1 May 1999 (1999-05-01), "RecName: Full=Uncharacterized deaminase RP831; EC=<A HREF="http://srs.ebi.ac.uk/srsbin/cgi-bin/ wgetz?[enzyme-ECNumber:3.5.*.*]+-e">3.5.-. -</A>;" XP002560731 retrieved from EBI accession no. UNIPROT:Q9ZCC6 Database accession no. Q9ZCC6
- DATABASE UniProt [Online] 7 December 2004 (2004-12-07), "RecName: Full=Neuritin-B; Flags: Precursor;" XP002560733 retrieved from EBI accession no. UNIPROT:Q5U523 Database accession no. Q5U523
- DASGUPTA S ET AL: "CO-ORDINATED EXPRESSION OF MULTIPLE ENZYMES IN DIFFERENT SUBCELLULAR COMPARTMENTS IN PLANTS" PLANT JOURNAL, BLACKWELL SCIENTIFIC PUBLICATIONS, OXFORD, GB, vol. 16, no. 1, 1 October 1998 (1998-10-01), pages 107-116, XP002927976 ISSN: 0960-7412
- LEE M C S ET AL: "A novel two-chain proteinase inhibitor generated by circularization of a multidomain precursor protein" NATURE STRUCTURAL BIOLOGY,, vol. 6, no. 6, 1 January 1999 (1999-01-01), pages 526-530, XP008110135
- MILLER E A ET AL: "Identification of a novel four-domain member of the proteinase inhibitor II family from the stigmas of Nicotiana alata." PLANT MOLECULAR BIOLOGY JAN 2000, vol. 42, no. 2, January 2000 (2000-01), pages 329-333, XP002559907 ISSN: 0167-4412
- SCANLON M J ET AL: "Structure of a putative ancestral protein encoded by a single sequence repeat from a multidomain proteinase inhibitor gene fromNicotiana alata" STRUCTURE, CURRENT BIOLOGY LTD., PHILADELPHIA, PA, US, vol. 7, no. 7, 15 July 1999 (1999-07-15), pages 793-802, XP004742001 ISSN: 0969-2126
- LIN LI ET AL: "Efficient linking and transfer of multiple genes by a multigene assembly and transformation vector system" PROCEEDINGS OF THE NATIONAL ACADEMY OF SCIENCES OF USA, NATIONAL ACADEMY OF SCIENCE, WASHINGTON, DC, US, vol. 100, no. 10, 13 May 2003 (2003-05-13), pages 5962-5967, XP002323544 ISSN: 0027-8424
- PLUNKETT G ET AL: "Proteinase inhibitors I and II from leaves of wounded tomato plants: Purification and properties" ARCHIVES OF BIOCHEMISTRY AND BIOPHYSICS, ACADEMIC PRESS, US, vol. 213, no. 2, 1 February 1982 (1982-02-01), pages 463-472, XP024758590 ISSN: 0003-9861 [retrieved on 1982-02-01]
- THOMMA B P H J ET AL: "Plant defensins" PLANTA, SPRINGER VERLAG, DE, vol. 216, no. 2, 1 December 2002 (2002-12-01), pages 193-202, XP002404682 ISSN: 0032-0935
- FRANCOIS LE.J.A. ET AL.: 'Processing in Arabidopsis thaliana of a heterologous polyprotein resulting in differential targeting of the individual plant defensins' PLANT SCIENCE vol. 166, 2004, pages 113 - 121, XP008110103
- LEE M.C.S. ET AL.: 'A novel two-chain proteinase inhibitor generated by circularization of a multidomain precursor protein' NATURE STRUCTURAL BIOLOGY vol. 6, no. 6, 1999, pages 526 - 530, XP008110135

## Description

### BACKGROUND OF THE INVENTION

The potato type two inhibitors are a family of serine proteinase inhibitors that are found in many Solanaceous plants. The inhibitors are so named because the first members described were isolated from potato and tomato plants [Bryant, J. et al. (1976) Biochemistry 15:3418-3424; Plunkett, G. et al. (1982) Arch. Biochem. Biophys. 213:463-472]. The inhibitors often consist of two repeated domains each domain of about 6kDa and with a reactive site to either chymotrypsin or trypsin. These two-domain inhibitors are encoded by genes, termed the *Pin2* gene family, which are expressed in tomato fruit and potato tubers, as well as in the leaves of both plants after mechanical wounding or insect damage [Graham, JS, et al. (1985) J. Biol. Chem. 260:6561-6564; Keil, M. et al. (1986) Nuc. Acids Res. 14:5641-5650; Thornberg, RW, et al. (1987) Proc. Natl. Acad. Sci. USA 84:744-748]. Several members of this gene family have been cloned from potato and tomato and most have the same two-domain structure as the original members described [Sanchez-Serrano, J. et al. (1986) Mol. Gen. Genet. 203:15-20; Thornberg *supra].*

The potato type two inhibitors are referred to simply as "type two" inhibitors herein. Type two inhibitors are structurally related proteins that are encoded by a family of genes knows as *Pin2.* At least 11 homologous *Pin2* genes have been found in both mono- and di-cotyledonous plants. *Pin 2* genes can encode either a single 6kDa proteinase inhibitor (PI) domain, two 6kDa PI domains like those that are common in potato and tomato or several highly homologous repeated 6kDa domains that inhibit trypsin or chymotrypsin, often circularly permuted. For a catalog of sequences and discussion of structural relationships, see Barta et al., (2002) Trends in Genetics 18:600-603. Sequences have been complied in a database accessible at http://www.ba.itb.cnr.it/Plant-PIs (see also DeLeo, F. et al., (2002) Nucl. Acid Res. 30:347-348.)

In addition to the two-domain 12kDa inhibitors, potatoes also contain lower levels of a series of single-domain inhibitors of approximately 6kDa [Hass, GM, et al. (1982) Biochemistry 21:752-756] which are identical in sequence to the central portion of the two-domain proteins and are likely to be proteolytic products [Sanchez-Serrano *supra*]. Similar single-domain proteinase inhibitors (PI's) have been isolated from eggplant [Richardson, M. (1979) FEBS. Lett. 104:322-326] and tobacco [Pearce, G. et al. (1993) Plant Physiol. 102:639-644], although it is not known if they are derived from a larger precursor molecule. Both tomato and tobacco contain a gene encoding a three-domain inhibitor [Taylor, BH, et al. (1993) Plant Mol. Biol. 23:10 05-1014; Baladin, R. et al. (1995) Plant Mol. Biol. 27:1197-1204], and a gene encoding a six-domain inhibitor (NaPI-ii) has been isolated from the reproductive tissues of the ornamental tobacco, *Nicotiana alata* [Atkinson, AH, et al. (1993) Plant Cell 5:203-213].

NaPI-ii (SEQ ID NO:1) encodes a 40.3kDa precursor protein that contains six inhibitory domains, two reactive against chymotrypsin and four reactive against trypsin [Atkinson *supra*]. Proteolytic processing of the precursor protein occurs in a linker region between domains resulting in the release of six mature, active inhibitors [Heath, RL, et al. (1995) Eur. J. Biochem. 230:250-257; Lee, MCS, et al. (1999) Nature Struct. Biol. 6:526-530]. In addition to the proteinase inhibitory domains, the precursor also has an N-terminal putative ER signal peptide and a C-terminal non-repeated domain which probably functions as a vacuolar sorting signal [Miller, EA, et al, (1999) Plant Cell 11:1499-1508; Nielsen, KJ, et al. (1996) Biochemistry 35:369-378]. Previously we have shown that immature stigmas express two mRNAs that hybridise to the NaPI-ii cDNA [Atkinson *supra*]. One message of 1.4kb corresponds to the six-domain inhibitor, while a second message of approximately 1.0kb encodes a smaller isoform.

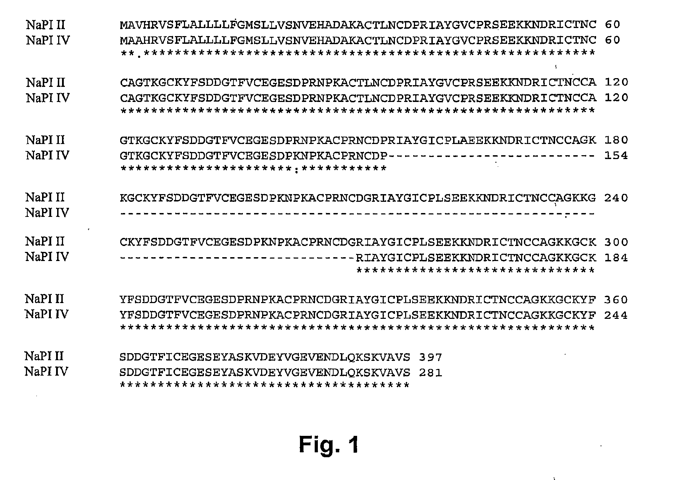

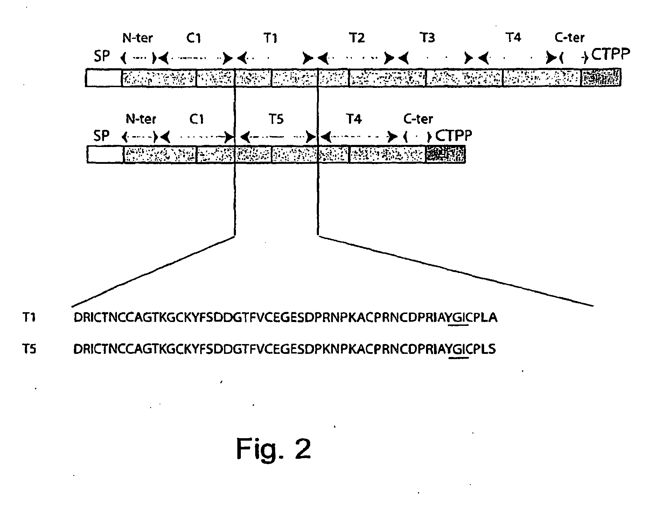

A second type two PI proteinase precursor having four repeated proteinase inhibitor domains has been isolated from *N. alata* stigmas, designated NaPI-iv, [Miller, EA, et al. (2000) Plant Mol. Biol. 42:329-333] (SEQ ID NO:2). The amino acid sequences of NaPI-ii and NaPI-iv align to reveal a high level of identity between the two proteins. (See Fig. 1.) A single amino acid change is present within the predicted signal peptide. A second conservative amino acid change is present within the second repeat, which has been designated T1 in NaPI-ii (SEQ ID NO:3). Therefore the second repeat in NaPI-iv has been designated T5 (SEQ ID NO:4). The relationship between the functional domains of NaPI-ii and NaPI-iv is diagrammed in Fig. 2. A C-terminal non-repeated domain (CTPP) identical in amino acid sequence to that of NaPI-ii is found with NaPI-iv (SEQ ID NO:1, amino acids 374-397, SEQ ID NO:2, amino acids 268-281).

A nucleotide sequence of cDNA encoding NaPI-ii has been disclosed in PCT Publication No. WO 94/138810, SEQ ID NO:1 thereof. The NaPI-iv cDNA sequence SEQ ID NO:2, GenBank Accession No. AF105340, is essentially that of NaPI-ii except for two alterations that result in the two conservative amino acid changes shown in Fig. 1 and several silent changes having no effect on the translated amino acid sequence.

Expression of both NaPI-ii and NaPI-iv results in a protein which is post-translationally processed to yield individual mature 6 kDa proteinase inhibitor (PI) proteins having the designated trypsin (T) or chymotrypsin (C) inhibitory activities. Post-translational glycosylation has not been observed following expression in plant cells. Unprocessed precursor PI's retain the CTPP and are located outside the vacuole of the cell. Once the precursor protein is deposited in the vacuole, the C-terminal domain is rapidly removed and processing that yields individual 6 kDa PI's occurs [Miller (1999) *supra*].

The NaPI-ii precursor PI has been shown to adopt a circular structure by formation of disulfide bonds between the cys residues in the C2_{N} (SEQ ID NO:1 or 2, amino acids 31-53) and C2c (SEQ ID NO:1, amino acids 344-373, SEQ ID NO:2, amino acids 228-257) domains, [Lee (1999) *supra*]. The resulting product of cyclization of the precursor followed by post-translational proteolysis is a unique heterodimeric PI having chymotrypsin-inhibitor activity (C2).

Like other members of the type two family, the *N.alata* Pi's inhibit the digestive proteases of several insect species [Heath, RL, et al. (1997) J. Insect Physiol. 43:833-842] and probably function to limit damage to floral tissues and leaves by insect pests. The PI's significantly retard the growth and development of *Helicoverpa punctigera* larvae when incorporated into artificial diets or expressed in the leaves of transgenic tobacco [Heath (1997) *supra*].

Various strategies have been adopted for expressing more than one transgene in a single transgenic plant. One technique has been to transform individual parent plants each with a single transgene and then to combine the transgenes in a single plant by crossing the parents, [Zhu, Q. et al. (1994) Bio/Technology 12:807-812; Bizily, SP, et al. (2000) Nat. Biotechnol. 18:213-217]. The breeding can be complicated where individual transgenes are recombined at different loci. The method is not applicable for vegetatively propagated plants.

Sequential single gene transformations have been carried out but have limited practical value because of limited availability of selectable markers for each transformation step.

The use of multiple transgenes linked on the same vector each separately controlled by its own copy of the same promoter has resulted in unexpected transcriptional silencing. [Matzke, AJM, et al. (1998) Curr. Opin. Plant Biol. 1:142-148] or non-uniform expression [Van der Elzen, PJM, et al. (1993) Phil. Trans. R. Soc. Land. B 342:271-278]. The use of different individual promoters to drive multiple linked transgenes appears feasible but expression is presumably subject to individual characteristics of each promoter.

Several investigators have reported adaptation of virus systems for expressing a polyprotein followed by specific protease cleavage in cis to release individual proteins. (See, e.g. Marcos, JF, et al. (1994) Plant Mol. Biol. 24:495-503; Beck von Bodman, S. et al. (1995) Bio/technology 13:587-591). The systems require introducing a viral protease to cleave the polyprotein with the possibility of undesired side effects of the introduced protease.

Urwin, PE, et al. (1998) Planta 204:472-479 described a dual proteinase inhibitor construct joined by a protease-sensitive propeptide from *Pisum sativum,* expressed in Arabidopsis. Only partial cleavage of the expressed polyprotein was reported. Using a 20 amino acid long linkage sequence, termed 2A, from foot-and-mouth disease virus, Halpin, C. et al. (1999) Plant J. 17:453-459 described constructing a polyprotein having two reporter coding regions joined by 2A in a single open reading frame. The 2A linker was reported to mediate co-translational cleavage at its own carboxy terminus by an enzyme-independent reaction. Although expression of the polyprotein and cleavage did occur, one of the resulting protein products retained 19 amino acids of the 2A linker and the 20^{th} was attached to the other protein.

A similar result was described by Francois, IEJA, et al. (2002) Plant Physiol. 28:1346-1358, who joined coding regions of two proteins, DmAMPI, a plant defensin from seeds of *Dahlia mercki1* and RsAFP2, a defensin from *Raphanus sativus,* using a propeptide of 16 amino acids from seeds of *Impatiens balsamina.* The propeptide of *I. balsamina* was obtained from a polyprotein precursor, IbAMP, described by Tailor, RA, et al. (1997) J. Biol. Chem. 272:24480-24487. The described polyprotein construct of DmAMPI and RsAFP2 was expressed and post-translationally cleaved in Arabidopsis; however, portions of the linking propeptide were found attached to the C- and N- termini of the linked proteins, regardless of their orientation in the polyprotein construct relative to the linker.

Using a composite linker of 29 amino-acids in length, Francois, I.F.I.A. et al. (2004) Plant Science 166:113-121 reported expression in *Arabidopsis* of DmAMP1 and RsAFP2 as a polyprotein precursor. The precursor was processed to yield DmAMP1 cross-reactive protein primarily in intracellular extracts and RsAFP2 cross-reactive protein primarily In extracellular fluid. The linker sequence was a composite of part of the *I. balsamina* linker and part of the foot-and-mouth disease 2A linker sequence. A recombination-based system for introducing a plurality of genes into a plant cell has been described by Chen, Q.-J., et al. (2006) Plant Mol. Biol. 62:927-936. Each gene has its own promoter and terminator.

### SUMMARY

Described herein is a multi-gene expression vehicle (MGEV) for concurrently expressing a plurality of genes in a plant cell, tissue or whole plant, under control of a single promoter. A MGEV can be constructed to express a linear polyprotein that lacks features necessary to cause the C-terminal and N-terminal ends to join together. The MGEV includes a single isolated polynucleotide whose sequence includes the following segments described by the function encoded by each segment from 2 to 8 open reading frames (D₂₋₈), each of which encodes a functional protein which Is not a type two proteinase inhibitor, and a plurality of linker segments (L₁₋₇), each one situated between two D segments, wherein the D and L segments are in the same reading frame and each linker segment has the sequence of SEQ ID NO: 5.

The MGEV preferably includes, in addition, a 5' terminal segment encoding an endoplasmic reticulum signal sequence (S) and a 3'-terminal segment encoding a C-terminal vacuole targeting peptide (V). Translation of a linear MGEV yields a linear polyprotein which Is further processed by cleavage at the linker (L) segments, to separate the protein domains from one another. Optionally, in its circular form, the MGEV additionally includes segments encoding a first "Clasp" peptide (C2_{N}) on the C-terminal side of S and a second "Clasp" peptide (C2c) on the N-terminal side of V. Preferably, the C2_{N} and C2c proteins have secondary and tertiary structures that allow them to Interact to form a hetero-dimer that can be covalently linked together by post-translational formation of disulfide bonds, thereby forming a "circular" polyprotein (having a cyclic topology). In one embodiment, the cross-linked C2_{N}-C2_{C} dimer has activity as a chymotrypsin inhibitor (C2). The circular MGEV can have from 3-8 reading frames (D₃₋₈) with linkers between each domain and each "clasp" peptide (L₄₋₈). Ultimately, the circular polyprotein is also cleaved at each L segment. In both linear and circular forms, the signal polypeptide (S) and the vacuole targeting peptide (V) function to control intracellular transport of the entire polyprotein, prior to cleavage at L sites.

### BRIEF DESCRIPTION OF THE FIGURES

**Fig. 1** shows an amino acid alignment of NaPI-ii (SEQ ID NO:1) and NaPI-iv (SEQ ID NO:2).
**Fig. 2** is a diagram showing how expression of both NaPI-ii and NaPI-iv results in a precursor protein which is post-translationally processed to yield individual mature 6 kDa proteinase inhibitor proteins (arrowed). The proteins either have trypsin (T) or chymotrypsin (C) inhibitory activity. Amino acid sequences of T1 (SEQ ID NO:3) and T5 (SEQ ID NO:4) are shown. SP, signal peptide; CTPP, C-terminal propeptide; N-ter (C2_{N}) and C-ter (C2c) are the clasp peptides that interact via disulphide bonds to form a two chain proteinase inhibitor (C2) of 6kDa.
**Fig. 3** is a plasmid map of pHEX29 used in Example 1.
   The following abbreviations are used in all plasmid maps herein (Figs. 3, 5, 7, 9, 11, 13, 15, 17, 19, 21, 23, 25, 27, 29, 31 and 33):
   oriV - origin of replication
   ColE1 ori - origin of DNA replication from Colicin E1
   TDNA RB - right hand border of TDNA from *Agrobacterium tumefaciens.*
   Nos promoter - Nopaline synthase promoter from TDNA of *A. tumefaciens.*
   NPTII - neomycin phosphotransferase coding segment.
   Nos terminator- Nopaline synthase terminator from TDNA of A. *tumefaciens.*
   Disrupted lacZ - partial segment of β-galactosidase gene of *Eschericia coli.*
   CaMV 35S promoter - promoter segment of the Cauliflower Mosaic Virus (CaMV) gene encoding CaMV 35S protein.
   Pot1A in MGEV - described herein.
   CaMV 35S terminator - terminator segment of the CaMV gene encoding CaMV 35S protein.
   M13 ori - origin of replication from M13 bacteriophage coat protein.
   TDNA LB - left hand border of TDNA from *A. tumefaciens.*
   Arrows indicate direction of transcription.
   Unless described in detail herein, the abbreviated features are standard components well-known to those of skill in the art and described in standard textbooks. See, e.g., Molecular Cloning (2001) Sambrook J., and Russell, D.W., Cold Spring Harbor Press, Cold Spring Harbor, New York.

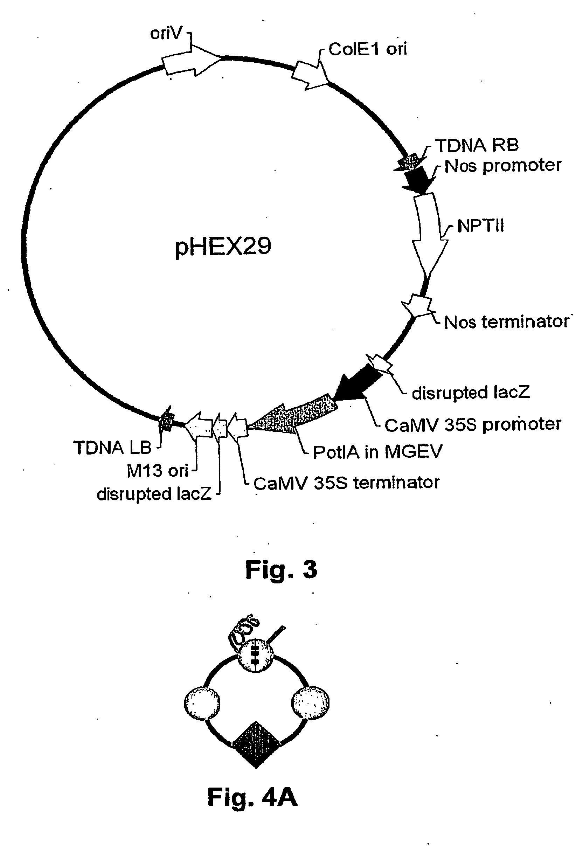

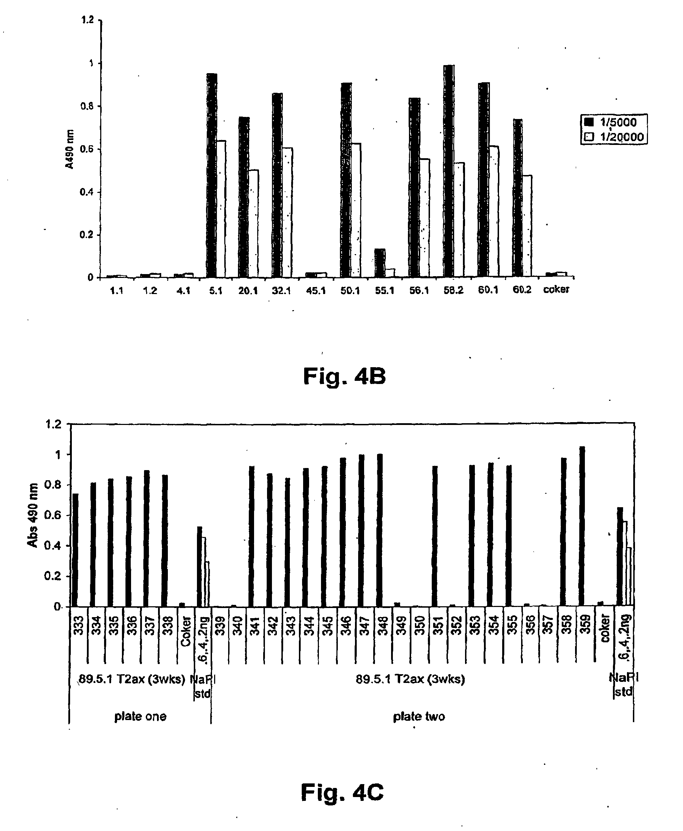

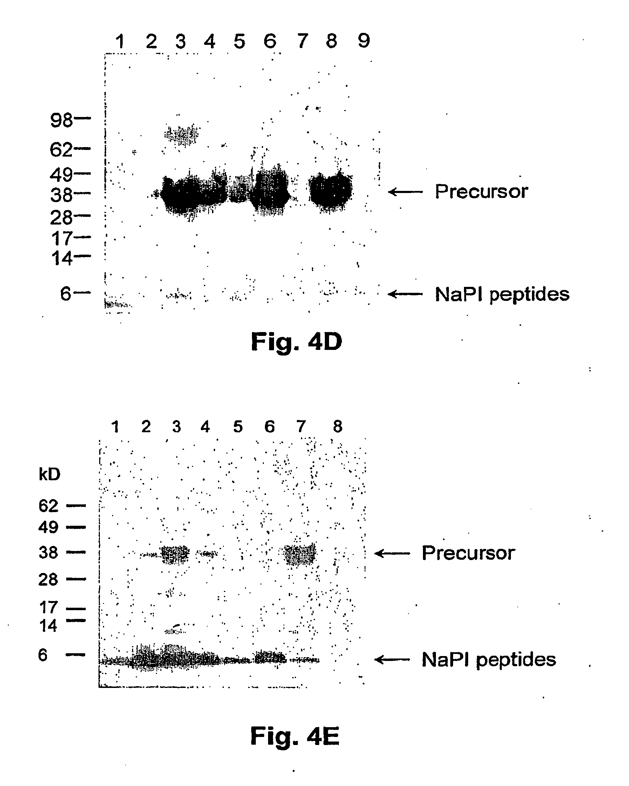

**Figs. 4A-4E** provide data from a 4-domain MGEV for expression of two NaPIs and PotIA in cotton, as described in Example 1. Figure 4A is a diagram of the circular protein encoded by MGEV-5 and expressed in pHEX29 which has an endoplasmic reticulum signal sequence (stick), two 6kDa proteinase inhibitor domains (spheres), one PotIA domain (diamond) and a vacuolar targeting sequence (helix). A third proteinase inhibitor domain is represented by a sphere with 3 horizontal lines to illustrate the 3 disulphide bonds that link the two peptides [N-ter (C2_{N}) and C-ter (C2c)], that form the clasp. A linker peptide is indicated by a solid line connecting each protein domain. The predicted size of the unprocessed MGEV-5 product is 31.4 KDa minus the signal sequence. Figure 4B is a bar graph of data from ELISA detection of NaPIs in extracts from leaves of primary transgenic cotton lines from experiment CT89. Samples were diluted 1:5,000 and 1:20,000. Coker is a non-transgenic control. Figure 4C is a bar graph of data from ELISA detection of NaPIs in extracts from leaves of T2 plants of line 89.5.1. Samples were diluted 1:5,000. Coker is a non-transgenic control. NaPI standard is 2, 4 or 6 µg of pure 6kDa NaPIs isolated from *Nicotiana alata* flowers. Figure 4D is a protein blot of leaf extracts prepared from primary transgenic cotton lines (T1) from experiments CT89 and CT90. Leaf proteins were extracted directly into NuPAGE LDS sample buffer (4x) (NOVEX), separated on a 4-12% Novex Bis-Tris SDS gel and transferred onto a 0.22 micron nitrocellulose membrane. The blot was probed with NaPI antibody. The precursor protein and 6kDa NaPI peptides are arrowed. Lane 1: 80 ng of purified NaPI, lane 2: 89.5, lane 3: 89.20, lane 4: 89.60, lane 5: 89.111, lane 6: 89.120, lane 7: 89.122, lane 8: 90.131, lane 9: untransformed Coker. Figure 4E is an immunoblot blot of extracts prepared from cotton leaves of T1 and T2 plants from selected lines from experiments CT89 and CT90. Proteins were precipitated with acetone prior to solubilisation in sample buffer, separated on a 4-12% Novex Bis-Tris SDS gel and transferred onto a 0.22 micron nitrocellulose membrane. The blot was probed with NaPI antibody. The precursor protein and 6kDa NaPI peptides (arrowed) were observed in both the primary lines (T1) and their progeny (T2). Processing intermediates can be observed in lanes 3 and 7. Lane 1: 150 ng purified NaPI, lane 2: 89.177 (T1), lane 3: 89.177 (T2), lane 4: 90.73 (T1), lane 5: 90.73 (T2), lane 6: 89.5 (T1), lane 7: 89.5 (T2), lane 8: untransformed Coker.

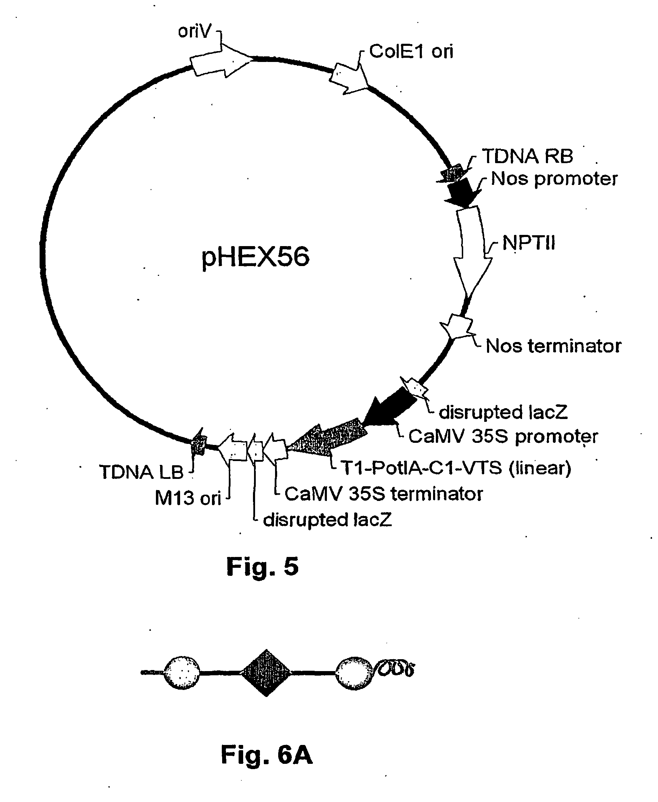

**Fig. 5** is a plasmid map of pHEX56 used in Example 2.

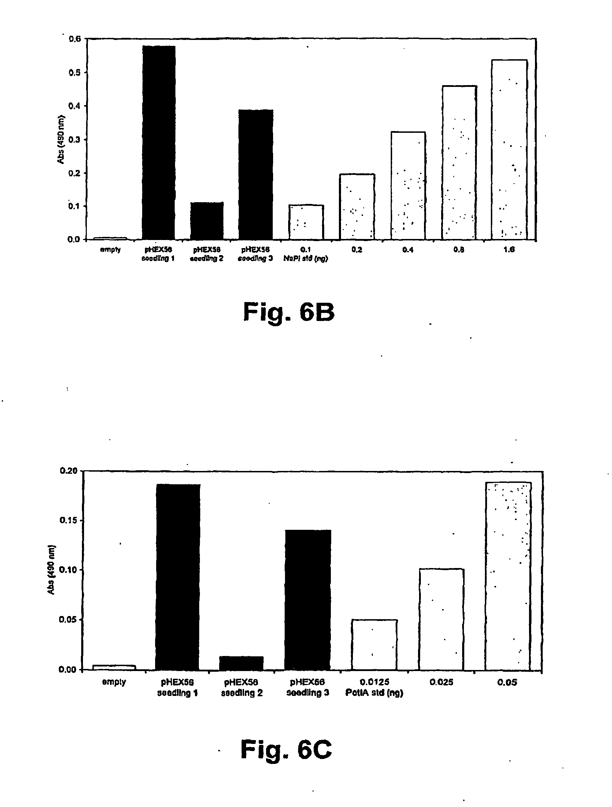

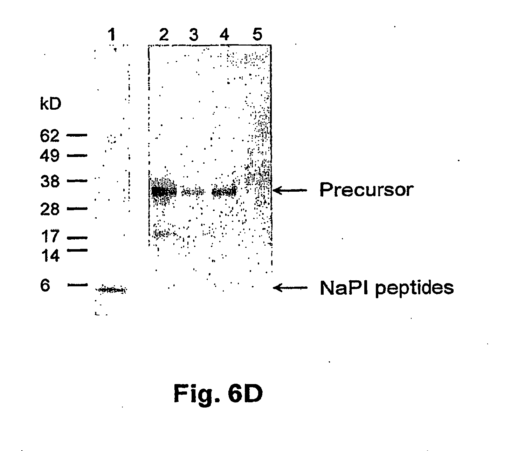

**Figs. 6A-6D** provide data based on use of a 3-domain linear MGEV for expression of NaPI and PotIA in cotton cotyledons, as described in Example 2. Fig. 6A is a diagram of the linear protein encoded by MGEV-8 and expressed in pHEX56 which has an endoplasmic reticulum signal sequence (stick), two 6kDa proteinase inhibitor domains (spheres), one PotIA domain (diamond) and a vacuolar targeting sequence (helix). A linker peptide is indicated by a solid line connecting each protein domain. The predicted size of the unprocessed MGEV-8 product is 25.4 kDa minus the signal sequence. Fig. 6B Is a bar graph of data from ELISA detection of NaPIs in extracts from cotton cotyledons after transient expression with pHEX56 or PBIN19 empty vector. Samples were diluted 1:1,000 and compared to various amounts of purified 6kDa NaPIs. Fig. 6C is a bar graph of data from ELISA detection of PotIA in extracts from cotton cotyledons after transient expression with pHEX56. Samples were diluted 1:20 and compared to purified Pot1A standards. Fig 6D is a protein blot of extracts prepared from cotton cotyledons after transient expression with pHEX56. Proteins were precipitated with acetone prior to solubilisation in sample buffer, separated on a 4-12% Novex Bis-Tris SDS gel and transferred onto a 0.22 micron nitrocellulose membrane. The blot was probed with NaPI antibody. Lane 1: 150 ng of purified NaPI, lane 2: seedling 1, lane 3: seedling 2, lane 4: seedling 3, lane 5: cotyledon sample transfected with pBIN19 empty vector. The precursor protein and 6kDa NaPI peptides (arrowed) were detected in all three seedlings infiltrated with *Agrobacterium* containing the pHEX56 construct.

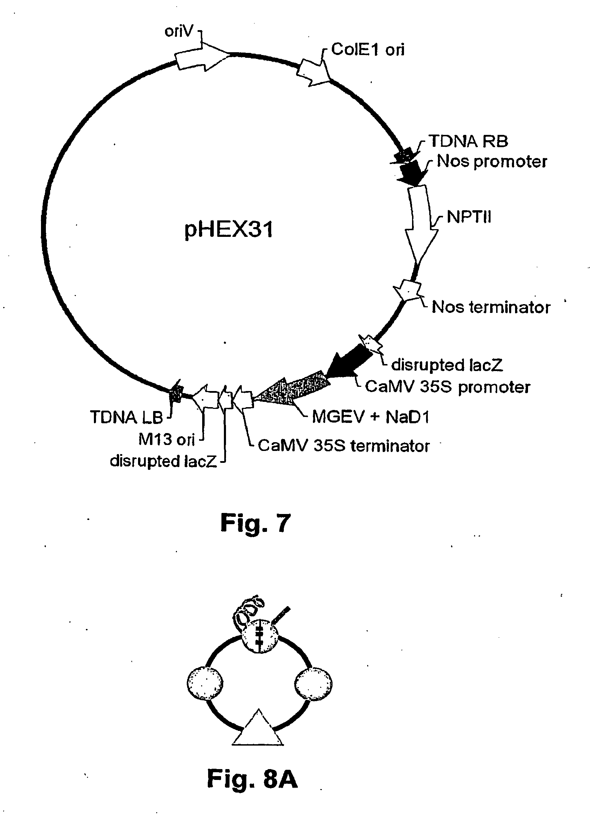

**Fig. 7** is a plasmid map of pHEX31 used in Example 3.

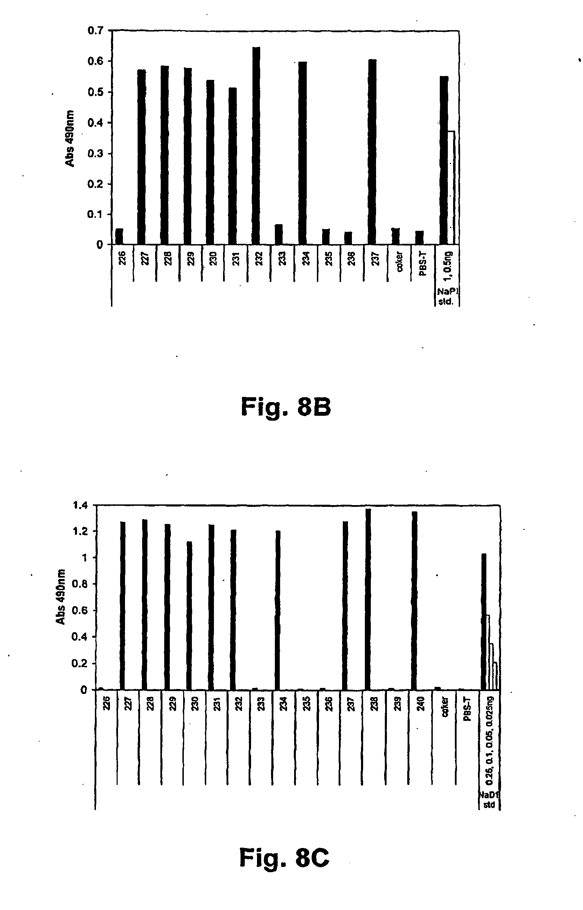

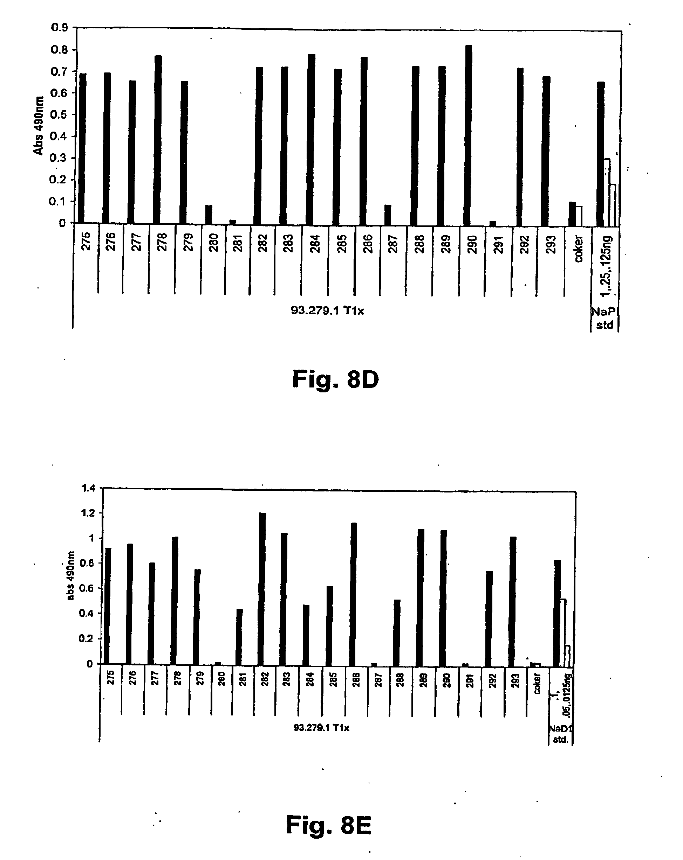

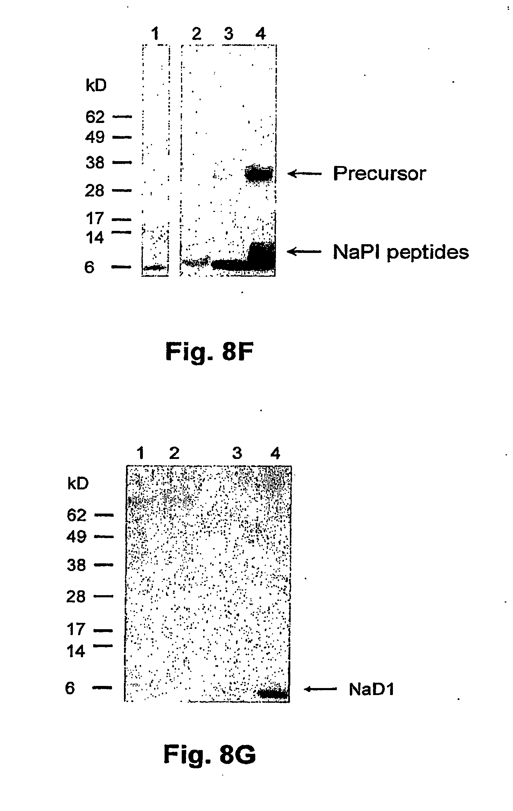

**Figs. 8A-8G** provide data based on use of a 4-domain MGEV for expression of NaPI and mature NaD1 in cotton, as described in Example 3. Fig. 8A is a diagram of the circular protein encoded by MGEV-6 and expressed in pHEX31 which has an endoplasmic reticulum signal sequence (stick), two 6kDa proteinase inhibitor domains (spheres), one NaD1 domain (triangle) and a vacuolar targeting sequence (helix). A third proteinase inhibitor domain is represented by a sphere with 3 horizontal lines to illustrate the 3 disulphide bonds that link the two peptides [N-ter (C2_{N}) and C-ter (C2c)] that form the clasp. A linker peptide is indicated by a solid line connecting each protein domain. The predicted size of the unprocessed MGEV-6 product is 28.2 kDa minus the signal sequence. Fig. 8B is a bar graph of data from ELISA detection of NaPIs in extracts from leaves of T2 plants of line 93.4. Samples were diluted 1:5,000. Coker is a non-transgenic control. PBS-T is a negative control. NaPI standard is the positive control of purified 6kDa NaPI. Fig. 8C is a bar graph of data from ELISA detection of NAD1 in extracts from leaves of T2 plants of line 93.4 Samples were diluted 1:50. Fig. 8D is a bar graph of data from ELISA detection of NaPIs in extracts from leaves of T2 plants of line 93.279 Samples were diluted 1:1,000. Fig. 8E is a bar graph of data from ELISA detection of NAD1 in extracts from leaves of T2 plants of line 93.279. Samples were diluted 1:50. Fig. 8F is a protein blot of extracts prepared from cotton leaves of transgenic cotton lines (T1 and T2) from experiment CT93. Proteins were separated on a 4-12% Novex Bis-Tris SDS gel and transferred onto a 0.22 micron nitrocellulose membrane. The blot was probed with NaPI antibody. Lane 1: 150 ng purified NaPI, lane 2: 93.4.1 T2, lane 3: 93.36.2 T1, lane 4: 93.36.2 T2. Both the precursor protein and 6kDa NaPI peptides (arrowed) were present. Fig. 8G is a protein blot of extracts prepared from cotton leaves of transgenic cotton lines (T1 and T2) from experiment CT93. Proteins were separated on a 4-12% Novex Bis-Tris SDS gel and transferred onto a 0.22 micron nitrocellulose membrane. The blot was probed with NaD1 antibody. Lanes 1 and 2: 93.4.1, lane 3: 50 ng mature NaD1, lane 4: 150 ng mature NaD1. NaD1 is arrowed. A faint band of about 6 kDa was observed in lanes 1 and 2 confirming that the mature NaD1 was present in transgenic line 93.4.1 and had been processed correctly.

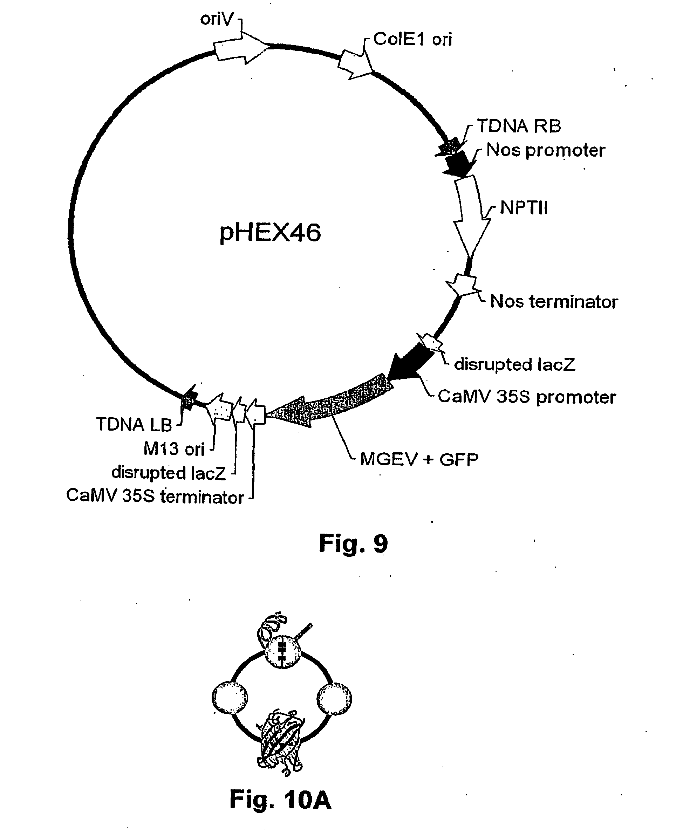

**Fig. 9** is a plasmid map of pHEX46 used in Example 4.

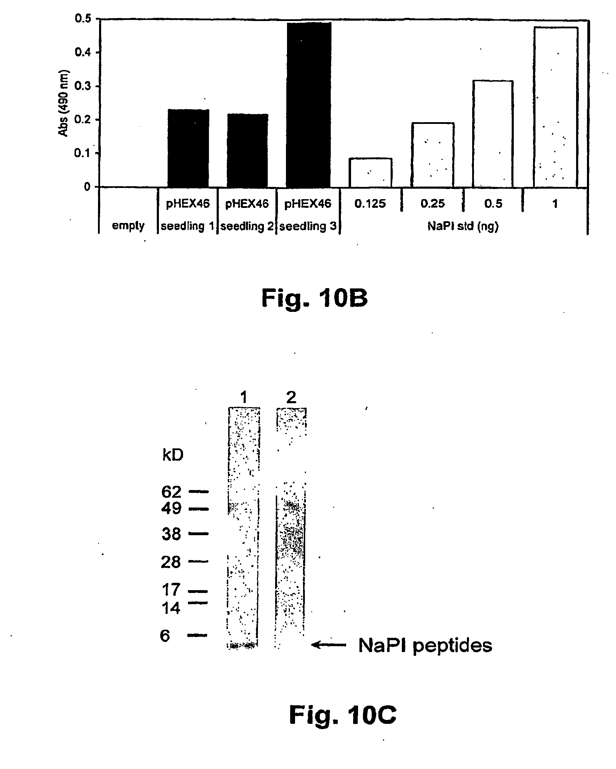

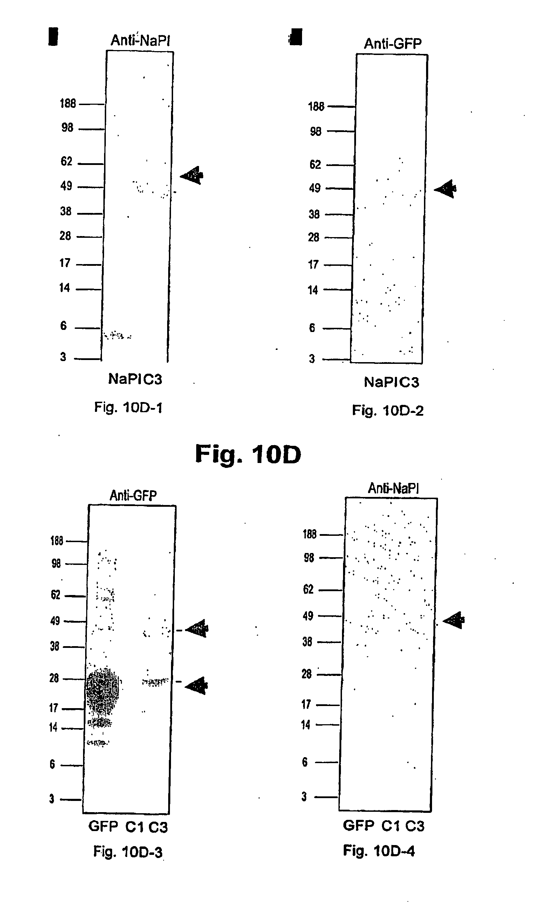

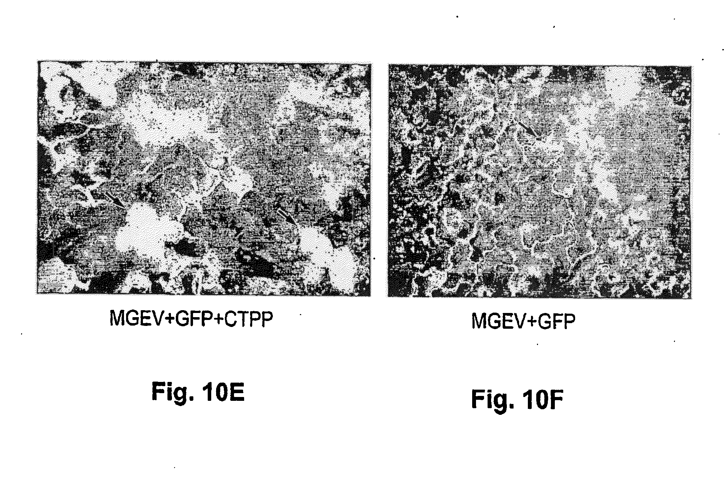

**Figs. 10A-10F** provide data based on use of the MGEV for expression and targeting of GFP to the vacuole in cotton cotyledons and *Nicotiana tabacum* leaves, as described in Example 4. Fig. 10A is a diagram of the circular protein encoded by MGEV-7 and expressed in pHEX46 which has an endoplasmic reticulum signal sequence (stick), three 6kDa proteinase inhibitor domains (spheres), GFP (cylinder) and a vacuolar targeting sequence (helix). A linker peptide is indicated by a solid line connecting each protein domain. The predicted size of the unprocessed MGEV-7 product is 49.6 kDa minus the signal sequence. Fig. 10B is a bar graph of data from ELISA detection of NaPIs in extracts from cotton cotyledons after transient expression with pHEX46 or BIN19 empty vector. Samples were diluted 1:1,000 and compared to purified 6kDa standards. Fig. 10C is a protein blot of extracts prepared from cotton cotyledons after transient expression with pHEX46. Proteins were precipitated with acetone prior to solubilisation in sample buffer, separated on a 4-12% Novex Bis-Tris SDS gel and transferred onto a 0.22 micron nitrocellulose membrane. The blot was probed with NaPI antibody. Lane 1: cotyledon sample transfected with pHEX46, lane 2: cotyledon sample transfected with pBIN19 empty vector. The 6kDa NaPI peptides (arrowed) were present in the cotyledon sample transfected with pHEX46. Fig. 10D shows protein blot of extracts prepared from *Nicotiana benthamiana* leaves after transient expression with pHEX46 (MGEV-7). Fig. 10D-1 and Fig. 10D-2 are the same protein blot containing 6kD NaPIs purified from *N. alata* flowers in lane 1 (NaPI) and an extract from *N*. *benthamiana* leaves after transient expression of pHEX46 (MGEV-7) in the second lane. Fig. 10D-1. NaPI antibodies bound to the 6kDa PIs in lane 1 and to a protein of the expected size for MGEV-7 (∼50kDa, arrowed) in the leaf extracts. Fig. 10D-2 is the blot from Fig. 10D-1 after stripping and reprobing with the GFP antibody. The GFP antibody did not bind to the 6kDa Pis but did bind to the protein of the expected size of MGEV-7 (∼50kDa, arrowed). Thus the ∼50kDa protein (arrowed) has both 6kDa PI domains and a GFP domain. Fig. 10D-3 and Fig. 10D-4 are a second protein blot that was probed with GFP antibodies (Fig. 10D-3) before it was stripped and reprobed with NaPI antibody (Fig. 10D-4). The blot has bacterially expressed GFP in lane one and an extract from *N. benthamiana* leaves after transient expression of pHEX46 (MGEV-7) in the second lane. The GFP antibody bound to the bacterially expressed GFP (28kDa, arrowed) and to a protein of the same size in extracts from leaves expressing MGEV-7. It also bound to a protein of the expected size of the unprocessed MGEV-7 as well as a potential processing intermediate of about 34kDa. The NaPI antibody (Fig. 10D-4) bound to the ∼50kDa protein (arrowed) in leaf extracts confirming that this protein has both NaPI and GFP domains as expected for unprocessed MGEV-7 (∼50kDa, arrowed). The NaPI antibody did not bind to the 28kDa protein in leaf extracts that was highlighted by the GFP antibody. This is consistent with release of free GFP from the MGEV in the leaves of *N. benthamiana.* Fig. 10E Is a micrograph showing transient expression of GFP from pHEX46 in the epidermal cells of cotton leaves. The GFP fluorescence is located in the vacuoles (arrowed). GFP fluorescence examined with an Olympus BX50 fluorescence microscope. Fig. 10F is a micrograph showing transient expression of GFP from pHEX45 in the epidermal cells of cotton leaves. The GFP fluorescence is extracellular (arrowed). GFP fluorescence examined with an Olympus BX50 fluorescence microscope.

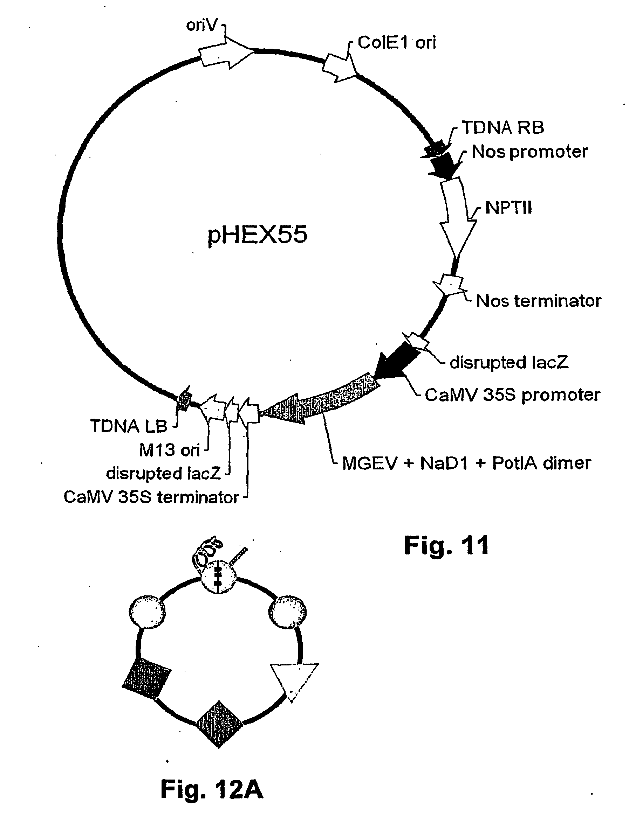

**Fig. 11** **is** a plasmid map of pHEX55 used in Example 5.

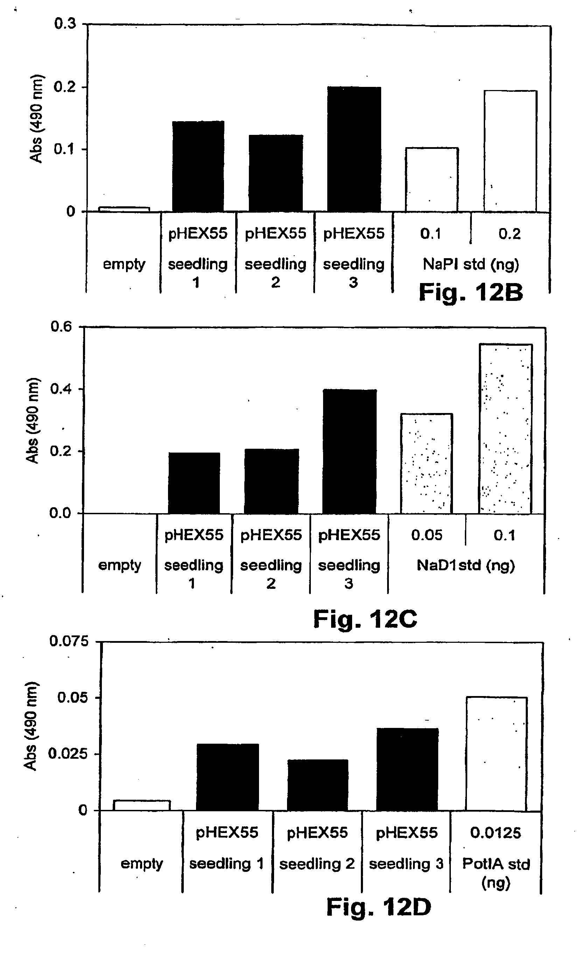

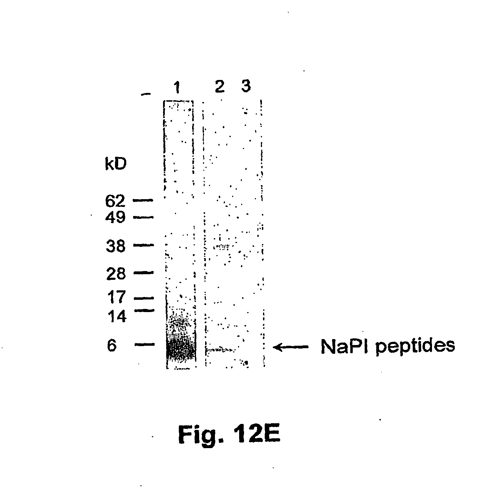

**Figs. 12A-12E** provide data based on use of a 6-domain MGEV for the expression of NaPI, NaD1 and Pot 1A in cotton cotyledons, as described in Example 5. Fig. 12A is a diagram of the circular protein encoded by MGEV-9 and expressed in pHEX55 which has an endoplasmic reticulum signal sequence (stick), two 6kDa proteinase inhibitor domains (spheres), two Pot1A domains (diamonds), one NaD1 domain (triangle) and a vacuolar targeting sequence (helix). A third proteinase inhibitor domain is represented by a sphere with 3 horizontal lines to illustrate the 3 disulphide bonds that link the two peptides [N-ter (C2_{N}) and C-ter (C2c)] that form the clasp. A linker peptide is indicated by a solid line connecting each protein domain. The predicted size of the unprocessed MGEV-9 product is 46.6 kDa minus the signal sequence. Fig. 12B is a bar graph of data from ELISA detection of NaPIs in extracts from cotton cotyledons after transient expression with pHEX55 or pBIN19 empty vector. Samples were diluted 1:1,000. Fig. 12C is a bar graph of data from ELISA detection of NaD1 in extracts from cotton cotyledons after transient expression with pHEX55 or pBIN19 empty vector. Samples were diluted 1:100. Fig. 12D is a bar graph of data from ELISA detection of Pot 1A in extracts from cotton cotyledons after transient expression with pHEX55 or pBIN19 empty vector. Samples were diluted 1:20. Fig. 12E is a protein blot of extracts prepared from cotton cotyledons after transient expression with pHEX55. Proteins were precipitated with acetone prior to solubilisation in sample buffer, separated on a 4-12% Novex Bis-Tris SDS gel and transferred onto a 0.22 micron nitrocellulose membrane. The blot was probed with NaPI antibody. Lane 1: 400 ng purified NaPI, lane 2: cotyledon sample transfected with pHEX55, lane 3: untransformed Coker. The 6kDa NaPI peptides (arrowed) were present in the cotyledon sample transfected with pHEX55. Several processing intermediates ranging from about 17 kDa to 38 kDa were also detected.

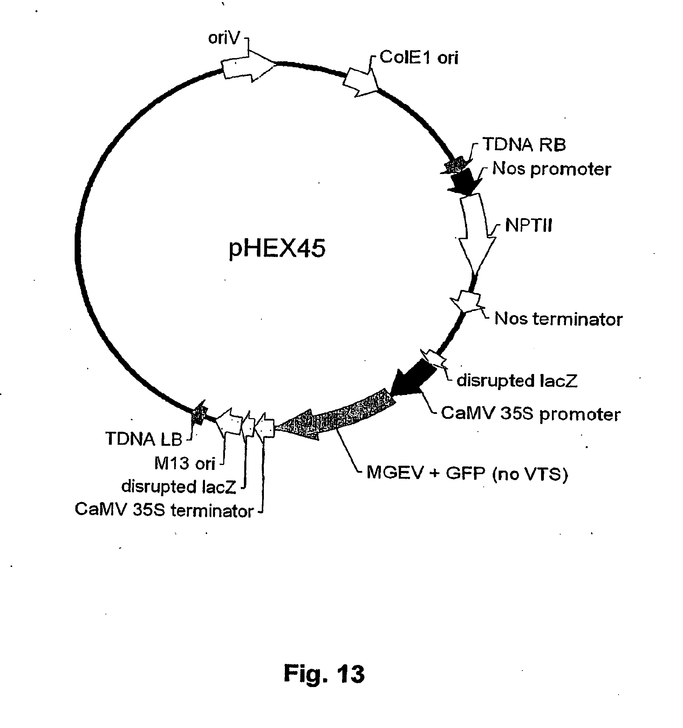

**Fig. 13** is a plasmid map of pHEX45 used in Example 6.

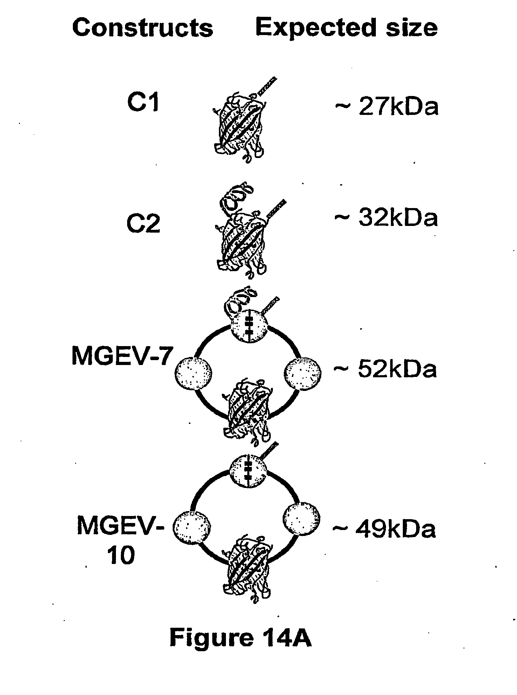

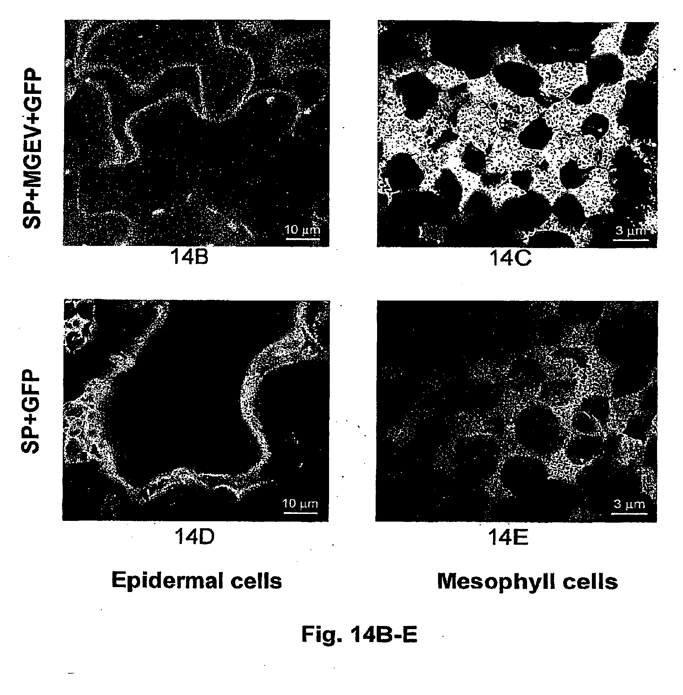

**Figs. 14A-14F** provide data based on use of the MGEV for expression and targeting of GFP to the extracellular space in *Nicotiana benthamiana* leaves, as described in Example 6. Fig. 14A is a diagram of each of the proteins encoded by the four constructs. The endoplasmic reticulum signal sequences are represented by a stick, the 6kDa proteinase inhibitor domains including the clasp domain are spheres, GFP is a cylinder and the vacuolar targeting sequence (V) is represented as a helix. A linker peptide is indicated by a solid line connecting each protein domain. The predicted size of the unprocessed encoded proteins minus the signal sequence is given next to the cartoons. Figs. 14B,-E are micrographs showing transient expression of GFP from pHEX45 (MGEV 10) and C1 (Fig 14A). In the absence of V the GFP from both constructs is directed outside the cell. GFP fluorescence was examined using a Leica TCS SP2 confocal laser-microscope.
   **Fig. 14B** **-** Transient expression of pHEX45 in epidermal cells.
   **Fig. 14C** **-** Transient expression of pHEX45 in mesophyll cells.
   **Fig. 14D** **-** Transient expression of control gene construct C1 in epidermal cells.
   **Fig. 14E** **-** Transient expression of control gene construct C1 in mesophyll cells.

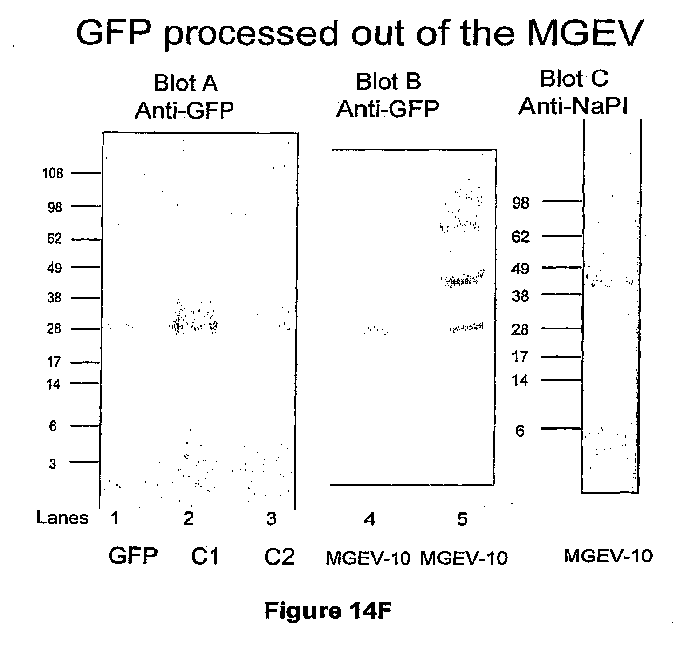

   **Fig. 14F** **-** Protein blots of extracts prepared from *Nicotiana benthamiana* leaves after transient expression with pHEX45 (MGEV-10), pHEX46 (MGEV-7), C1 (S-GFP) and C2 (S-GFP-V). Blots A and B are probed with GFP antibody. Lane 1. Positive control. Bacterially expressed GFP. Lanes 2-5 are extracts from leaves after transient expression of C1, C2, pHEX 46 (MGEV-7) and pHEX45 (MGEV-10) respectively. All constructs produced a protein of 28kDa that bound the GFP antibody. pHEX 46 (MGEV-7) and pHEX45 (MGEV-10) also produced a protein of about 50kDa that was the expected size of MGEV-7 and MGEV-10 that reacted with the GFP-antibody. The ∼50kDa protein corresponding to MGEV-10 also bound the NaPI antibody, Blot C, showing that this protein has both NaPI and GFP domains.

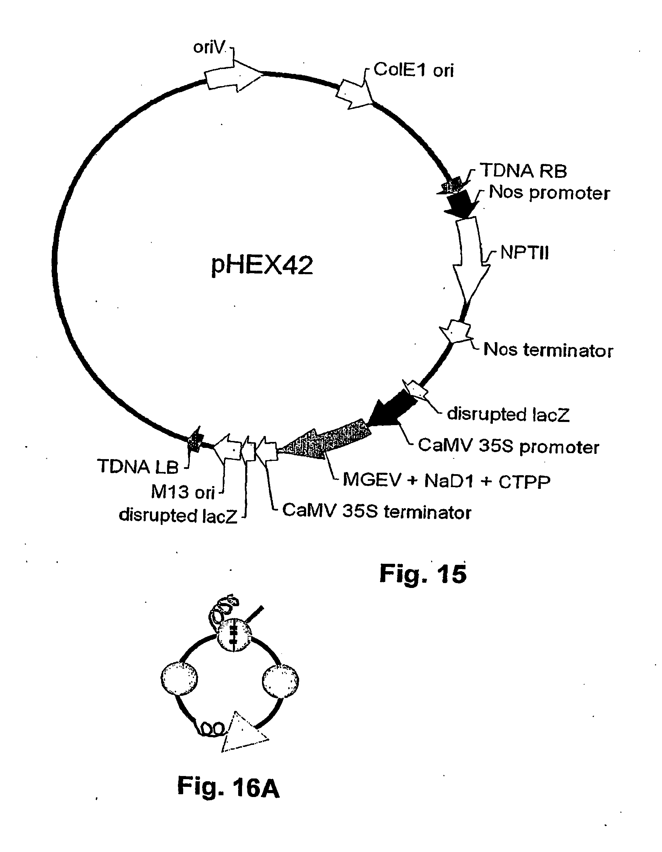

**Fig. 15** is a plasmid map of pHEX42 used in Example 7.

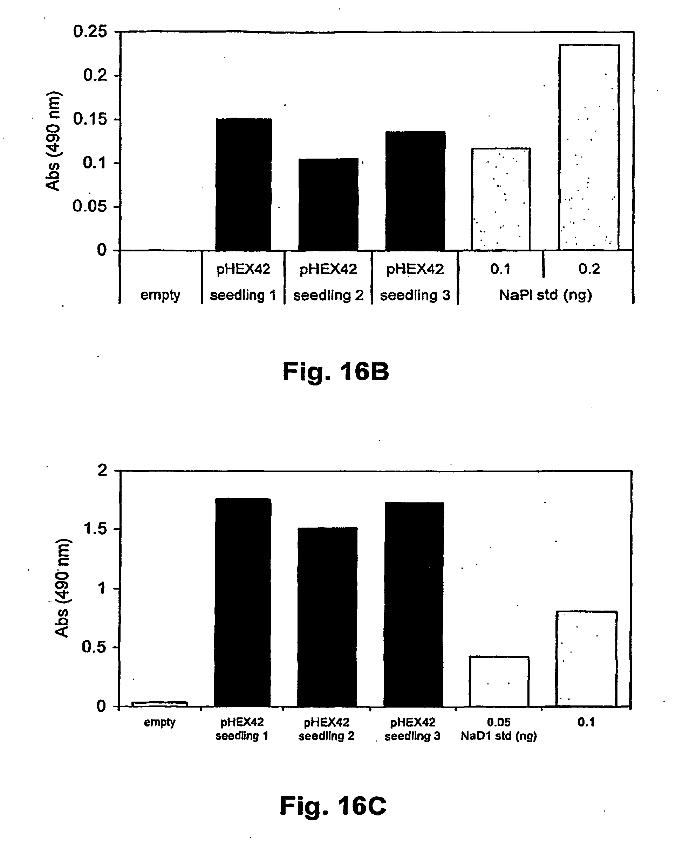

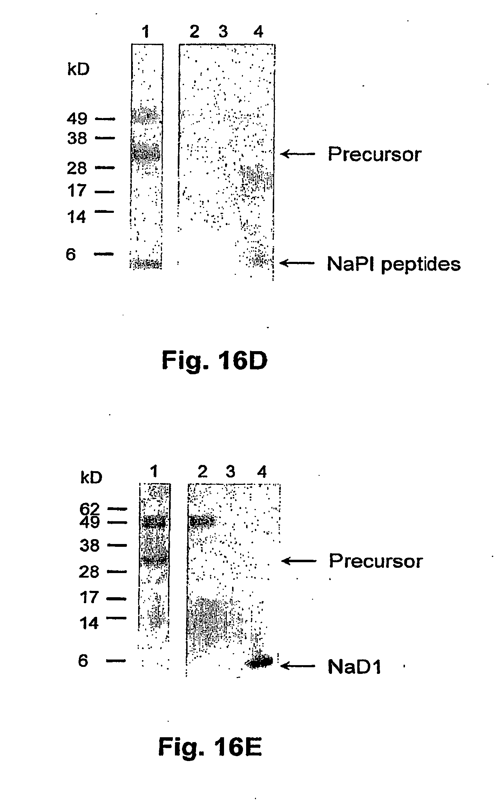

**Figs. 16A-16E** provide data based on use of a 4-domain MGEV for expression of NaPI and NaD1 (with CTPP) in cotton cotyledons, as described in Example 7. Fig. 16A is a diagram of the circular protein encoded by MGEV-11 and expressed in pHEX42 which has an endoplasmic reticulum signal sequence (stick), three 6kDa proteinase inhibitor domains (spheres), one NaD1 domain (triangle) + CTPP tail (helix) and a vacuolar targeting sequence (helix). A linker peptide is indicated by a solid line connecting each protein domain. The predicted size of the unprocessed MGEV-11 product is 31.8 kDa minus the signal sequence. Fig. 16B is a bar graph of data from ELISA detection of NaPI in extracts from cotton cotyledons after transient expression with pHEX42 or empty vector. Samples were diluted 1:100. Fig. 16C is a bar graph of data from ELISA detection of NaD1 in extracts from cotton cotyledons after transient expression with pHEX42. Samples were diluted 1:5,000. Fig. 16D is a protein blot of extracts prepared from cotton cotyledons after transient expression with pHEX42. Proteins were precipitated with acetone prior to solubilisation in sample buffer, separated on a 4-12% Novex Bis-Tris SDS gel and transferred onto a 0.22 micron nitrocellulose membrane. The blot was probed with NaPI antibody. Lane 1: cotyledon sample transfected with pHEX42, lane 2: cotyledon sample transfected with pBIN19 empty vector, lane 3: blank, lane 4: 200 ng purified NaPI. The precursor and 6kDa NaPI peptides (arrowed) were present in the cotyledon sample transfected with pHEX42. Fig. 16E is a protein blot of extracts prepared from cotton cotyledons after transient expression with pHEX42. Proteins were precipitated with acetone prior to solubilisation in sample buffer, separated on a 10 - 20% Novex Tricine SDS gel and transferred onto a 0.22 micron nitrocellulose membrane. The blot was probed with NaD1 antibody. Lane 1: cotyledon sample transfected with pHEX42, lane 2: cotyledon sample transfected with pBIN19 empty vector, lane 3: blank, lane 4: 150 ng purified NaD1. The precursor and 6kDa NaD1 (arrowed) were present in the cotyledon sample transfected with pHEX42.

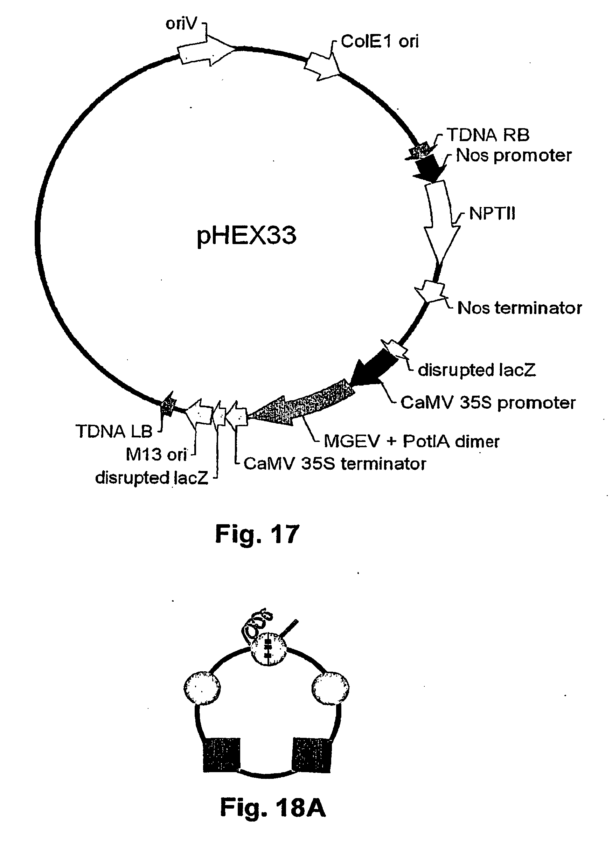

**Fig. 17** is a plasmid map of pHEX33 used in Example 8.

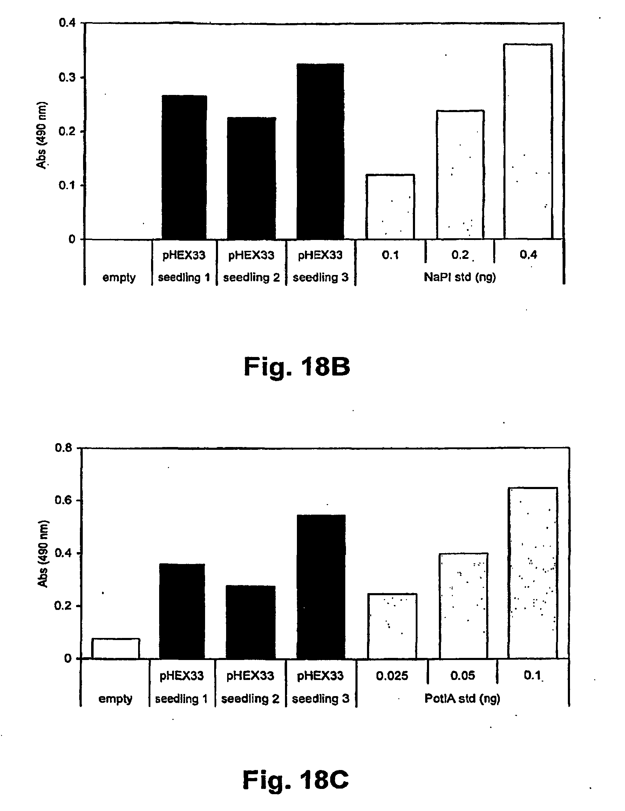

**Figs. 18A-18C** provide data based on use of a 5-domain MGEV for expression of NaPI and PotIA in cotton cotyledons. Fig. 18A has a diagram of the circular protein MGEV-12 encoded by pHEX33 which has an endoplasmic reticulum signal sequence (stick), three 6kDa proteinase inhibitor domains (spheres), two PotIA domains (diamond) and a vacuolar targeting sequence (helix). A linker peptide is indicated by a solid line connecting each protein domain. The predicted size of the unprocessed MGEV-12 product is 40.4 kDa minus the signal sequence. Fig. 18B is a bar graph of data from ELISA detection of NaPIs in extracts from cotton cotyledons after transient expression with pHEX33. Samples were diluted 1:1,000. Fig. 18C is a bar graph of data from ELISA detection of Pot 1A in extracts from cotton cotyledons after transient expression with pHEX33. Samples were diluted 1:20.

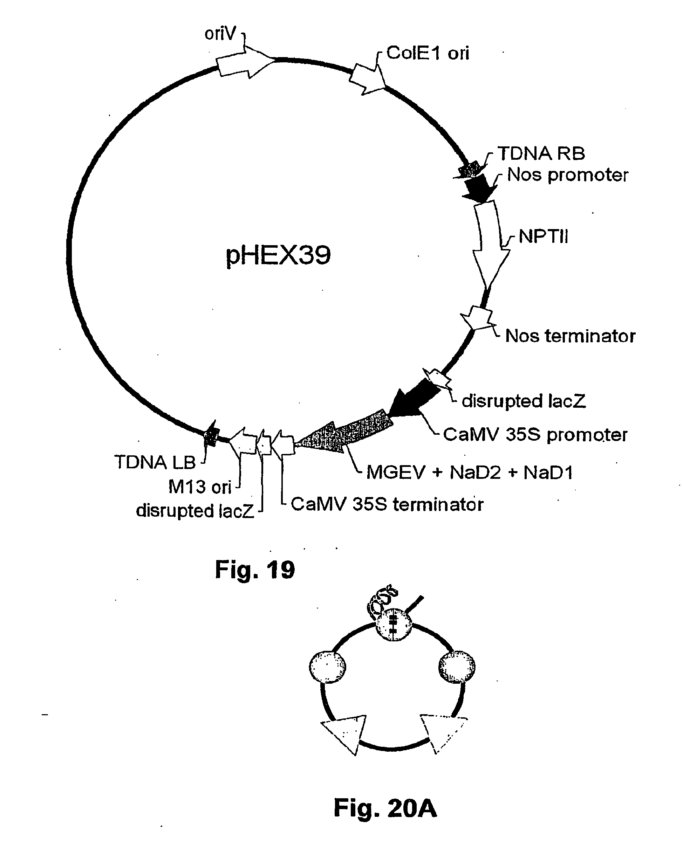

**Fig. 19** is a plasmid map of pHEX39 used in Example 9.

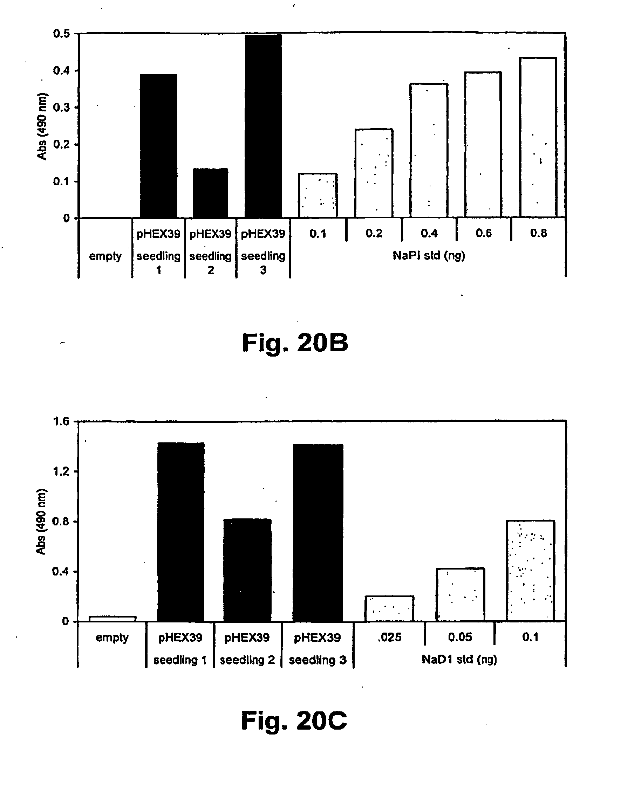

**Figs. 20A-20C** provide data based on use of a 5-domain MGEV for expression of NaPI, mature NaD1 and NaD2 in cotton cotyledons. Fig. 20A is a diagram of the circular protein MGEV-13 encoded by pHEX39 which has an endoplasmic reticulum signal sequence (stick), three 6kDa proteinase inhibitor domains (spheres), one NaD2 domain (triangle), one NaD1 domain (triangle) and a vacuolar targeting sequence (helix). A linker peptide is indicated by a solid fine connecting each protein domain. The predicted size of the unprocessed MGEV-13 product is 34 kDa minus the signal sequence. Fig. 20B is a bar graph of data from ELISA detection of NaPIs in extracts from cotton cotyledons after transient expression with pHEX39. Samples were diluted 1:1,000. Fig. 20C is a bar graph of data from ELISA detection of NaD1 in extracts from cotton cotyledons after transient expression with pHEX39. Samples were diluted 1:100.

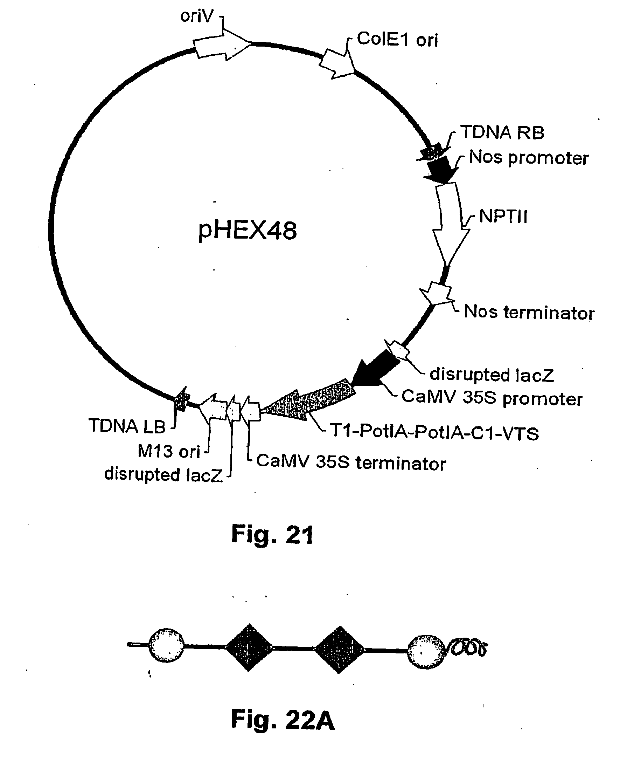

**Fig. 21** is a plasmid map of pHEX48 used in Example 10.

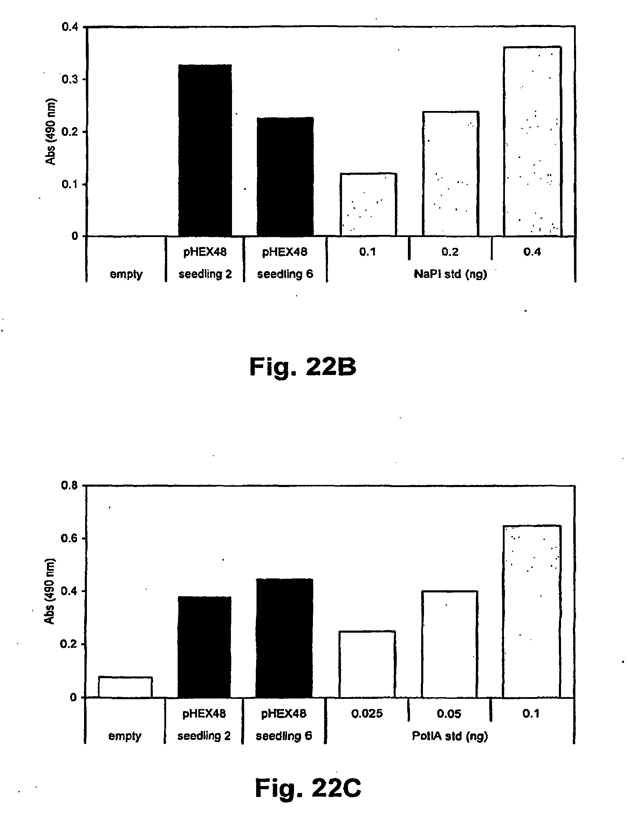

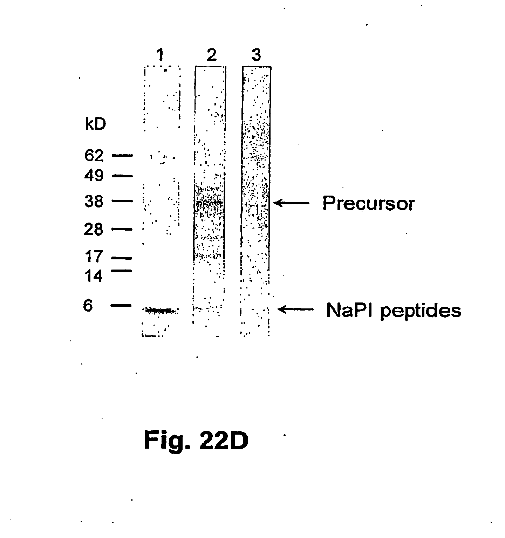

**Figs. 22A-22D** provide data based on use of a 4-domain linear MGEV for expression of NaPI and PotIA in cotton cotyledons. Fig. 22A is a diagram of the linear protein encoded by MGEV-14 and expressed in pHEX48 which has an endoplasmic reticulum signal sequence (stick), two 6kDa proteinase inhibitor domains (spheres), two PotIA domains (diamond) and a vacuolar targeting sequence (helix). A linker peptide is indicated by a solid line connecting each protein domain. The predicted size of the unprocessed MGEV-14 product is 34.5 kDa minus the signal sequence. Fig. 22B is a bar graph of data from ELISA detection of NaPIs in extracts from cotton cotyledons after transient expression with pHEX48. Samples were diluted 1:1,000. Fig. 22C is a bar graph of data from ELISA detection of Pot 1A in extracts from cotton cotyledons after transient expression with pHEX48. Samples were diluted 1:20. Fig. 22D is a protein blot of extracts prepared from cotton cotyledons after transient expression with pHEX48. Proteins were precipitated with acetone prior to solubilisation in sample buffer, separated on a 4-12% Novex Bis-Tris SDS gel and transferred onto a 0.22 micron nitrocellulose membrane. The blot was probed with NaPI antibody. Lane 1: 150 ng of purified NaPI, lane 2: cotyledon sample transfected with pHEX48, lane 3: cotyledon sample transfected with pBIN19 empty vector. The 6kDa NaPI peptides are arrowed. The NaPI peptides and several processing intermediates were detected in the cotyledon tissue transfected with pHEX48.

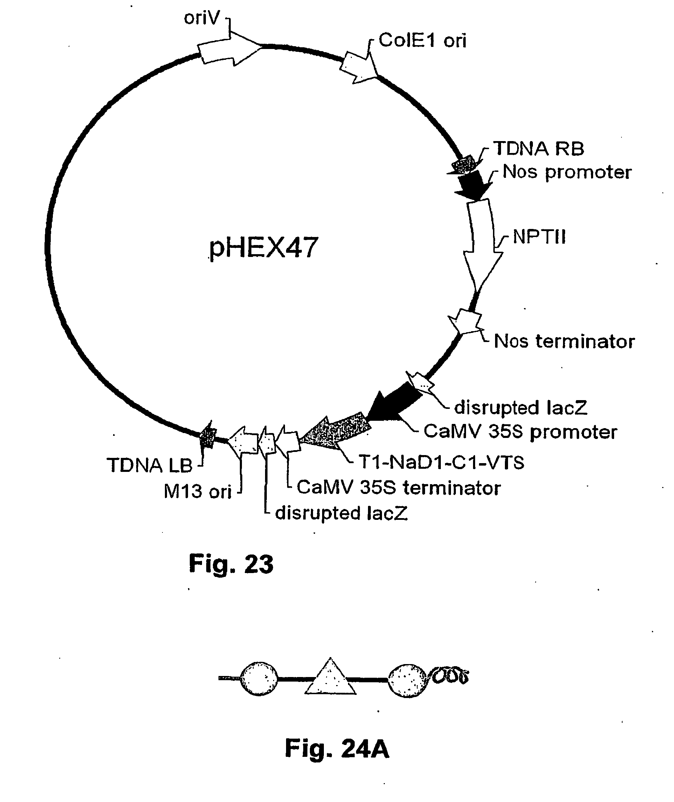

**Fig. 23** is a plasmid map of pHEX47 used in Example 11.

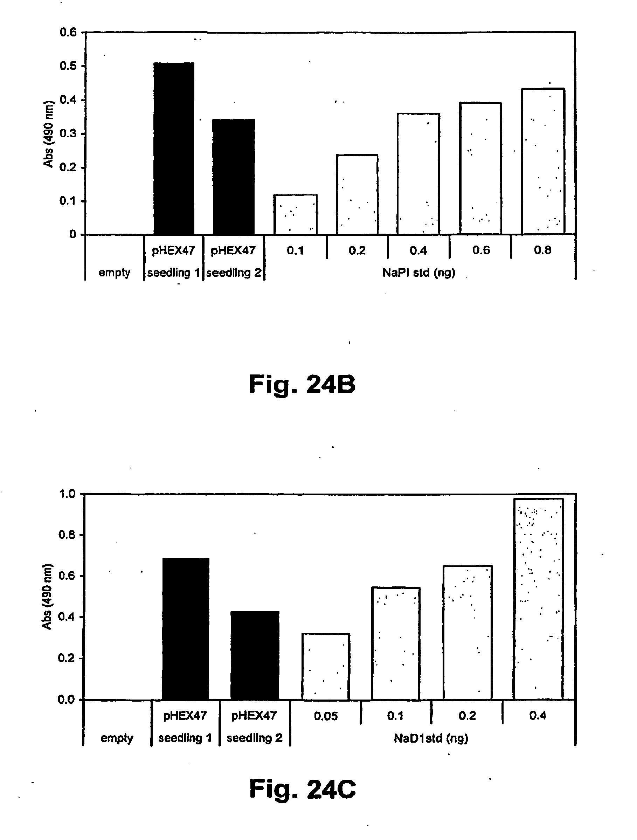

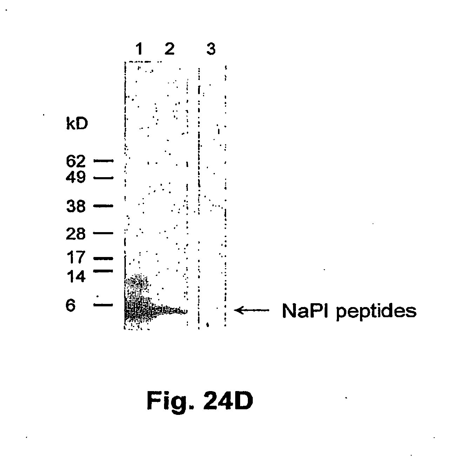

**Figs. 24A-24D** provide data based on use of a 3-domain linear MGEV for expression of NaPI and mature NaD1 in cotton cotyledons. Fig. 24A is a diagram of the linear protein encoded by MGEV-15 and expressed in pHEX47 which has an endoplasmic reticulum signal sequence (stick), two 6kDa proteinase inhibitor domains (spheres), one NaD1 domain (triangle) and a vacuolar targeting sequence (helix). A linker peptide is indicated by a solid line connecting each protein domain. The predicted size of the unprocessed MGEV-15 product is 22.3 kDa minus the signal sequence. Fig. 24B is a bar graph of data from ELISA detection of NaPls in extracts from cotton cotyledons after transient expression with pHEX47. Samples were diluted 1:1,000. Fig. 24C is a bar graph of data from ELISA detection of NaD1 in extracts from cotton cotyledons after transient expression with pHEX47. Samples were diluted 1:100. Fig. 24D is a protein blot of extracts prepared from cotton cotyledons after transient expression with pHEX47. Proteins were precipitated with acetone prior to solubilisation in sample buffer, separated on a 4-12% Novex Bis-Tris SDS gel and transferred onto a 0.22 micron nitrocellulose membrane. The blot was probed with NaPI antibody. Lane 1: 400 ng purified NaPI, lane 2: cotyledon sample transfected with pHEX47, lane 3: untransformed Coker. The 6kDa NaPI peptides (arrowed) were present in the cotyledon sample transfected with pHEX47.

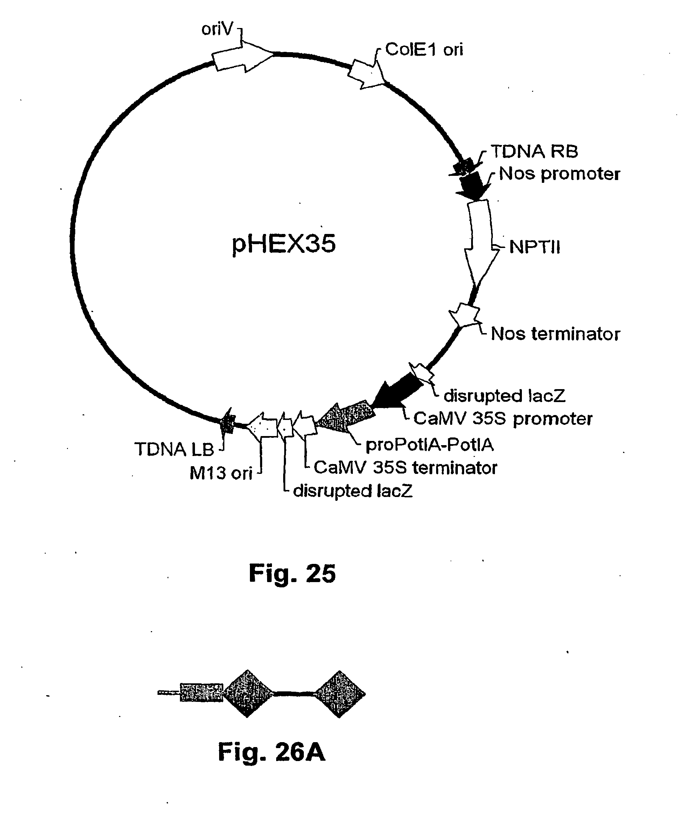

**Fig. 25** is a plasmid map of pHEX35 used in Example 12.

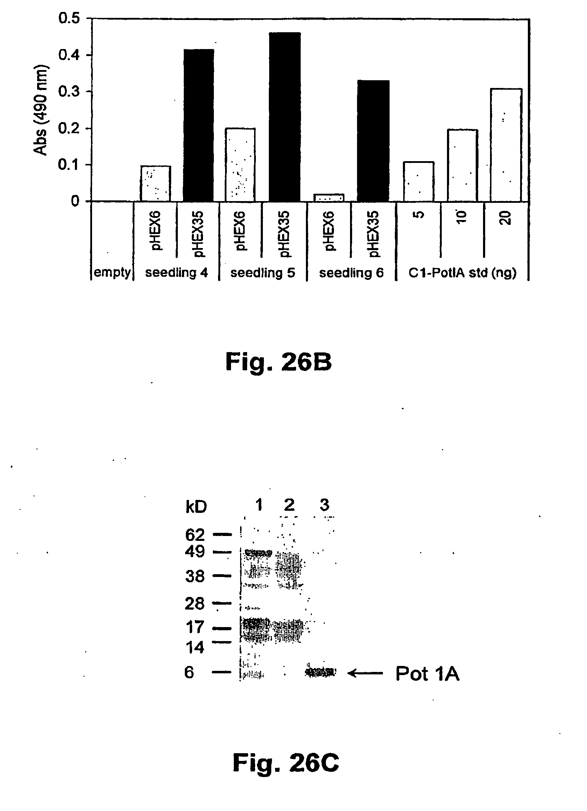

**Figs. 26A-26C** provide data based on use of a 2-domain linear MGEV for expression of PotIA in cotton cotyledons. Fig. 26A is a diagram of the linear protein encoded by MGEV-16 and expressed in pHEX35 which has an endoplasmic reticulum signal sequence (stick), a PotIA prodomain (rectangle) and two PotIA domains (diamond). A linker peptide is indicated by a solid line connecting each protein domain. The predicted size of the unprocessed MGEV-16 product is 19.4 kDa minus the signal sequence. Fig. 26B is a bar graph of data from ELISA detection of Pot1A in extracts from cotton cotyledons after transient expression with pHEX35 and pHEX6. Samples were diluted 1:50. pHEX6 is the same as construct pHEX35 except that there is only one copy of the Pot1A gene. In the 3 seedlings assessed, expression of Pot1A was higher when the Pot1A dimer was used (pHEX35) compared to a single Pot1A domain (pHEX6). pHEX6 is disclosed in published patent application (WO2004/094630). Fig. 26C is a protein blot of extracts prepared from cotton cotyledons after transient expression with pHEX35. Proteins were precipitated with acetone prior to solubilisation in sample buffer, separated on a 4-12% Novex Bis-Tris SDS gel and transferred onto a 0.22 micron nitrocellulose membrane. The blot was probed with PotIA antibody. Lane 1: cotyledon sample (seedling 2) transfected with pHEX35, lane 2: cotyledon sample transfected with pBIN19 empty vector, lane 3: 100 ng purified Pot 1A. The mature Pot 1A (arrowed) was produced in the cotyledon seedling transfected with pHEX35.

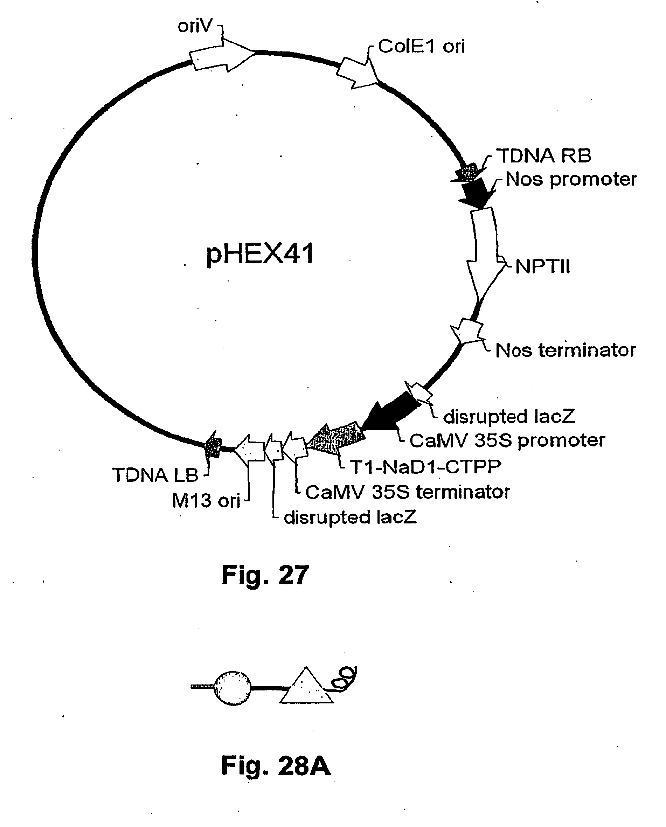

**Fig. 27** is a plasmid map of pHEX41 used in Example 13.

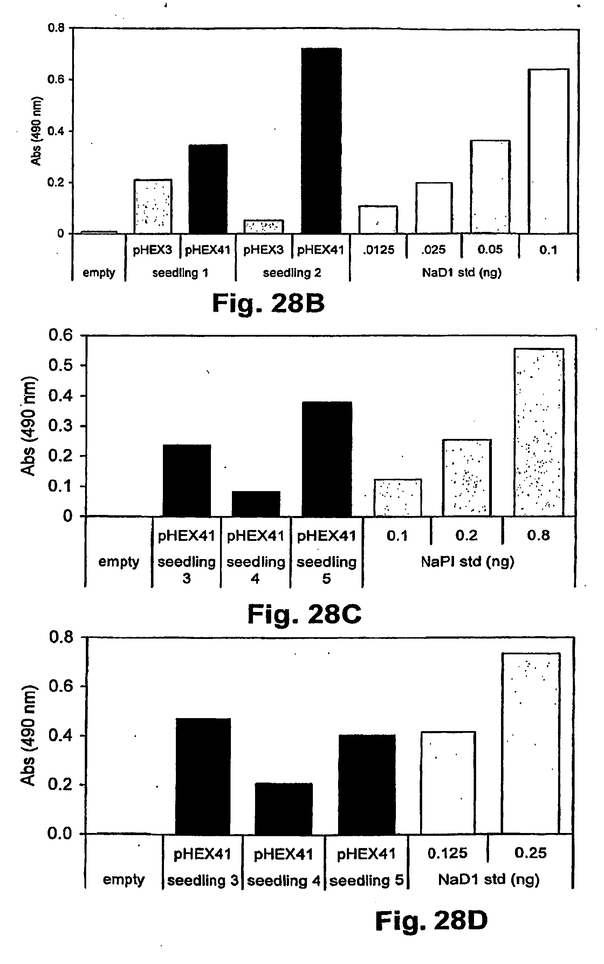

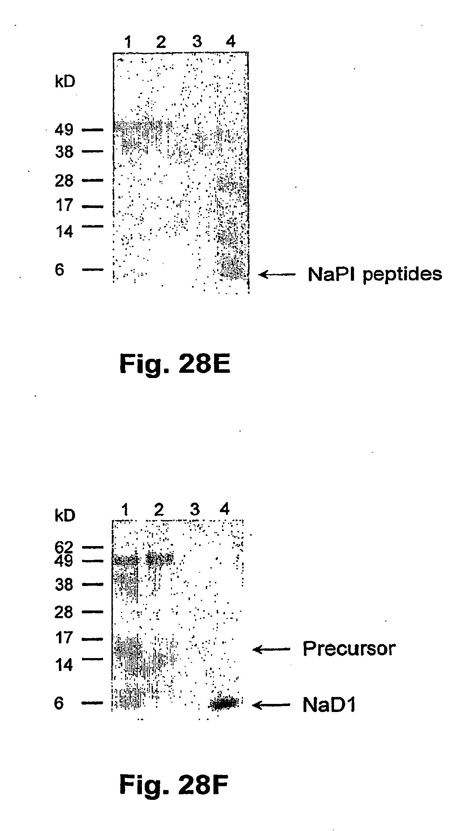

**Figs. 28A-28F** provide data based on use of a 2-domain linear MGEV for expression of NaD1 in cotton cotyledons. Fig. 28A is a diagram of the linear protein encoded by MGEV-17 and expressed in pHEX41 which has an endoplasmic reticulum signal sequence (stick), one 6kDa proteinase inhibitor domain (sphere), one NaD1 domain (triangle) and the CTPP tail that enables targeting to the vacuole (helix). A linker peptide is indicated by a solid line connecting each protein domain. The predicted size of the unprocessed MGEV-17 product is 15.8 kDa minus the signal sequence. Fig. 28B is an ELISA detection of NaD1 in extracts from cotton cotyledons after transient expression with pHEX41 and pHEX3. Samples were diluted 1:500. pHEX3 is the same as pHEX41 except that it does not contain the NaPI domain. In the 2 seedlings assessed, expression of NaD1 was higher when expressed with the NaPI domain (pHEX35) compared to expression of NaD1 alone (pHEX6). pHEX3 is disclosed in U.S. Patent 6,031,087. Fig. 28C is a bar graph of data from ELISA detection of NaPI in extracts from cotton cotyledons after transient expression with pHEX41. Samples were diluted 1:1,000. Fig. 28D is a bar graph of data from ELISA detection of NaD1 in extracts from cotton cotyledons after transient expression with pHEX41. Samples were diluted 1:500. Fig. 28E is a protein blot of extracts prepared from cotton cotyledons after transient expression with pHEX41. Proteins were precipitated with acetone prior to solubilisation in sample buffer, separated on a 4-12% Novex Bis-Tris SDS gel and transferred onto a 0.22 micron nitrocellulose membrane. The blot was probed with NaP1 antibody. Lane 1: cotyledon sample (seedling 2) transfected With pHEX41, lane 2: cotyledon sample transfected with pBIN19 empty vector, lane 3: blank, lane 4: 200 ng purified NaPI. The 6kDa NaPI peptides (arrowed) were present in the cotyledon sample transfected with pHEX41. Fig. 28F is a protein blot of extracts prepared from cotton cotyledons after transient expression with pHEX41. Proteins were precipitated with acetone prior to solubilisation in sample buffer, separated on a 4-12% Novex Bis-Tris SDS gel and transferred onto a 0.22 micron nitrocellulose membrane. The blot was probed with NaD1 antibody. Lane 1: cotyledon sample (seedling 2) transfected with pHEX41, lane 2: cotyledon sample transfected with pBIN19 empty vector, lane 3: blank, lane 4: 150 ng purified NaD1. The precursor and 6kDa NaD1 (arrowed) were present in the cotyledon sample transfected with pHEX41.

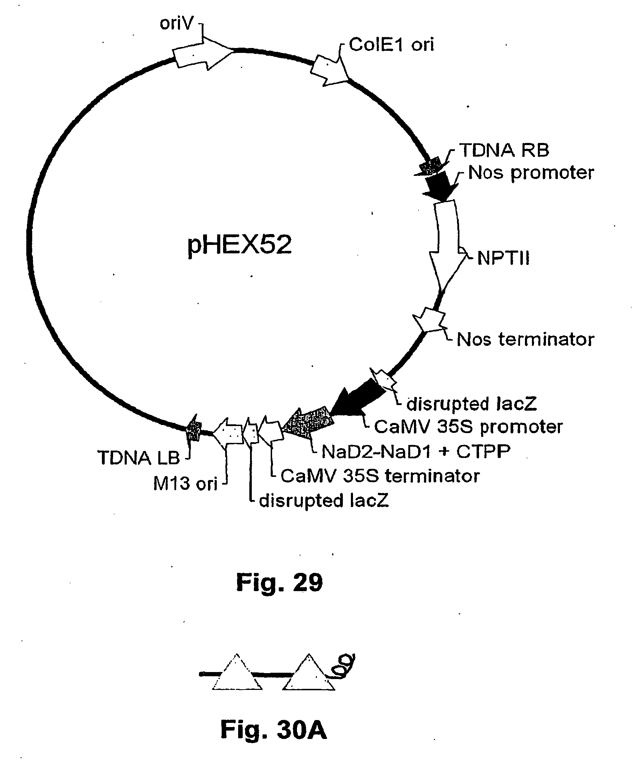

**Fig. 29** is a plasmid map of pHEX52 used in Example 14.

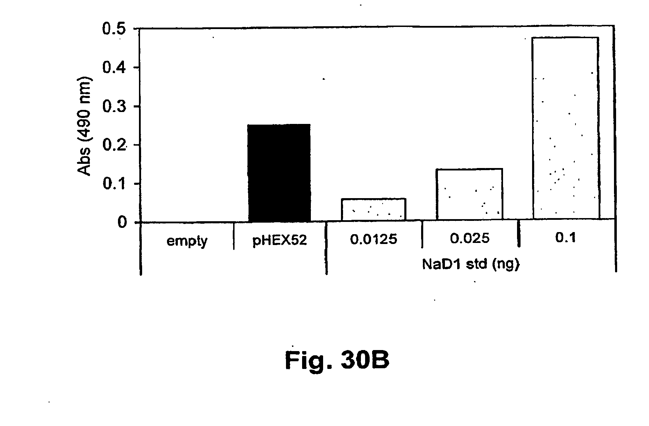

**Fig. 30** provides data based on use of a 2-domain linear MGEV for expression of NaD2 and NaD1 in cotton cotyledons. Fig. 30A is a diagram of the linear protein encoded by MGEV-18 and expressed in pHEX52 which has an endoplasmic reticulum signal sequence (stick), one NaD2 domain (triangle), one NaD1 domain (triangle) and the CTPP tail that enables targeting to the vacuole (helix). A linker peptide is indicated by a solid line connecting each protein domain. The predicted size of the unprocessed MGEV-18 product is 14.7 kDa minus the signal sequence. Fig. 30B is a bar graph of data from ELISA detection of NaD1 in extracts from cotton cotyledons after transient expression with pHEX52.

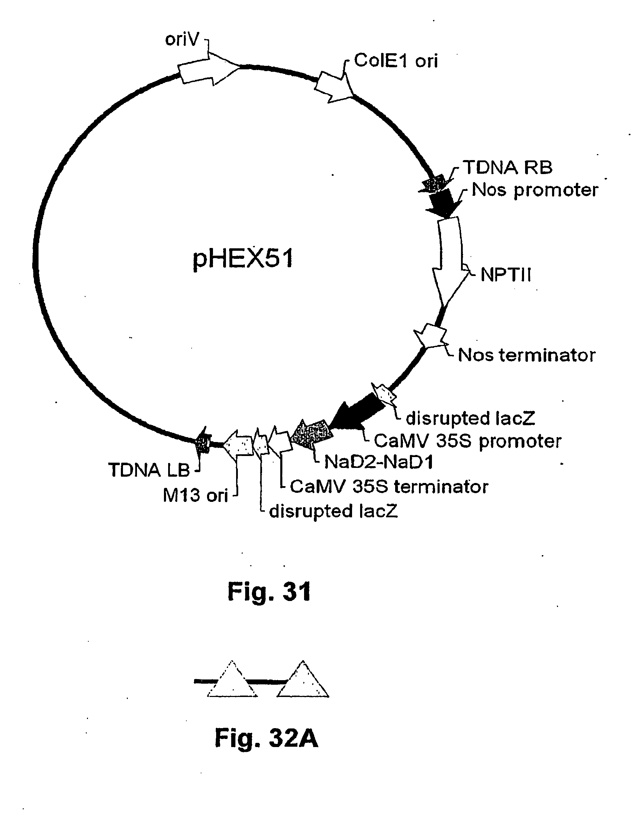

**Fig. 31** is a plasmid map of pHEX51 used in Example 15.

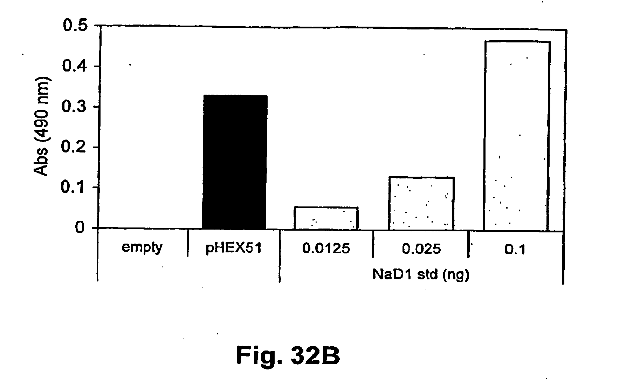

**Figs. 32A-32B** provide data based on use of a 2-domain linear MGEV for expression and targeting of NaD2 and NaD1 to the extracellular space in cotton cotyledons. Fig. 32A is a diagram of the linear protein encoded by MGEV-19 and expressed in pHEX51 which has an endoplasmic reticulum signal sequence (stick), one NaD2 domain (triangle) and one NaD1 domain (triangle). A linker peptide is indicated by a solid line connecting each protein domain. The predicted size of the unprocessed MGEV-19 product is 11.1 kDa minus the signal sequence. Fig. 32B is a bar graph of data from ELISA detection of NaD1 in extracts from cotton cotyledons after transient expression with pHEX51. Samples were diluted 1:100.

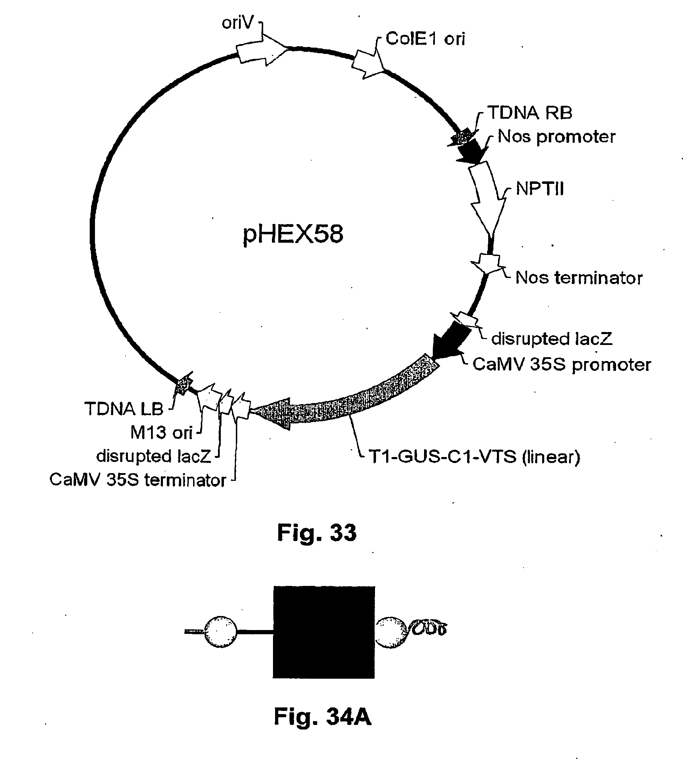

**Fig. 33** is a plasmid map of pHEX58 used in Example 16.

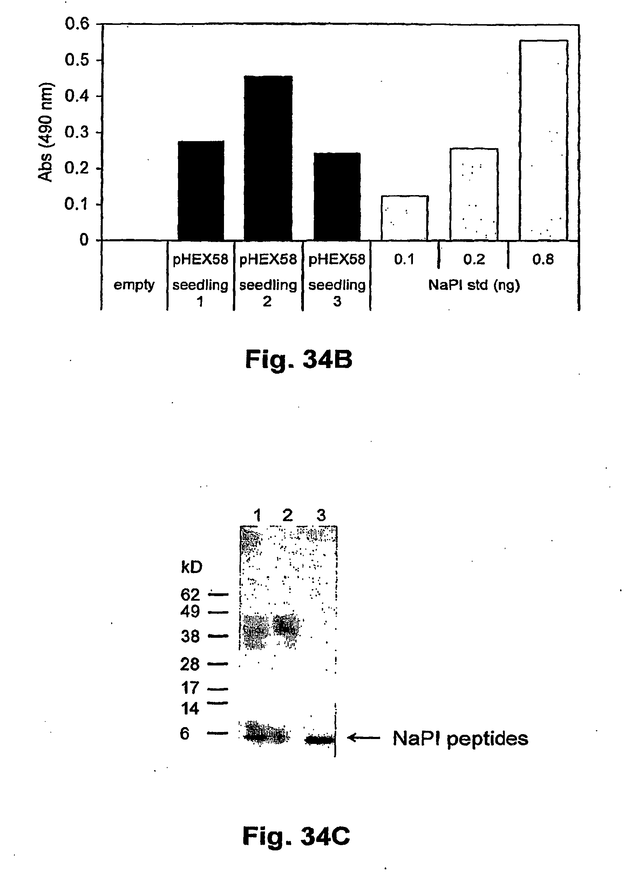

**Figs. 34A-34C** provide data based on use of a 2-domain linear MGEV for expression and targeting of GUS to the vacuole in cotton cotyledons. Fig. 34A is a diagram of the linear protein encoded by MGEV-20 and expressed in pHEX58 which has an endoplasmic reticulum signal sequence (stick), two 6kDa proteinase inhibitor domains (spheres), one GUS (square) and a vacuolar targeting sequence (helix). A linker peptide is indicated by a solid line connecting each protein domain. The predicted size of the unprocessed MGEV-20 product is 84.8 kDa minus the signal sequence. Fig. 34B is a bar graph of data from ELISA detection of NaPI in extracts from cotton cotyledons after transient expression with pHEX58. Samples were diluted 1:1,000. Fig. 34C is a protein blot of extracts prepared from cotton cotyledons after transient expression with pHEX58. Proteins were precipitated with acetone prior to solubilisation in sample buffer, separated on a 4-12% Novex Bis-Tris SDS gel and transferred onto a 0.22 micron nitrocellulose membrane. The blot was probed with NaPI antibody. Lane 1: cotyledon sample (seedling 2) transfected with pHEX58, lane 2: cotyledon sample transfected with pBIN19 empty vector, lane 3: 150 ng purified NaPI peptides. The NaPI peptides (arrowed) were produced in the cotyledon seedling transfected with pHEX58.

### DETAILED DESCRIPTION OF THE INVENTION

Various MGEV structures are detailed herein and in the following examples. A general MGEV structure encoding a circular polyprotein (MGEV-P) is diagrammed as follows:

S-C2_{N}-(LⱼDₖ)ₘ-LⱼC2_{C}-V

where each capital letter symbolizes a polynucleotide encoding a segment of amino acids designated according to its function, thus: S is a polynucleotide segment with an open reading frame encoding a signal peptide; Dₖ is a polynucleotide segment with an open reading frame encoding a functional protein (hereinafter a "Domain") wherein k represents an ordinal number to identify any single functional Domain selected from a group of domains having from 3 to m members and wherein D does not encode a type two protease inhibitor; Lⱼ is a polynucleotide segment with an open reading frame encoding a linker polypeptide of SEQ ID NO: 5 where Lⱼ is a ordinal number to identify each single linker (L) selected from a group having from 3 to m+1 members; C2_{N} is a polynucleotide segment with an open reading frame encoding a N-terminal clasp peptide; C2c is a polynucleotide with an open reading frame encoding a C-terminal clasp peptide; V is a vacuolar targeting peptide; m is a cardinal number from 3-8; and S,C2_{N},L,D,C2_{C} and V are all in the same reading frame same as each other. As an example, a MGEV encoding 3 functional domains (D) can be diagrammed as shown above, where m is 3, k is 1, 2 or 3, j is 1, 2, 3, or 4. In another linear embodiment, described below, clasp proteins are omitted or truncated.

Lⱼ encodes a linker amino acid sequence of SEQ ID NO: 5.

In a plant cell, the MGEV encoded protein (MGEV-P) undergoes several steps of post-translational processing. These include intracellular transport to the endoplasmic reticulum, provided the leader (S) is present, followed by removal of S and subsequent transport to an intracellular storage vacuole provided the vacuolar targeting sequence (V) is present. V is removed in the vacuole. If C2_{N} and C2c are present, the ends of the MGEV-P become joined together to form a closed loop, diagrammed as follows: where C2_{N},L₁₋₄, D₁₋₃, C2_{C}, and V are as described *supra.*

Post-translational proteolysis cleavage at each linker and between C2c and V results in release of D₁, D₂, D₃ and, in one embodiment, C2, as separate proteins. Expression of the MGEV thereby results in concurrent expression of at least three separate proteins which are not type two proteinase inhibitors, from a single promoter.

A circular MGEV can encode from 3 to 8 functional domains (D), concurrently expressed. Concurrent expression is defined herein to mean the intracellular synthesis of a plurality of functional proteins from a single transcript. Concurrent expression is especially useful when it is desired or necessary to produce and accumulate large amounts of proteins in a plant cell, for example, plant protectant proteins, or economically significant proteins, or when it is advantageous to control the relative amounts of expressed proteins, or for expression of certain proteins, such as cysteine-rich peptides, that are normally expressed poorly in plant cells. When the MGEV includes a vacuole targeting peptide (V), the concurrently expressed proteins are accumulated in a storage vacuole in the cell, which can serve two purposes: (1) to provide the proteins in concentrated form to maintain an effective dose of plant protectant in the event of pathogen attack, or to ease purification of an economically valuable protein; and (2) to sequester otherwise toxic proteins which can confer added pest resistance and economic value to a plant expressing such proteins. V can be combined with any domain to be expressed, most conveniently at the 3'-end of MGEV. More than one V can be included if desired. In the absence of V, proteins released from MGEV-P by proteolysis can be exported from the cell.

The expressed components of an MGEV are described herein in greater detail.

The protein domains (D) encoded by open reading frames of the MGEV nucleotide sequence can, in principle, be any protein although type two proteinases are excluded. No upper size limit is known for a protein expressible as a component of a MGEV. Exemplified herein are data demonstrating concurrent expression of individual domains encoding proteins ranging from about 5kDa to greater than 65kDa. Practical considerations known to those skilled in the art can be considered when choosing proteins appropriate for expression using an MGEV. For example, very large proteins may be expressed individually more efficiently, rather than as part of a MGEV. Certain proteins may sterically interfere with cyclization under certain circumstances. Each protein domain (D) is connected to a linker peptide (L) by peptide bonds at the N-terminal and C-terminal amino acids of the domain.

It is presently believed that efficiency of post-translational peptide cleavage that liberates individual protein domains from the MGEV-P is maximized when the N-and C-termini of each domain and connecting linkers are exposed by the protein conformation to the aqueous environment on the surface of the protein, rather than sequestered internally within the protein. Therefore, candidate proteins for expression as part of the MGEV-P preferably have exposed N- and C-terminal amino acids.

Examples of proteins which can be expressed using an MGEV include (without limitation) potato type one Pl's, plant defensins, animal defensins, proteinaceous toxins, chimeric and fusion proteins, as well as indicator proteins such as Green Fluorescent Protein (GFP), 28kDa, and beta-glucuronidase (GUS), 686kDa. Examples of protein-coding domains that can be expressed in the MGEV include plant protection proteins such as potato proteinase inhibitors of type one (Pot 1A), plant seed defensins, plant floral defensins, insect-toxic peptides such as scorpion toxin, *Bacillus thuringiensis* toxins, heat shock proteins, Bowman-Birk trypsin inhibitors, and cystatins and indicators such as green fluorescent protein (GFP) and beta-glucuronidase (GUS). Proteins of economic value for purposes other than plant protection can be expressed using the MGEV, taking advantage of high expression levels, including anti-microbial peptides. antibody fragments and the like suitable for medical use. Also large hetero-dimeric. or hetero-multimeric proteins are especially suitable for MGEV expression where concurrent and correctly proportional expression is desired. The proteins encoded by a MGEV are not type two Pis. The MGEV is particularly useful for expression of proteins that may be toxic to the cell in which they are expressed, by providing for transport to, and sequestration in, a storage vacuole within the plant cell.

A linker (L) is a short peptide positioned between each domain that separates each adjacent domain and exposes a peptidase-sensitive site for post-translational cleavage between individual domains. The amino acid sequence of the linker peptide is EEKKN (SEQ ID NO:5). The linker provides a highly hydrophilic segment that exposes a proteolytic cleavage site (N - X) to the outer surface of MGEV-P. The linker peptides described herein are advantageous because post-translational processing of domains joined by a linker can result in removal of the entire linker in transgenic plants. (See Heath, R.L. et al., (1995) Eur. J. Biochem. 230:250-257).

The leader peptide, also referred to as a signal peptide (S), is a sequence of about 10 to about 30 mostly hydrophobic amino acids which serves a transport function for intracellular transport. Many signal peptides are known in the art. Any known signal peptide can be used in the MGEV-P, as well as modifications thereof wherein homologous amino acids are substituted.

The vacuole targeting peptide (V) is located at the C-terminus of the MGEV-P. A variety of vacuolar targeting determinants are known to exist in plant cells, see, e.g. Maruyama et al. Plant Cell (2006) 18:1253-1273. Suitable vacuolar targeting peptides can be chosen from a wide variety of known candidates. Also, a suitable V segment need not be placed at the C-terminus of the MGEV, but could, in principle be located elsewhere in the sequence; for example attached to the N-terminus of C2_{N}, between S and C2_{N}. In one embodiment, a suitable sequence can be one which binds to the known BP-80 vacuolar sorting receptor. Any such vacuole targeting sequence that binds BP-80 or a homolog thereof can be used as a component of the MGEV-P. Another example of a suitable vacuole targeting sequence is shown in Miller, et al. *supra,* Fig. 1, amino acids 258-281 of the NaPI-iv sequence (SEQ ID NO:2). Other examples include the C-terminal propeptide of NaD1 (SEQ ID NO:14, amino acids 27-105 and the Pot1A prodomain, SEQ ID NO:20),

The clasp segments, C2_{N} and C2c are represented herein by amino acids 30-48 (C2_{N}) and 228-257 (C2c) SEQ ID NO:6. The folded configuration of peptides C2_{N} and C2c is such that they readily bind to one another, and the heterodimer formed by the binding is then stabilized covalently by formation of inter-peptide disulfide cross-links. The cross-linked [C2_{N} : C2c] protein has chymotrypsin activity and is designated simply as C2 herein. In the MGEV-P structure, formation of C2 results in cyclization of MGEV-P with a C-terminal extension, the vacuole targeting peptide, V. A clasp structure can be formed using any of the type 2 inhibitors regardless of protease specificity, because of the high degree of homology among them. Deletion of the four amino acid sequence PRNP (or PKNP in the case of T5) which is common to these inhibitors will create the appropriate N-terminal and C-terminal segments of a clasp peptide. Formation of a cyclic structure is not necessary for activity of MGEV-P. A cyclic structure of MGEV-P is considered advantageous for efficient intracellular transport. A further advantage of the cyclic configuration is that the additional inhibitor thereby formed is a useful plant protectant against insect damage.

The total or partial deletion of C2_{N} and C2c can prevent formation of a cyclic structure and result in a linear configuration. The invention includes both linear and cyclic configurations of MGEV-P. A linear MGEV is advantageous whenever a large protein, a mix of large and small proteins, or a protein lacking a compact tertiary structure is to be expressed. In certain circumstances expression levels can be increased by use of a linear MGEV-P instead of the cyclic form. Targeting to the endoplasmic reticulum by S and vacuolar targeting by V can occur as previously described. A linear MGEV can have as few as two domains. Post-translational processing of linear MGEV-P can occur as described, with release of individual active domains (Dₖ). A diagram of a linear MGEV-P having 3 protein domains lacking C2_{N} and C2c is shown, wherein non-specific peptides P_{N} and Pc are provided in place of C2_{N} and C2c, respectively.

S-P_{N}-(LⱼDₖ)ₘLⱼPc-V

where j is 1, 2 or 4, k is 1, 2 or 3, m is 3.

PN and PC can be modified or partially deleted versions of C2_{N} and C2c, respectively. Preferably, C2_{N} and C2c are entirely deleted, such that a linear 3-domain MGEV has the diagram structure:

S - (DₖLⱼ)ₘ Dₖ₊₁ V

where j and k are 1 or 2 and m is 2.

As noted previously, V need not be at the C -terminus, but could be located elsewhere in the sequence, for example between S and D.

The linear MGEV-P can have up to eight functional protein domains, which are not type two proteinase inhibitors. As with cyclic MGEV-P, the linear form can be exported from the cell by deletion of the vacuole targeting sequence, V.

Constructing a MGEV can be carried out by known methods of combining the nucleic acid segments in the designated order, by DNA synthesis, or a combination of both methods. A convenient method is to employ components of naturally-occurring type two PI multimers, such as NaPI-iv from *N. alata,* SEQ ID NO:2 [Miller, (2000) *supra,* GenBank accession number AF105340]. One or more open reading frames encoding a functional protein domain of interest that is not a type two PI can be inserted together with appropriate linkers into the naturally-occurring multimer, thereby increasing the number of expressed domains, and pre-existing domains can be deleted, followed by insertion of desired domain-coding segments to keep the total number of domains unchanged as long as all coding segments remain in the same reading frame from one to the next. Examples of protein-coding domains that can be expressed in the MGEV include plant protection, proteins such as potato proteinase inhibitors of type one, for example as disclosed in International Publication No. WO 2004/094630, including Pot1A exemplified herein, plant seed defensins, plant floral defensins, insect-toxic peptides such as scorpion toxin, *Bacillus thuringiensis* toxins, heat shock proteins, Bowman-Birk trypsin inhibitors, and cystatins and indicators such as green fluorescent protein (GFP) and beta-glucuronidase (GUS). Proteins of economic value for purposes other than plant protection can be expressed using the MGEV, taking advantage of high expression levels, including anti-microbial peptides. antibody fragments and the like suitable for medical use. Also large hetero-dimeric or hetero-multimeric proteins are especially suitable for MGEV expression where concurrent and correctly proportional expression is desired.

The following Examples demonstrate construction of MGEV's encoding plant-protective proteins, plant transformation with MGEV, transgenic plants containing and expressing the MGEV and protection from plant pests due to expression of non- Potato Type Two proteins encoded within a MGEV, and MGEVs encoding a mix of large and small proteins. These Examples are presented to illustrate, but not limit, the invention as claimed.

A MGEV can be expressed in plants or plant cells after being incorporated into a plant transformation vector. Many plant transformation vectors are well known and available to those skilled in the art, e.g., BIN19 (Bevan, (1984) Nucl. Acid Res. 12:8711-8721), pBI 121 (Chen, P-Y, et al., (2003) Molecular Breeding 11:287-293), pHEX 22 (U.S. Patent 7,041,877), and vectors exemplified herein. Such vectors are well-known in the art, often termed "binary" vectors from their ability to replicate in a bacteria such as *Agrobacterim tumefaciens* and in a plant cell. A typical plant transformation vector, such as exemplified herein, includes genetic elements for expressing a selectable marker such as NPTII under control of a suitable promoter and terminator sequences, active in the plant cells to be transformed (hereinafter "plant-active" promoter or terminator) a site for inserting a gene of interest, including a MGEV under expression control of suitable plant-active promoter and plant-active terminator sequences and T-DNA borders flanking the MGEV and selectable marker to provide integration of the genes into the plant genome.

Plants are transformed using a strain of *A. tumefaciens,* typically strain LBA4404 which is widely available. After constructing a plant transformation vector that carries a MGEV encoding the desired proteins, the vector is used to transform an A. *tumefaciens* strain such as LBA4404. The transformed LBA4404 is then used to transform the desired plant cells using an art-known protocol appropriate for the plant species to be transformed. Standard and art-recognized protocols for selecting transformed plant cells, multiplication and regeneration of selected cells are employed to obtain transgenic plants. The examples herein further disclose methods and materials used for transformation and regeneration of cotton plants, as well as transgenic cotton plants transformed by and expressing a variety of MGEVs. A MGEV can be transferred into plant cells by any of several known methods besides those exemplified herein. Examples of well-known methods include microprojectile bombardment, electroporation, and other biological vectors including other bacteria or viruses.

The MGEV can be used for multigene expression in any monocotylodenous or dicotyledonous plant. Particularly, useful plants are food crops such as com (maize) wheat, rice, barley, soybean and sugarcane and oilseed crops such as sunflower and rape. Particularly useful non-food common crops include cotton, flax and other fiber crops. Flower and ornamental crops include rose, carnation, petunia, lisianthus, lily, iris, tulip, freesia, delphinium, limonium and pelargonium.

Techniques for introducing vectors, chimeric genetic constructs and the like into cells include, but are not limited to, transformation using CaCl₂ and variations thereof, direct DNA uptake into protoplasts, PEG-mediated uptake to protoplasts, microparticle bombardment, electroporation, microinjection of DNA, microparticle bombardment of tissue explants or cells, vacuum-infiltration of tissue with nucleic acid, and T-DNA-mediated transfer from *Agrobacterium* to the plant tissue.

For microparticle bombardment of cells, a microparticle is propelled into a cell to produce a transformed cell. Any suitable ballistic cell transformation methodology and apparatus can be used in performing the present invention. Exemplary procedures are disclosed in Sanford and Wolf (U.S. Patent Nos. 4,945,050, 5,036,006, 5,100,792, 5,371,015). When using ballistic transformation procedures, the genetic construct can incorporate a plasmid capable of replicating in the cell to be transformed.

Examples of microparticles suitable for use in such systems include 0.1 to 10 µm and more particularly 10.5 to 5 µm tungsten or gold spheres. The DNA construct can be deposited on the microparticle by any suitable technique, such as by precipitation.

Plant tissue capable of subsequent clonal propagation, whether by organogenesis or embryogenesis, can be transformed with a MGEV of the present invention and a whole plant generated therefrom, as exemplified herein. The particular tissue chosen will vary depending on the clonal propagation systems available for, and best suited to, the particular species being transformed. Examples of tissue targets include leaf disks, pollen, embryos, cotyledons, hypocotyls, megagametophytes, callus tissue, existing meristematic tissue (e.g. apical meristem, axillary buds, and root meristems), and induced meristem tissue (e.g. cotyledon meristem and hypocotyl meristem).

The regenerated transformed plants can be propagated by a variety of means, such as by clonal propagation or classical breeding techniques. For example, a first generation (or T1) transformed plant may be selfed to give a homozygous second generation (or T2) transformant and the T2 plants further propagated through classical breeding techniques.

Accordingly, this aspect of the present invention, insofar as it relates to plants, further extends to progeny of the plants engineered to express the nucleic acid of the MGEV as well as vegetative, propagative and reproductive parts of the plants, such as flowers (including cut or severed flowers), parts of plants, fibrous material from plants (for example, cotton) and reproductive portions including cuttings, pollen, seeds and callus.

A genetically modified plant cell or multicellular plant or progeny thereof or parts of a genetically modified plant may be capable of producing a protein or peptide encoded by the MGEV as herein described wherein said transgenic plant has acquired a new phenotypic trait associated with expression of the protein or peptide.

MGEV structures and MGEV expression vectors exemplified herein are listed in Table 2, together with the number of the Example where they are described. Sequence ID listings are listed in Table 3.

### EXAMPLE 1

### Construction and expression of an MGEV having one type one PI and 3 potato type twoPI's.

The MGEV described in this example (MGEV-5) SEQ ID NO:6 has the structure diagrammed as:

S-C2_{N}-L₁D₁-L₂D₂-L₃D₃-L₄-C2c-V;

wherein L₁₋₄ encodes the linker amino acid sequence -EEKKN- SEQ ID NO:5, D₁ encodes a potato type two trypsin inhibitor, T1 SEQ ID NO:3; SEQ ID NO:1 amino acids 112-164; D₂ encodes a potato type one chymotrypsin inhibitor, potato Pot 1A SEQ ID NO:11, (also SEQ ID NO:5, bases 352-376); D₃ encodes a Type Two chymotrypsin inhibitor, C1 SEQ ID NO:2 amino acids 54-106; C2_{N} SEQ ID NO:1 amino acids 31-48 and C2c SEQ ID NO:1 amino acids 344-373 encode peptides that interact with each other to form a heterodimer C2 stabilized by disulfide crosslinks, the cross-linked protein having potato type two chymotrypsin inhibitor activity. S encodes a signal peptide and V encodes a vacuole translocation peptide.

Amino acid sequences encoded by the above-identified segments are described in the following sources:
For S-C2_{N}-L₁D₁, amino acids 1-29(S); 30-48 (C2_{N}); 112-164 (D₁) of SEQ ID NO:2
For L₂, amino acids EEKKN, SEQ ID NO:5
For D₂, (SEQ ID NO:11) [see also International Publication No. WO2004/094630 (Nov. 4, 2004) SEQ ID NO:81, incorporated herein by reference to the extent not inconsistent herewith]
For L₃D₃, amino acids 49-106 of SEQ ID NO:2
For L₄C2cV, SEQ ID NO:2 amino acids 223-257 (L₄C2c); and 258-281 (V)

A multipurpose vector, pRR19 was constructed. The vector contained sequences obtained from NaPI-iv SEQ ID NO:2 and NaPI-ii SEQ ID NO: 1 [Miller (2000) *supra]* plus restriction sites for insertion of new genes. The entire MGEV-1 sequence was assembled in consecutive order into pRR19.

The vector pRR19 was designed to allow convenient modular assembly of linkers (L) and open reading frames (D) into a MGEV having the desired combination of components. As step 1, polymerase chain reaction (PCR) was used to amplify the respective N- and C- terminal end segments of NaPI-iv, specifically S-C2_{N} (SEQ ID NO:2 amino acids 1-48) and C2c-V (SEQ ID NO:2 amino acids 228-281), and to provide Xho1 restriction sites. The Xho1 restriction sites were provided to permit joining of desired segments between the terminal segments, such that the amplified segments had the diagram structure S-C2_{N}L₁- Xho1 and Xho1 -C2c-V, respectively. After cutting and ligation, the segment S-C2_{N}-L₁-Xho1-C2c-V was cloned into the pGEM T-Easy (Promega, Madison, WI) vector.

Any desired DNA segment having Xho1 sites at its N and C termini could then be inserted into the Xho1 site of the resulting vector.

As the step 2, in parallel preparations, DNA encoding the T1 of NaPI-ii (SEQ ID NO:1, amino acids 112-164) (to be in position D₁ in MGEV-5) and the DNA encoding the C1 domain of NaPI-iv (SEQ ID NO:2, amino acids 54-106) (to be position D₃ in MGEV-5) were PCR-amplified with restriction sites added as diagrammed:
Xho1 - T1 - L₁ - Xba1, and Xba1- C1- L₁ - Xho1.

Each of the constructs was separately cloned into pGEM T-easy vectors, digested with Xba1 and Xho1 and purified.

The modified T1 and C1 domains from the preceding step were combined in a DNA ligation reaction mixture with Xho1-digested product of the first step. The ligation mixture was transformed into E. *coli* XL1-Blue cells (Stratagene, LaJolla, CA) and restriction digests and sequencing were carried out to confirm the desired orientation of arid order of the proteinase inhibitor domains. The predicted ligation reactions were DNA segments encoding the following components:

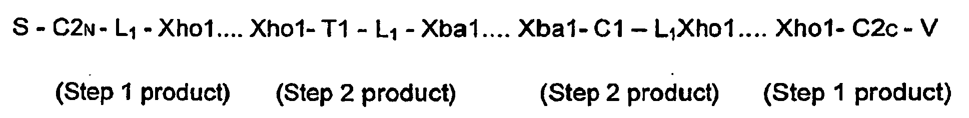

The ligation product, as verified by electrophoresis of restriction digests and sequence analysis, was
S - C2_{N}- L₁- Xho1- T1- L₁- Xba1- C1 - L₁- Xho1- C2c - V

The ligation product contained a unique Xba1 site (underlined) into which could be inserted any desired coding sequence provided with Xba1 restriction sites at both ends. The vector having the described construct was designated pRR19.

For the D₂ domain, the DNA coding for Pot 1A, previously described, was provided with a linker (L) at the C-terminal-coding end, followed by Xba1 restriction sites at the 3' and 5' ends. Insertion at the Xba1 site of pRR19 resulted in a construct that was then inserted into pAM9 (pAM9 was modified from pDHA, Tabe et al., Journal of Animal Science, 73: 2752-2759, 1995) to produce MGEV-5. Insertion in pAM9 resulted in the attachment of the 35S CaMV promoter at the 3' end and the 35S CaMV terminator at the 5' end. MGEV-5 was then inserted into pBIN19 at the EcoR1 site resulting in vector pHEX 29, diagrammed in Fig. 3. See also Fig. 4A.

The use of restriction sites in MGEV-5 could be avoided, if desired, by using DNA synthesis to make the disclosed MGEV-5 sequence of Table 1. See also SEQ ID NO:6 (DNA sequence) and SEQ ID NO:12 (deduced amino acid sequence).

Seeds of *Gossyipium hirsutum* cultivar Coker 315 were surface sterilized in sodium hypochlorite (2% available chlorine) for 60 min followed by several washes in sterile water. The sterilized seed were sown onto Cotton Seed Medium (CSM) [0.22% w/v MS (Murashige and Skoog salt mixture Austratec M524), 0.05% w/v B5 vitamins (Sigma G1019), 1.5% w/v glucose (Austratec G386), 0.2% w/v gellan gum Gelrite, trademark of Merck & Co., (Phyto Technology Laboratories), pH 5.8] and incubated at 30°C in the dark for 10 days. *A. tumefaciens* (LBA4404) transformed with the pHEX29 construct was grown overnight in 25 ml LB medium supplemented with the antibiotic kanamycin (50 µg/mL) at 28°C. The absorbance at 550 nm was measured and the cells were diluted to 2 X 10⁸ cells per ml in MS liquid media (0.43% w/v Murashige and Skoog basal salts, pH 5.8). Cotton hypocotyls were cut into 1.5-2 cm pieces and mixed briefly (0.5-3 min) in the diluted *Agrobacterium* culture. The explants were drained and transferred to medium 1 (0.43% w/v Murashige and Skoog salt mixture, 0.1% v/v Gamborg's B5 vitamin solution (Sigma), 0.1 g/L myo-inositol, 0.9 g/L MgCl₂, (hexahydrate), 1.9 g/L potassium nitrate, 0.2% w/v Gelrite, 3% w/v glucose, pH 5.8) overlayed with sterile filter paper and incubated for 3 days at 26°C under lights.

Following co-cultivation, explants were transferred to medium 2 (medium 1 plus 0.1 mg/L kinetin, 0.1 mg/L 2,4-D, 500 mg/L carbenicillin, 35 mg/L kanamycin) and maintained at 30°C under low light. After 4 weeks explants were transferred to medium 3 (medium 1 plus 500 mg/L carbenicillin, 25 mg/L kanamycin) and maintained at 30°C under low light. Explants and callus were sub-cultured every 4 weeks on medium 3 and maintained at 30°C under low light. Embryos were excised from the tissue and germinated in medium 4 (1.2 mM CaCl₂2H₂O, 5.0 mM KNO₃, 2.0 mM MgSO₄7H₂O, 3.0 mM NH₄NO₃, 0.2 mM KH₂PO₄, 4 µM nicotinic acid, 4 µM pyridoxine HCl, 4 µM thiamine HCl, 30 µM H₃BO₃, 30 µM MnSO₄H₂O, 9 µM ZnSO₄7H₂O, 1.5 µM KI, 0.9 µM Na₂MoO₄2H₂O, 0.03 µM CuSO₄5H₂O, 0.03 µM CoCl₂6H₂O, 15 µM FeNaEDTA, 0.5% w/v glucose, 0.3% w/v gellan gum Gelrite, pH 5.5) and maintained at 30°C under high light.

Germinated embryos were then transferred to Magenta boxes containing medium 4 and maintained at 30°C under high light. Once a plant has formed a good root system and produced several new leaves it was transferred to soil in pots and acclimatised in a growth cabinet at 28°C and then grown in a glasshouse at (27-29°C day, 20-24°C night).

### PCR analysis

DNA isolation: Cotton leaf discs (0.5 - 0.7 cm) were sampled from the 2^{nd} fully expanded leaf, avoiding vein tissue. Extraction solution (100 µl) from the REDExtract-N-Amp Plant PCR kit (Sigma) was added to each leaf disc ensuring the tissue was fully submerged. Samples were heated at 95°C on a heat block for 10 minutes before vortexing. Dilution solution (100 µl, Sigma) was added and the sample was vortexed thoroughly and placed on ice.

The PCR reaction mix consisted of the following components: 10 µl PCR ready mix (REDExtract-N-Amp, Sigma) 0.8 µl forward primer, 0.8 µl reverse primer, 2.8 µl H₂O, 4 µl DNA extract (from above). PCR conditions were 94°C, 4 min, followed by 33 cycles of 94°C 30 sec, 62°C 30 sec, 72°C 1 min followed by 72°C for 10 min. Samples were stored at 4°C.

Primers:
nptII forward: GTGGAGAGGCTATTCGGCTATGAC - SEQ ID NO:7
nptII reverse: CGGGTAGCCAACGCTATGTCC - SEQ ID NO:8
StPot 1A forward: GCTCTAGAAAGGAATCGGAATCTGAATC - SEQ ID NO:9
StPot 1A reverse: GCTCTAGAATTCTTCTTTTCTTCAGCCACCCTAGGAATTTG - SEQ ID NO:10

### Detection of NaPI and StPot1A in transgenic cotton

### ELISA

Protein extract: leaves were excised from plants grown either in the growth cabinet or in the glasshouse. The tissue (100 mg) was frozen in liquid nitrogen and ground in a mixer mill (Retsch MM300) for 2 x 15 sec at frequency 30. 1 mL of 2% insoluble PVP (Polyclar)/PBS/0.05% Tween 20 was added prior to vortexing for 20 sec. The samples were centrifuged for 10 min and the supernatant was collected.

Coat ELISA plate (Nunc Maxisorp #442404) with 100 µL/well of primary antibody in PBS.

100ng/well of anti-NaPI (polyclonal antibody was made by a standard method to purified NaPI peptides isolated from stigmas) or anti-Pot 1A (antibody made to Pot 1A that was expressed as a dimer with C1 in *E. coli* and then cleaved and separately purified), Incubate overnight at 4°C in a humid box. Wash plates 2 min x 4 with PBS/0.05 % Tween 20. Block plate with 200 µL/well 3 % BSA (Sigma A- 7030: 98% ELISA grade) in PBS. Incubate for 2 hr at 25°C. Wash plates 2 min x 4 with PBS/0.05 % Tween 20. The anti-NaPI antibody binds to the T and C protease inhibitors of *N. alata.*

Apply 100 µL/well of cotton protein extracts (diluted in PBS/0.05 % Tween 20). Incubate 2hr at 25°C. Wash plates 2 min x 4 with PBS/0.05 % Tween 20. Apply 100 µL/well of secondary antibody in PBS (50ng/well biotin-labelled NaPI antibody, 200ng/well biotin-labelled Pot 1A antibody). Incubate for 1 hr at 25°C. The biotin labelled antibody is prepared using the EZ-link Sulfo-NHS-LC-biotinylation kit (Pierce). Use 2 ml of protein A purified antibody and 2 mg of the biotin reagent.

Wash plates 2 min x 4 with PBS/0.05 % Tween 20. Apply 100 µL/well NeutriAvidin HRP-conjugate (Pierce #31001; 1:1000 dilution; 0.1 µL/well) in PBS. Incubate for 1 hr at 25°C.

Wash plates 2 min x 4 with PBS/0.05 % Tween 20, followed by 2 min x 2 with H₂O. Just before use, prepare substrate by dissolving 1 ImmunoPure OPD tablet (Pierce #34006) in 9 mL H₂O, then add 1 mL stable peroxide buffer (10x, Pierce #34062). Add 100 µL/well substrate. Incubate at 25°C until colour develops. Stop reaction with 50 µL 2.5 M sulfuric acid. Measure absorbance at 490 nm in plate reader (Molecular Devices, Milenia Kinetic Analyzer).

### Immunoblot analysis

Leaves were excised from plants grown either in the growth cabinet or in the glasshouse. Leaf tissue (100 mg) was frozen in liquid nitrogen and ground to a fine powder in a mixer mill (Retsch MM300), for 2 x 15 sec at frequency 30. The powder was added to 2 x sample buffer (300 µl, Novex NuPAGE LDS sample buffer, 10% v/v β- mercaptoethanol), vortexed for 30 sec, boiled for 5 min and then centrifuged at 14,000 rpm for 10 min and the supernatant retained for SDS-PAGE. Alternatively, the powder was added to 1 ml acetone, vortexed thoroughly and centrifuged at 14,000 rpm (18,000g) for 2 min and the supernatent discarded. The pellet was resuspended in 300 µl of IP lysis buffer (50mM Tris pH 8, 5mM EDTA, 150mM NaCl, 0.1% Triton X-100) with 2% Polyclar AT (water-soluble polyvinyl polypyrrolidine) by vortexing thoroughly and supernatant was collected after centrifugation at 14,000 rpm for 10 min. For analysis by SDS-PAGE, 30 µl of sample in 1 X sample buffer (Novex NuPAGE LDS sample buffer) and 5% v/v β-mercaptoethanol was used.

Extracted leaf proteins were separated by SDS-PAGE on preformed 4-12% w/v polyacrylamide gradient gels (Novex, NuPAGE bis-tris, MES buffer) for 35 min at 200V in a Novex X Cell II mini-cell electrophoresis apparatus. Prestained molecular weight markers (Novex SeeBlue Plus 2) were included as a standard. Proteins were transferred to nitrocellulose membrane (Osmonics 0.22 micron NitroBind) for 60 min at 30V using the Novex X Cell mini-cell electrophoresis apparatus in NuPAGE transfer buffer with 10% v/v methanol. After transfer, membranes were incubated for 1 min in isopropanol, followed by a 5 min wash in TBS.

The membrane was blocked for 1 h in 3% w/v BSA at RT followed by incubation with primary antibody overnight at RT (NaPI antibody: 1:2000 dilution in TBS/1%BSA of 1mg/ml stock, Pot 1A antibody: 1:1000 in TBS/1%BSA of 1mg/ml stock). The membrane was washed 5 x 10 min in TBST before incubation with goat anti-rabbit IgG conjugated to horseradish peroxidase for 60 min at RT (Pierce, 1:100,000 dilution in TBS). Five further 10 min TBST washes were performed before the membrane was incubated with the SuperSignal West Pico Chemiluminescent substrate (Pierce) according to the Manufacturer's instructions. Membranes were exposed to ECL Hyperfilm (Amersham).

### Results

From 2 experiments (CT 89 and CT 90) we produced 86 potential transgenic plants. All plants were screened by PCR using the npt primers and the StPotlA primers. Plants positive for npt II were assessed for NaPI protein expression by ELISA. 38 plants were expressing detectable levels of NaPI (Figure 4).

Line 89.5.1 was selfed and the T2 progeny seed grown and the plants assessed for NaPI expression by ELISA. 20 of the 27 plants (74%) were expressing NaPI and 7 plants (26%) were null segregants (Fig. 4C) demonstrating that the genes had been transferred to the next generation in a heritable manner.

Immunoblot analysis of selected lines using the NaPI antibody confirmed that the precursor protein and the processed peptides were present (Fig. 4D and 4E). However, detection of Potl was unsuccessful suggesting that detection sensitivity in the assay was not sufficient.

The results demonstrate that a MGEV encoding four peptides, at least one of which is not a type 2 protease inhibitor, can be constructed using conventional methods and used to successfully transform a plant (cotton) of a different species than that from which any of the component DNA segments were derived. The encoded protein is expressed and post-translationally processed to yield component peptides of the expected size.

### EXAMPLE 2

### Construction and expression of a linear MGEV having one type one PI and 2 potato type two Pis

The MGEV described in this example (MGEV-8) has the structure diagrammed as:

S-D₁L₁D₂L₂D₃L₃-V

where D₁ is T1 of NaPI-ii-SEQ ID NO:2. aa 112-164
D₂ is potato Pot 1A - SEQ ID NO:11
D₃ is C1 or amino acids 200 to 252 of SEQ ID NO:2
L₁ and L₂ and L₃ are each EEKKN (SEQ ID NO:5)
S is the signal peptide of NaPI-iv- SEQ ID NO:2. aa 1-29
and V is the vacuole targeting peptide of NaPI-iv - SEQ ID NO:2, aa 258-281

A linear MGEV (MGEV-8) (Fig. 6A) was constructed as follows. The signal sequence of NaPI-iv SEQ ID NO:2, aa 1-29 was PCR-amplified with a Bam H1 site at the 5' end and a Xho 1 site at the 3' end. The vacuole targeting peptide of NaPI-iv was PCR-amplified with a Xho 1 site at the 5' end and a Sal 1 site at the 3' end. These DNA fragments were ligated together into pAM9 cut with Bam H1 and Sal 1 (see Example 1).

The Xho 1-flanked T1-Xba 1-C1 fragment was cut from the multipurpose vector pRR20 (see Example 3) and ligated into the S-Xho 1-V construct described above, resulting in a S-Xho 1-T1-Xba 1-C1-Xho 1-V construct. This linear multipurpose vector was designated pSP1.

The mature domain of potato Pot 1A (see Example 1) was PCR-amplified with an EEKKN linker sequence (SEQ ID NO: 5) at the 3' end and with Xba 1 sites at both ends. This was then ligated into the Xba 1 site of pSP1 to produce MGEV-8 (Fig. 6A). MGEV-8 was inserted into pBIN19 to produce the vector pHEX 56, diagrammed in Fig. 5.

### Transient expression in cotton cotyledons

pHEX 56 was introduced into *A. tumefaciens* and the expression of T1, C1 and Pot 1A was determined by a transient assay with cotton cotyledons.

Bacterial "lawns" of the *Agrobacterium* were spread on selective plates and grown in the dark at 30 °C for 3 days. Bacteria were then resuspended to an OD600 of 1.0 in infiltration buffer (10 mM magnesium chloride and 10 µM acetosyringone (0.1 M stock in DMSO)) and incubated at room temperature for 2-4 h. Cotton plants were grown for 8 days in a controlled temperature growth cabinet (25 °C, 16 h/8 h light/dark cycle). The underside of the cotyledons was infiltrated by gently pressing a 1 mL syringe against the leaf and filling the leaf cavity with the *Agrobacterium* suspension. The area of infiltration (indicated by darkening) was noted on the topside of the leaf. A maximum of 4 infiltrations were performed per cotyledon. Plants were grown for a further 4 days. The infiltrated areas were then cut out, weighed and frozen in liquid nitrogen. Protein expression was determined by ELISA and immunoblots as described in Example 1.

### Results

NaPI (Fig. 6B) and Pot 1A (Fig. 6C) were detected by ELISA in cotton cotyledons. Immunoblot analysis using the NaPI antibody confirmed that the precursor protein and the processed peptides were present (Fig. 6D).

The results confirm previous conclusions from Example 1 and demonstrate, in addition, expression of Pot1A. The results also demonstrate that cyclization of a primary MGEV expression product is not required for processing to yield predicted component peptides.

### EXAMPLE 3

### Construction and expression of an MGEV having one defensin and 3 potato type two PIs

Note: In Examples 3-16, linker peptides (L) are omitted from the MGEV diagram in order to simplify the diagram.

The MGEV described in this example (MGEV-6) has the structure diagrammed as: (See also Fig. 8A).

MGEV-6, expressing a defensin and 3 potato type two PI's, was constructed essentially as described for MGEV-5 (Example 1) except that a modified multipurpose vector (pRR20) was used and a defensin coding sequence was inserted instead of Pot 1A. The defensin was NaD1 as described in U.S. Patent 7,041,877, and herein SEQ ID NO:14, amino acids 26-72, having a mature defensin domain but lacking the C-terminal acidic peptide tail, and without the N-terminal signal peptide.

The modified multipurpose vector (pRR20) is the same as the multipurpose vector (pRR19) described in Example 1, except that the codon encoding N in the EEKKN linker (SEQ ID NO:5) (L₁) of the Xho1-T1-L₁-Xbal DNA fragment was changed from AAT to AAC SEQ ID NO:12. This deleted an undersired Eco R1 restriction site that was present in pRR19.

NaD1 DNA was ligated into the Xba 1 site of pRR20, then excised with Bam H1 and Sal 1 and the complete fragment inserted into pAM9 to produce MGEV-6. MGEV-6 was then inserted into pBIN19 to produce the vector pHEX31, diagrammed in Fig. 7.

### Transformation of cotton

Cotton transformation with pHEX31 was carried out as described in Example 1.

### Protein Detection

Protein expression was determined by ELISA as described in Example 1. The primary NaD1 antibody and the secondary NaD1-biotin antibody were used at 50 ng/well.

Immunoblot analysis was carried out as described in Example 1 with the modification described in Example 2. The primary NaD1 antibody was diluted 1:1,000 dilution from a 1mg/ml stock arid the secondary antibody (goat anti-rabbit IgG conjugated to horseradish peroxidase) was used at a 1:50,000 dilution.

### Results

From one experiment (CT 93) 88 potential transgenic plants were produced. All plants were screened by PCR using the *nptII* primers and primers specific for NaD1. 57 plants were positive for the presence of the *nptII* gene, with 33 of these plants also carrying the NaD1 gene. PCR positive plants were assessed for NaPI and NaD1 protein expression by ELISA. 13 primary transgenic plants were expressing detectable levels of NaPI and NaD1.

Three transgenic lines (93.4, 93.36 and 93.279) were selected for further study. The primary transgenic lines were selfed and the T2 seed collected. T2 plants from two of these lines (93.4 and 93.279) were assessed for NaPI expression (Figs. 8B, 8D) and NaD1 expression (Figs. 8C, 8E) by ELISA. Both lines produced a segregating population consistent with genes being transferred in a Mendelian manner.

Immunoblot analysis of lines 93.4 and 93.36 using the NaPI antibody confirmed that the precursor protein and the processed peptides were present (Fig. 8F). Further analysis of line 93.4 with the NaD1 antibody confirmed that the mature NaD1 protein was present (Fig. 8G), although at low levels.

The results demonstrate utility of MGEV for simultaneously expressing a protein other than a protease inhibitor (NaD1, a defensin).

### EXAMPLE 4

### Construction and expression of an MGEV having one GFP and 3 potato type two PI's

The MGEV described in this example (MGEV-7) has the structure diagrammed as: (See also Fig. 10A)

MGEV-7 has a similar structure to MGEV-5 (Example 1) except that a DNA sequence encoding a Green Fluorescent Protein (GFP) was inserted in place of Pot 1A. The GFP is a soluble, highly fluorescent variant of green fluorescent protein (GFP) for use in higher plants (Davies, SJ and Vierstra, RD: Plant Mol. Biol. 36(4): 521-528 (1998). The DNA was obtained from TAIR (the Arabidopsis information resource) (SEQ ID NO:13). Sequence information is available from Genbank at accession number U70495, and herein at SEQ ID NO:13.

For construction of MGEV-7, a third multipurpose vector (pRR21) was used. This was made in the same way as pRR20 except that the DNA encoding the C1 domain of NaPI-iv was PCR-amplified with an extra EEKKN linker sequence (SEQ ID NO:5) at the 3' end resulting in an Xba1-L-C1-L-Xho1 DNA fragment. pRR21 has the following structure: S-C2_{N}-L-Xho1-T1-L-Xba1-L-C1-L-Xho1-C2_{C}-V. In addition this construct was inserted into pAM9 before additional insertions were made. The DNA sequence encoding GFP was PCR-amplified with Xba1 ends (no 3' linker sequence) and inserted into the Xba1 site between T1 and C1 of pRR21 to produce MGEV-7. MGEV-7 was inserted into pBIN19 to produce the vector pHEX 46, diagrammed in Fig. 9.

### Transient expression in tobacco leaves

pHEX 46 was introduced into A. *tumefaciens* and the expression of T1, C1 and GFP was determined by a transient assay with tobacco leaves. The method was that essentially described in Example 2 for cotton cotyledons except that *Nicotiana benthamiana* plants were grown for 5 weeks in a controlled temperature growth cabinet (25 °C, 16 h/8 h light/dark cycle). The underside of leaves (4-6 nodes from the top, 6-10 cm in maximum width) was infiltrated by gently pressing a 1 mL syringe and filling the leaf cavity with the *Agrobacterium* suspension. Four to. six infiltrations were made on each leaf. Plants were grown for a further 4 days. The infiltrated areas were then cut out, weighed and frozen in liquid nitrogen. Protein expression was determined by immunoblots as described in Example 1.

### Transient expression in cotton cotyledons

Expression of pHEX 46 was also determined in a transient assay with cotton cotyledons as previously described in Example 2.

### Protein detection

Expression of NaPI was determined by ELISA as described in Example 1.

Immunoblot analysis was carried out as described in Example 1 with the modification described in Example 2.

### Microscopy

Three days after infiltration with A. *tumefaciens* the *N. benthamiana* and cotton plants were placed in the dark for 24h. The infiltrated leaf areas were then removed and epidermal peels (∼5mm²) were prepared. Small pieces (1-2mm² of the epidermal or mesodermal tissue were placed on a glass slide with water as a mounting medium. A cover slip was placed over the top and sealed with hot wax. The sections were examined for GFP fluorescence using an Olympus BX50 fluorescence microscope. A W1B filter (excitation range 460-490nm) was used for fluorescence excitation and a long pass filter which detects signals at 515nm plus was used for emission. GFP fluorescence was also examined using a Leica TCS SP2 confocal laser-microscope. The Argon laser excitation wavelength was 488nm;GFP emission was detected with the filter set for FITC (505-530nm).

### Results

Several transient assays with both tobacco leaves and cotton cotyledons were conducted. NaPI was detected by ELISA in cotton cotyledons (Fig. 10B). Immunoblot analysis using the NaPI antibody confirmed that the processed peptides were present in cotton cotyledons (Fig. 10C).

Immunoblot analysis of tobacco leaf extracts after transient expression confirmed that the GFP protein was present (Fig. 10D). The GFP and the NaD1 antibodies both bound to a protein of about 50kDa which is consistent with the expected size of the precursor protein encoded by pHEX 46. The GFP antibody also highlighted a protein of ∼28kDa which is the same size as bacterially expressed GFP and thus represents GFP that has been proteolytically excised from the precursor encoded by pHEX 46.

GFP produced from transient expression of MGEV-7 in the epidermal cells of cotton leaves was located in the vacuole (Fig. 10E). This contrasted to GFP fluorescence produced from a construct (MGEV-7A) that was identical to MGEV-7 except the vacuole targeting peptide (V) was deleted (see example 7). Transient expression of MGEV-7A resulted in an extracellular location for the GFP fluorescence (Fig. 10F).

The results demonstrate that proteins of disparate sizes can be expressed as a polyprotein using a MGEV, and correctly processed after translation to yield individual protein components. In this example, 4 proteins ranging in size from ∼6kDa to ∼28kDa were affectively expressed together and correctly processed. A single vacuole targeting sequence resulted in transfer of each expressed protein to the cell vacuole prior to processing. The use of GFP in a MGEV is therefore a convenient means to indicate intracellular location of proteins co-expressed in a MGEV.

### EXAMPLE 5

### Construction and expression of an MGEV having one defensin, one type one PI and 3 potato type two Pis

The MGEV described in this example (MGEV-9) has the structure diagrammed as: (See Fig. 12A).

MGEV-9 expressing six proteins, a defensin, two potato type one PI's and 3 type two PI's was constructed using the following method. NaD1 was prepared as per Example 3. The Pot 1A dimer was constructed by splice overlap PCR. The first Pot 1A was PCR-amplifed with a 5' XbaI site and a 3' linker sequence. The second Pot 1A was PCR-amplified with linker sequences at both ends and a 3' XbaI site. The two PCR fragments were annealed to each other and extended for 8 cycles; outer primers were then added to PCR-amplify the dimer sequence. The NaD1 and Pot 1A dimer fragments were inserted into the Xba 1 site of pSP1 (Example 2) in a 3 way ligation. The new larger fragment (T1-NaD1-Pot 1A-Pot 1A-C1) was cut at the Xho 1 sites to produce MGEV-9. MGEV-9 was inserted into pBIN19 to produce the vector pHEX55, diagrammed in Fig. 11.

### Transient expression in cotton cotyledons

Expression of pHEX55 was determined in a transient assay with cotton cotyledons as previously described in Example 2.

### Protein detection

Expression of NaPI was determined by ELISA as described in Examples **1, 2 and 3.**

Immunoblot analysis was carried out as described in Example 1 with the modification described in Example 2.

### Results

NaPI (Fig. 12B), NaD1 (Fig. 12C) and Pot 1A (12D) were detected by ELISA in cotton cotyledons. Immunoblot analysis using the NaPI antibody confirmed that the precursor protein and the processed NaPI 6 kDa peptides were present (Fig. 12E).

The results demonstrate simultaneous expression and correct processing of several different proteins in a 6-domain circular MGEV.

### EXAMPLE 6

### Construction and expression of an MGEV that targets proteins to the extracellular space in plant tissues.

The MGEV described in this example has the structure diagrammed as: (See Fig. 14A, MGEV 10).

This MGEV (MGEV-10) was essentially the same as MGEV-7 (Example 4) except that it did not have the NaPI vacuole targeting peptide (V) and the multipurpose vector pRR20 was used (Example 3). pRR20 was PCR-amplified using a reverse primer which excluded the vacuole targeting peptide (V). XbaI-flanked GFP was then ligated into the XbaI site. Details of the GFP are given in Example 4. The fragment (S - C2_{N} - T1 - GFP - C1 - C2_{C}) was then inserted into pAM9 to produce MGEV-10. MGEV-10 was then inserted into pBIN19 to produce the vector pHEX45, diagrammed in Fig. 13.

### Transient expression assays

Expression of pHEX45 was determined in transient assays with tobacco leaves and cotton cotyledons as described in Example 4. Protein expression was determined by immunoblots as described in Example 4. Two non-MGEV constructs C1 and C2 (Fig. 14A) were used as controls. These constructs employed the same promoters and terminators as the MGEV constructs and were cloned into the same vectors for expression in plant cells. The coding sequence of C1 contained GFP with the signal sequence (S) from the MGEV. The second control construct (C2) encoded GFP with the endoplasmic reticulum signal sequence (S) and the vacuolar targeting sequence (V) from the MGEV. The location of the GFP in the plant tissue was confirmed by microscopy as described in Example 4.

### Results

Several transient assays with tobacco leaves were conducted. Immunoblot analysis of tobacco leaf extracts after transient expression confirmed that the GFP protein was produced from both the control constructs (C1 and C2) and was the same size as the 28kDa bacterially expressed GFP (Fig 14F). Additionally, the C2 construct produced another slightly larger protein that corresponds to GFP plus the vacuolar targeting sequence (V). The GFP-antibody detected proteins of ∼50kDa and 28kDa in extracts from leaves that were expressing MGEV-7 and MGEV-10. The 50kDa protein also bound to the NaPI antibody as expected for the unprocessed product encoded by the MGEV. The presence of the 28kDa protein which corresponds to free GFP is consistent with processing of the linker in the MGEV to release the individual PI and GFP domains.

GFP produced from transient expression of MGEV-7 in the epidermal cells of cotton leaves was located in the vacuole (Example 4, Fig. 10E). This contrasted to GFP fluorescence produced from the construct that was identical to MGEV-7 except the vacuole targeting peptide (V) was deleted (MGEV-10). Transient expression from MGEV-10 resulted in an extracellular location for the GFP fluorescence (Example 4, Fig. 10F).

GFP was also directed extracellularly when MGEV-10 was expressed transiently in the leaves of *N. benthamiana.* Figs 14B, C, D and E show the confocal images obtained when MGEV-10 and a control construct that encodes only GFP and a signal peptide (C1) were expressed in *N. benthamiana.* Both constructs lack the vacuolar targeting sequence (V) and hence the GFP was secreted outside both epidermal and mesophyll cells and was not directed to the vacuole.

The results confirm and amplify those obtained in Example 4. Vacuolar targeting of GFP was observed regardless of whether the targeting sequence was directly attached to GFP protein or to the unprocessed MGEV protein.

### EXAMPLE 7

### Construction and expression of an MGEV having one defensin with CTPP and 3 potato type two Pis

The MGEV described in this example (MGEV-11) has the structure diagrammed as: (See also Fig. 16A).

A MGEV expressing a defensin and 3 potato type two PI's was constructed, essentially as described for MGEV-7 (Example 4) except that NaD1 defensin included the C-terminal acidic peptide tail. NaD1CTPP, SEQ ID NO:14 amino acids 26-105 was inserted into the Xba1 site of the multipurpose vector pRR21 (Example 4) to produce MGEV-11. MGEV-11 was then inserted into pBIN19 to produce the vector pHEX 42, diagrammed in Fig. 15.

### Transient expression in cotton cotyledons

Expression of pHEX 42 was determined in a transient assay with cotton cotyledons as described in Example 2.

### Protein detection

Protein expression was determined by ELISA and immunoblots as described in Example 4.

### Results

NaPI (Fig. 16B) and NaD1 (Fig. 16C) were both detected by ELISA in cotton cotyledons transfected with pHEX42. Immunoblot analysis using the NaPI antibody confirmed that the precursor protein and the processed NaPI 6 kDa peptides were present (Fig. 16D). The precursor protein and the NaD1 protein could also be detected by the NaD1 antibody (Fig 16E). The processed protein was the correct size for the mature NaD1 protein (∼ 6 kDa) indicating that the CTPP tail had been correctly processed (Fig. 16E).

The results demonstrate expression and correct processing of NaD1 having its own vacuolar targeting sequence (CTPP), in addition to the vacuole targeting sequence of the MGEV.

### EXAMPLE 8

### Construction and expression of an MGEV having two type one Pis and 3 potato type two Pis

The MGEV described in this example has the structure diagrammed as: (See Fig. 18A).

A MGEV expressing two potato type 1 PIs and 3 potato type two PI's was constructed, using pSP2 (Example 6). The Pot 1A dimer was produced as described in Example 5 and inserted into pSP2 to produce MGEV-12. MGEV-12 was then inserted into pBIN19 to produce the vector pHEX 33, diagrammed in Fig. 17.

### Transient expression in cotton cotyledons

Expression of pHEX33 was determined in a transient assay with cotton cotyledons as described in Example 2.

### Protein detection

Protein expression was determined by ELISA.

### Results

NaPI (Fig. 18B) and Pot 1A (Fig. 18C) were both detected by ELISA in cotton cotyledons transfected with pHEX 33. The expression of Pot 1A was significant as expression of Pot 1A using pHEX29 (which only has one copy of the gene) could not be detected in the transient assay (data not shown).

The results indicate that Pot1A is expressed in a MGEV and correctly processed in concert with other proteins.

### EXAMPLE 9.

### Construction and expression of an MGEV having two defensins and 3 potato type two Pis

The MGEV described in this example has the structure diagrammed as: (See Fig. 20A).

A MGEV expressing one class one defensin (NaD2) SEQ ID NO:15 and 16, one class two defensin (NaD1) SEQ ID NO:14, amino acids 26-72, and 3 type two Pi's was constructed, essentially as described for MGEV-7 (Example 4) except that two defensins were inserted instead of GFP (see Lay, F.T., et al., (2005), Current Proteins and Peptide Science 6:85-101 for definition of one and class two defensins). NaD1 is described in Example 3. The NaD2 - NaD1 dimer was constructed by splice overlap PCR. NaD2 was PCR-amplified with a 5' XbaI site and a 3' linker sequence. NaD1 was PCR-amplified with a linker sequence at the 5' end and a 3' XbaI site. The two PCR fragments were annealed to each other and extended for 8 cycles; outer primers were then added to PCR-amplify the dimer sequence. The NaD2 - NaD1 dimer was.inserted into pRR21 to produce MGEV-13. MGEV-13 was then inserted into pBIN19 to produce the vector pHEX39, diagrammed in Fig. 19.

### Transient expression in cotton cotyledons

Expression of pHEX39 was determined in a transient assay with cotton cotyledons as described in Example 2.

### Protein detection

Protein expression was determined by ELISA as described in Example 3.

### Results

NaPI (Fig. 20B) and NaD1 (Fig. 20C) were both detected by ELISA in cotton cotyledons transfected with pHEX39.

The results demonstrate the value of using MGEV to express a plurality of plant protective proteins simultaneously.

### EXAMPLE 10

### Construction and expression of a linear MGEV having two type one PI s and 2 potato type two Pis

The MGEV described in this example has the structure diagrammed as:
S-T1-Pot1A-Pot1A-C1-V
(See Fig. 22A).

A linear MGEV expressing two potato type 1 Pis and 2 potato type two PI's was constructed, essentially as described for MGEV-8 (Example 2) except that two Pot 1As were inserted. The Pot 1A - Pot 1A dimer was produced by PCR overlap as described in Example 5 and inserted into the linear multipurpose vector pSP1 (Example 2) to produce MGEV-14. MGEV-14 was then inserted into pBIN19 to produce the vector pHEX 48, diagrammed in Fig. 21.

### Transient expression in cotton cotyledons

Expression of pHEX48 was determined in a transient assay with cotton cotyledons as described in Example 2.

### Protein detection

Protein expression was determined by ELISA and immunoblots as described in Example 2.

### Results

NaPI (Fig. 22B) and Pot 1A (Fig. 22C) were both detected by ELISA in cotton cotyledons transfected with pHEX 48. Expression levels of Pot 1A were similar to those produced in cotton cotyledons transfected with pHEX 33 which also contains 2 copies of the Pot 1A gene (Example 8). Immunoblot analysis using the NaPI antibody confirmed that the processed NaPI 6 kDa peptides were present (Fig. 22D).

The results demonstrate that expression of a linear MGEV protein is at least as effective for expressing multiple proteins as the circular form. MGEV efficacy does not depend on the presence of a "clasp" protein.

### EXAMPLE 11

### Construction and expression of a linear MGEV having one defensin and 2 potato type two Pis

The MGEV described in this example has the structure diagrammed as:
S - T1 - NaD1 - C1 - V
(See Fig. 24A).

A linear MGEV expressing one defensin (NaD1) SEQ ID NO:14 amino acids 26-72 and 2 potato type two PI's (T1 and C1) was constructed, essentially as described for MGEV-8 (Example 2) except that a defensin (NaD1) was inserted instead of Pot 1A. NaD1 (described in Example 3) was inserted into the linear multipurpose vector pSP1 (Example 2) to produce MGEV-15. MGEV-15 was then inserted into pBIN19 to produce the vector pHEX 47, diagrammed in Fig. 23.

### Transient expression in cotton cotyledons

Expression of pHEX47 was determined in a transient assay with cotton cotyledons as described in Example 2.

### Protein detection

Protein expression was determined by ELISA. and immunoblots as described in Example 3.

### Results

NaPI (Fig. 24B) and NaD1 (Fig. 24C) were both detected by ELISA in cotton cotyledons transfected with pHEX 47. Immunoblot analysis using the NaPI antibody confirmed that the processed NaPI 6 kDa peptides were present (Fig. 24D).

The results further demonstrate efficacy of simultaneously expressing multiple proteins having disparate functions using a linear MGEV lacking coding sequences for cyclization of the expressed poly-protein.

### EXAMPLE 12

### Construction and expression of a linear MGEV having two potato type one PIs

The MGEV described in this example has the structure diagrammed as:
S - ProPot 1A - Pot 1A
(See Fig. 26A).

A linear MGEV expressing 2 potato type one Pis was constructed by splice overlap PCR. The first fragment consisting of the Pot 1A signal sequence, prodomain (SEQ ID NO:20) (Pro) and mature domain Pot1A (SEQ ID NO:11, herein) was PCR amplified with a 5' Bam H1 site and a 3' linker sequence. The second fragment consisting of the mature Pot 1A was PCR amplified with a 5' linker sequence and a stop codon (TAA) followed by a Sal 1 site at the 3' end. The two PCR fragments were annealed to each other and extended for 8 cycles; outer primers were then added to PCR-amplify the complete sequence. The S - ProPot 1A - Pot 1A fragment was then inserted into pAM9 to produce MGEV-16. MGEV-16 then inserted into pBIN19 to produce the vector pHEX35, diagrammed in Fig. 25.

### Transient expression in cotton cotyledons

Expression of pHEX35 was determined in a transient assay with cotton cotyledons as described in Example 2.

### Protein detection

Expression of Pot 1A was determined by ELISA as described in Example 1 except that a different Pot 1A antibody was used. The antibody was produced using a bacterially expressed C1-PotIA dimer (the C1 domain is from NaPlii SEQ ID NO:1 aa 54 to 106) and can detect both the C1 and Pot1A proteins. This antibody is better at detecting Pot 1A than the Pot 1A specific antibodies described in Examples 1 and 2, however the C1-Pot 1A antibody can only be used when Pot 1A protein is expressed without the presence of the NaPI peptides. The primary C1-Pot 1A antibody and the secondary C1-Pot 1A-biotin antibody were used at 100 ng/well.

An immunoblot to detect Pot 1A was carried out as described in Example 1 with the modification described in Example 2. The primary C1-Pot 1A antibody was diluted 1:2,000 dilution from a 1mg/ml stock and the secondary antibody (goat anti-rabbit IgG conjugated to horseradish peroxidase) was used at a 1:50,000 dilution.

### Results

Pot 1A was detected by ELISA in cotton cotyledons transfected with pHEX 35 (Fig. 26B). Pot 1A expression was higher with this linear construct containing two copies of the Pot 1A gene compared to Pot 1A expression produced by a single copy of the Pot 1A gene (Fig. 26B). For comparison, expression of Pot1A as a single gene (not MGEV) was measured using the vector pHEX6 (see published application WO 2004/094630, Example 6). CaMV 35S promoter was used to drive expression in both pHEX6 and pHEX35.

Immunoblot analysis using the C1-Pot 1A antibody confirmed that the Pot 1A protein was present (Fig. 26C). Two other Pot 1A specific bands were detected in this sample. The band at approximately 20 kDa is probably the precursor protein. The band at around 49 kDa may be an aggregation of the Pot1A mature protein as it has been reported that the native Potl protein from potato tubers forms a oligomer.

The results further corroborate expression of Pot1A in a MGEV-like structure and show that the propeptide on Pot 1 which is a vacuolar targeting sequence is proteolytically removed.

### EXAMPLE 13

### Construction and expression of a linear MGEV having one potato type two PI and 1 defensin

The MGEV described in this example has the structure diagrammed as:
S - T1 - NaD1CTPP
(See Fig. 28A).

A linear MGEV expressing one potato type two PI (T1) and one defensin (NaD1) with C-terminal tail (CTPP) was constructed. NaD1CTPP (See example 7) was PCR amplified with a 5' Xba 1 site and a 3' Sal 1 site. This fragment was inserted into the Xba 1 - Sal 1 cut site of pSP1 (with C1-V removed) to produce MGEV-17. MGEV-17 was then inserted into pBIN19 to produce the vector pHEX41, diagrammed in Fig. 27.

### Transient expression in cotton cotyledons

Expression of pHEX41 was determined in a transient assay with cotton cotyledons as described in Example 2.

### Protein detection

Expression of NaD1 was determined by ELISA and immunoblots as described in Example 3.

### Results

NaPI and NaD1 were detected by ELISA in cotton cotyledons transfected with pHEX41 (Figs. 28B, C and D). Expression of NaD1 from this linear construct in which the NaD1 CTPP is linked to T1 is significantly higher than the expression of NaD1 CTPP alone (pHEX3) (see U.S. 7,041,877) in a transient cotton assay when both are driven by the 35S promoter (Fig. 28 B). CTPP targets NaD1 to the vacuole where it is proteolytically removed to release the mature ∼6kDa NaD1.

immunoblot analysis using the NaPI antibody confirmed that the processed NaPI 6 kDa peptides were present (Fig. 28E). The NaD1 antibody detected both the 16 kDa precursor and the mature NaD1 ∼6 kDa protein confirming correct processing of the linker between T1 and NaD1 and correct processing of the CTPP tail (Fig. 28D).

### EXAMPLE 14

### Construction and expression of a linear MGEV having one class 1 defensin and one class two defensin

The MGEV described in this example has the structure diagrammed as:
S - NaD2 - NaD1 CTPP

A linear MGEV expressing one class one defensin (NaD2) and one class two defensin (NaD1 with C-terminal tail) was constructed by splice overlap PCR essentially as described in Example 13 except that two defensins were used. NaD2 is described in Example 10 and NaD1-CTPP is described in Example 7. The first fragment consisted of the signal sequence and the coding sequence for NaD2, the second fragment consisted of the mature NaD1 and the CTPP tail from NaD1. Following PCR, the full fragment (S - NaD2 - NaD1 CTPP) was inserted into pAM9 to produce MGEV-18. MGEV-18 was then inserted into pBIN19 to produce the vector pHEX52, diagrammed in Fig. 29. A diagram of MGEV-18 is shown in Fig. 30A.

### Transient expression in cotton cotyledons

Expression of pHEX52 was determined in a transient assay with cotton cotyledons as described in Example 2.

### Protein detection

Expression of NaD1 was determined by ELISA as described in Example 3.

### Results

NaD1 was detected by ELISA in cotton cotyledons transfected with pHEX52 (Fig. 30B). The results demonstrate that a plurality of different proteins can be expressed in a linear MGEV in the absence of any type two PI.

### EXAMPLE 15

### Construction and expression of a linear MGEV having one class 1 defensin and one class two defensin (CTPP deteted)

The MGEV described in this example has the structure diagrammed as:
S - NaD2 - NaD1

A linear MGEV expressing one class one defensin (NaD2) and one class two defensin (NaD1) but lacking the CTPP tail was constructed as described in Example 15 except that the CTPP tail was not amplified. The S - NaD2 - NaD1 fragment was inserted into pAM9 to produce MGEV-19. MGEV-19 was then inserted into pBIN19 to produce the vector pHEX51, diagrammed in Fig. 31. A diagram of MGEV-19 is shown in Fig. 32A.

### Transient expression in cotton cotyledons

Expression of pHEX51 was determined in a transient assay with cotton cotyledons as described in Example 2.

### Protein detection

Expression of NaD1 was determined by ELISA as described in Example 3.

### Results

NaD1 was detected by ELISA in cotton cotyledons transfected with pHEX51 (Fig. 32B).

### EXAMPLE 16

### Construction and expression of a linear MGEV having one beta-Glucuronidase GUS and 2 potato type two Pis

The MGEV described in this example has the structure diagrammed as:
S - T1 - GUSC1 - V

A linear MGEV expressing one GUS and 2 potato type two PI's (T1 and C1) was constructed, essentially as described for MGEV-8 (Example 2) except that a DNA sequence encoding beta-Glucuronidase (GUS) was inserted in place of Pot 1A. GUS is an *E. coli* enzyme with a molecular mass of approximately 68,000 Da and is encoded by the *gusA* gene, SEQ ID NO:18 and SEQ ID NO:19 for GUS DNA and amino acid sequences, respectively. GUS was PCR amplified from the binary vector pBI121 (Invitrogen) with Xba 1 sites at each end, and inserted into the linear multipurpose vector pSP1 (Example 2) to produce MGEV-20. MGEV-20 was then inserted into pBIN19 to produce the vector pHEX58, diagrammed in Fig. 33. In this construct, there was no linker between GUS and C1. Expression and processing was not adversely affected.

### Transient expression in cotton cotyledons

Expression of pHEX58 was determined in a transient assay with cotton cotyledons as described in Example 2.

### Protein detection

Expression of NaPI was determined by ELISA as described in Example 1

Immunoblot analysis to detect the NaPIs was carried out as described in Example 1 with the modification described in Example 2.

### Results

NaPI was detected by ELISA in cotton cotyledons transfected with pHEX 58 (Fig. 34B).

Immunoblot analysis using the NaPI antibody confirmed that the mature NaPI peptides were present (Fig. 34C).

The results demonstrate that proteins of at least 68 kDa can be expressed in the MGEV and processed correctly.

As used herein, "comprising" is synonymous with "including," "containing," or "characterized by," and is inclusive or open-ended and does not exclude additional, unrecited elements or method steps. As used herein, "consisting of" excludes any element, step, or ingredient not specified in the claim element. As used herein, "consisting essentially of" does not exclude materials or steps that do not materially affect the basic and novel characteristics of the claim. Any recitation herein of the term "comprising", particularly in a description of components of a composition or in a description of elements of a device, is understood to encompass those compositions and methods consisting essentially of and consisting of the recited components or elements. The invention described herein suitably may be practiced in the absence of any element or elements, limitation or limitations which is not specifically disclosed herein.

In general the terms and phrases used herein have their art-recognized meaning, which can be found by reference to standard texts, journal references and contexts known to those skilled in the art. The following definitions are provided to clarify their specific use in the context of the invention.

**Table 2.**

| Example | MGEV | (fig) | Vector | (fig) |
|---|---|---|---|---|
| 1 | MGEV 5 | (4A) | pHEX 29 | (3) |
| 2 | MGEV 8 | (6A) | pHEX 56 | (5) |
| 3 | MGEV 6 | (8A) | pHEX 31 | (7) |
| 4 | MGEV 7 | (10A) | pHEX 46 | (9) |
| 5 | MGEV 9 | (12A) | pHEX 55 | (11) |
| 6 | MGEV 10 | (14A) | pHEX 45 | (13) |
| 7 | MGEV 11 | (16A) | pHEX 42 | (15) |
| 8 | MGEV 12 | (18A) | pHEX 33 | (17) |
| 9 | MGEV 13 | (20A) | pHEX 39 | (19) |
| 10 | MGEV 14 | (22A) | pHEX 48 | (21) |
| 11 | MGEV 15 | (24A) | pHEX 47 | (23) |
| 12 | MGEV 16 | (26A) | pHEX 35 | (25) |
| 13 | MGEV 17 | (28A) | pHEX 41 | (27) |
| 14 | MGEV 18 | (30A) | pHEX 52 | (29) |
| 15 | MGEV 19 | (32A) | pHEX 51 | (31) |
| 16 | MGEV 20 | (34A) | pHEX 58 | (33) |

**Table 3. Sequence ID Listings**

| **SEQ. ID NO:** | | | **(Fig)** |
|---|---|---|---|
| 1 | amino acid | Na PI-ii | (Fig. 1) |
| 2 | amino acid | Na PI-iv | (Fig. 1) |
| 3 | amino acid | N. alata T1 protease inhibitor | (Fig. 2) |
| 4 | amino acid | N. alata T5 | (Fig. 2) |
| 5 | amino acid | Linker peptide | (Fig. 2) |
| 6 | DNA | MGEV 5 | (Table 1) |
| 7 | DNA | Primer | (Example 1) |
| 8 | DNA | Primer | (Example 1) |
| 9 | DNA | Primer | (Example 1) |
| 10 | DNA | Primer | (Example 1) |
| 11 | amino acid | Pot 9A | (Example 1) |
| 12 | amino acid | MGEV5 | (Table 1) |
| 13 | amino acid | Green fluorescent protein | (Example 4) |
| 14 | amino acid | NₐD₁ | |
| 15 | DNA | NₐD₂ | |
| 16 | amino acid | NₐD₂ | |
| 17 | amino acid | Linker consensus | |
| 18 | DNA | Beta-glucuronidase | |
| 19 | amino acid | Beta-glucuronidase | |
| 20 | amino acid | Pot1A signal sequence prodomain | |

## Claims

1. A multigene expression vehicle (MGEV) comprising a polynucleotide having 2 to 8 domain segments, D, each domain encoding a functional protein, each domain being joined to the next in a linear sequence by a Linker (L) segment encoding a Linker peptide, the D and L segments all being in the same reading frame, wherein none of the domains is a type two proteinase inhibitor, and wherein each Linker has the sequence of SEQ ID NO: 5.

2. The MGEV of claim 1 wherein each domain segment, D, independently encodes a functional protein selected from a potato type I proteinase inhibitor, a plant defensin, an animal defensin, a proteinaceous toxin, a chimeric or fusion protein, an indicator protein, a heat shock protein, a Bownaan-Birk trypsin inhibitor, a cystatin, an anti-microbial peptide, a green fluorescent protein and an antibody fragment.

3. The MGEV of claim 1 or claim 2 further comprising a segment encoding a signal peptide (S) at the N-terminus of a functional protein, and optionally further comprising a segment encoding a vacuole targeting signal peptide V.

4. A MGEV according to claim 1, wherein the D and L coding segments are joined in translational order, designated as (DₖLⱼ), where k is an ordinal number for each Domain numbered from 1 to K and K is in the range from 2 to 8, and j is an ordinal number for each Linker numbered from 1 to K-1.

5. The MGEV of claim 4 further comprising a coding segment, S, encoding a signal peptide being combined in the order S-Dₖ-Lⱼ.

6. The MGEV of claim 4, further comprising a coding segment V encoding a vacuole transport peptide,
wherein V is combined with the coding segment of any of D₁-Dₖ, at either end of the segment encoding any of D₁-Dₖ.

7. A MGEV according to claim 1 or claim 2 further comprising a segment encoding an N-terminal clasp peptide, C2_{N} and a segment encoding a C-terminal clasp peptide, C2_{C}, the N-terminal and C-terminal clasp peptides being joined by disulfide bonds to one another after translation to form a single protein having protease inhibitor activity.

8. A MGEV according to claim 7, wherein the C2_{N}, D, L and C2c segments are joined in translational order, designated as C2_{N}-Lⱼ-Dₖ₊₁-C2_{C}, where k is an ordinal number for each domain numbered from 1 to K and K is in the range from 3 to 8, and j is an ordinal number for each Linker, numbered from 1 to K+1.

9. The MGEV of claim 8 further comprising a coding segment, S, encoding a signal peptide being combined in the order S-C2_{N}-Lⱼ-Dₖ-Lₖ₊₁-C2c.

10. The MGEV of claim 8 further comprising a coding segment V encoding a vacuole transport peptide, wherein V is combined with the coding segment of any of D₁-Dₖ₊₁, C2_{N} or C2c, at either end of the segment encoding any of D₁-D_{k+1;} C2_{N} or C2_{C}; or wherein V is combined with any of Dₖ, at either end of the segment encoding any of Dₖ, C2_{N} or C2c.

11. A MGEV expression vector comprising a plant transformation vector carrying and replicating a MGEV according to any one of claims 1 to 10, the MGEV being inserted at a locus in the vector that is under expression control of a plant-active promoter and a plant-active terminator.

12. A plant transformation vector comprising a MGEV according to any one of claims 1 to 10 under expression control of a plant-active promoter.

13. A plant cell containing the plant transformation vector of claim 12, and expressing proteins encoded by a MGEV according to any one of claims 1 to 10.

14. A transgenic plant, transformed by the plant transformation vector of claim 12, and concurrently expressing proteins encoded by a MGEV according to any one of claims 1 to 10.

15. A method of concurrently expressing from two to eight desired proteins in a plant cell comprising incorporating a MGEV as defined in any one of claims 1 to 10 into a plant transformation vector, transforming said vector into said plant cell and expressing said MGEV in said plant cell.

16. A method according to claim 15 of concurrently expressing from two to eight desired proteins in a plant cell comprising the steps of:
a. assembling a multi-gene expression vehicle (MGEV) as defined in claim 4;
b. combining the MGEV with a plant transformation vector, at a locus in the vector that is under expressive control of a plant-active promoter and a plant-active terminator, thereby providing a MGEV expression vector, and;
c. transforming a plant cell with the MGEV expression vector, thereby providing a MGEV-transformed cell, and;
d. maintaining the MGEV-transformed cell and progeny thereof under conditions suitable for gene expression within the cell, whereby genes encoded within MGEV-transformed cells are concurrently expressed; and optionally
regenerating an adult transgenic plant from the MGEV-transformed cell.

17. A method according to claim 15 of concurrently expressing from 3 to 8 proteins in a plant cell comprising the steps of:
a. assembling a multi-gene expression vehicle (MGEV) as defined in claim 8;
b. combining the MGEV with a plant transformation vector, at a locus in the vector that is under expression control of a plant-active promoter and a plant-active terminator, thereby providing a MGEV expression vector, and;
c. transforming a plant cell with the MGEV expression vector, thereby providing a MGEV-transformed cell, and;
d. maintaining the MGEV-transformed cell and progeny thereof under conditions suitable for gene expression within the cell, whereby genes encoded within MGEV transformed cells are concurrently expressed, and optionally
regenerating an adult transgenic plant from the MGEV-transformed cell.

18. The method of any one of claims 15 to 17 wherein the adult transformed plant is selected from the group of plants consisting of cotton, soybean, corn and rice.

19. A method according to claim 15 for concurrently expressing from two to eight proteins in a plant cell comprising transforming a plant cell with a MGEV according to claim 2, wherein the MGEV is under expression control of a single promoter.

## Patentansprüche

1. Multigen-Expressionsvehikel (MGEV), umfassend ein Polynukleotid mit 2 bis 8 Domänensegmenten, D, wobei jede Domäne für ein funktionelles Protein kodiert, jede Domäne mit der nächsten in einer linearen Sequenz durch ein Linker-(L)-Segment verbunden ist, das für ein Linkerpeptid kodiert, wobei sich die D- und L-Segmente alle im gleichen Leserahmen befinden, wobei keine der Domänen ein Proteinase-Inhibitor Typ 2 ist, und wobei jeder Linker die Sequenz von SEQ ID NO: 5 aufweist.

2. MGEV nach Anspruch 1, wobei jedes Domänensegment, D, unabhängig für ein funktionelles Protein kodiert, das aus einem Kartoffel-Proteinase-Inhibitor Typ 1, einem Pflanzendefensin, einem tierischen Defensin, einem proteinartigen Toxin, einem chimären Protein oder Fusionsprotein, einem Indikatorprotein, einem Hitzeschockprotein, einem Bowman-Birk-Trypsininhibitor, einem Cystatin, einem antimikrobiellen Peptid, einem grünen Fluoreszenz-Protein und einem Antikörperfragment ausgewählt ist.

3. MGEV nach Anspruch 1 oder Anspruch 2, weiter umfassend ein Segment, das für ein Signalpeptid (S) am N-Terminus eines funktionellen Proteins kodiert, und gegebenenfalls weiter umfassend ein Segment, das für ein auf eine Vakuole zielgerichtetes Signalpeptid V kodiert.

4. MGEV nach Anspruch 1, wobei die für D und L kodierenden Segmente in translationaler Reihenfolge miteinander verbunden sind, die als (DₖLⱼ) bezeichnet wird, wobei k eine Ordinalzahl für jede von 1 bis K nummerierte Domäne ist und K sich im Bereich von 2 bis 8 befindet und j eine Ordinalzahl für jeden von 1 bis K-1 nummerierten Linker ist.

5. MGEV nach Anspruch 4, weiter umfassend ein Kodierungssegment, S, das für ein Signalpeptid kodiert, das in der Reihenfolge S-Dₖ-Lⱼ kombiniert ist.

6. MGEV nach Anspruch 4, weiter umfassend ein Kodierungssegment V, das für ein Vakuolen-Transportpeptid kodiert,
wobei V mit dem Kodierungsegment von irgendeinem von D₁-Dₖ, an einem der beiden Enden des Segments, das für irgendeines von D₁-Dₖ kodiert, kombiniert ist.

7. MGEV nach Anspruch 1 oder Anspruch 2, weiter umfassend ein Segment, das für ein N-terminales Klammer-Peptid, C2_{N}, kodiert, und ein Segment, das für ein C-terminales Klammer-Peptid, C2_{C}, kodiert, wobei die N-terminalen und C-terminalen Klammer-Peptide nach der Translation zur Bildung eines einzelnen Proteins mit Protease-Inhibitoraktivität durch Disulfidbindungen miteinander verbunden werden.

8. MGEV nach Anspruch 7, wobei die C2_{N}-, D-, L- und C2_{C}-Segmente in translationaler Reihenfolge miteinander verbunden sind, die als C2_{N}-Lⱼ-Dₖ₊₁-C2_{C} bezeichnet wird, wobei k eine Ordinalzahl für jede von 1 bis K nummerierte Domäne ist und K sich im Bereich von 3 bis 8 befindet, und j eine Ordinalzahl für jeden von 1 bis K+1 nummerierten Linker ist.

9. MGEV nach Anspruch 8, weiter umfassend ein Kodierungssegment, S, das für ein Signalpeptid kodiert, das in der Reihenfolge S-C2_{N}-Lⱼ-Dₖ-Lₖ₋₁-C2_{C} kombiniert ist.

10. MGEV nach Anspruch 8, weiter umfassend ein Kodierungssegment V, das für ein Vakuolen-Transporfpeptid kodiert, wobei V mit dem Kodierungssegment von irgendeinem von D₁-Dₖ₊₁, C2_{N} oder C2_{C}, an einem der beiden Enden des Segments, das für irgendeines von D₁-Dₖ₊₁, C2_{N} oder C2_{C} kodiert, kombiniert ist; oder wobei V mit irgendeinem von Dₖ, an einem der beiden Enden des Segments, das für irgendeines von Dₖ, C2_{N} oder C2_{C} kodiert, kombiniert ist.

11. MGEV-Expressionsvektor, umfassend einen Pflanzentransformationsvektor, der ein MGEV nach einem der Ansprüche 1 bis 10 trägt und repliziert, wobei das MGEV an einem Locus in dem Vektor insertiert wird, der sich unter Expressionskontrolle eines pflanzenaktiven Promotors und eines pflanzenaktiven Terminators befindet.

12. Pflanzentransformationsvektor, umfassend ein MGEV nach einem der Ansprüche 1 bis 10 unter Expressionskontrolle eines pflanzenaktiven Promotors.

13. Pflanzenzelle, die den Pflanzentransformationsvektor nach Anspruch 12 enthält und Proteine exprimiert, die durch ein MGEV nach einem der Ansprüche 1 bis 10 kodiert sind.

14. Transgene Pflanze, die durch den Pflanzentransformationsvektor nach Anspruch 12 transformiert ist, und gleichzeitig durch ein MEGV nach einem der Ansprüche 1 bis 10 kodierte Proteine exprimiert.

15. Verfahren zur gleichzeitigen Expression von zwei bis acht gewünschten Proteinen in einer Pflanzenzelle, umfassend die Inkorporation eines MGEV, wie nach einem der Ansprüche 1 bis 10 definiert, in einen Pflanzentransformationsvektor, wobei der Vektor in die Pflanzenzelle transformiert und das MGEV in der Pflanzenzelle exprimiert wird.

16. Verfahren nach Anspruch 15 zur gleichzeitigen Expression von zwei bis acht gewünschten Proteinen in einer Pflanzenzelle, umfassend die Schritte von:
a. Assemblieren eines Multigen-Expressionsvehikels (MGEV), wie nach Anspruch 4 definiert;
b. Kombinieren des MGEV mit einem Pflanzentransformationsvektor, an einem Locus in dem Vektor, der sich unter Expressionkontrolle eines pflanzenaktiven Promotors und eines pflanzenaktiven Terminators befindet, wobei ein MGEV-Expressionsvektor bereitgestellt wird, und;
c. Transformieren einer Pflanzenzelle mit dem MGEV-Expressionsvektor, wobei eine MGEV-transformierte Zelle bereitgestellt wird, und;
d. Aufrechterhalten der MGEV-transformierten Zelle und Abkömmlingen davon unter Bedingungen, die zur Genexpression in der Zelle geeignet sind, wobei in MEGV transformierten Zellen kodierte Gene gleichzeitig exprimiert werden; und gegebenenfalls
Regenerieren einer adulten transgenen Pflanze aus der MGEV-transformierten Zelle.

17. Verfahren nach Anspruch 15 zur gleichzeitigen Expression von 3 bis 8 Proteinen in einer Pflanzenzelle, umfassend die Schritte von:
a. Assemblieren eines Multigen-Expressionsvehikels (MGEV), wie nach Anspruch 8 definiert;
b. Kombinieren des MGEV mit einem Pflanzentransformationsvektor an einem Locus in dem Vektor, der sich unter Expressionskontrolle eines pflanzenaktiven Promotors und eines planzenaktiven Terminators befindet, wobei ein MGEV-Expressionsvektor bereitgestellt wird, und;
c. Transformieren einer Pflanzenzelle mit dem MGEV-Expressionsvektor, wobei eine MGEV-transformierte Zelle bereitgestellt wird, und;
d. Aufrechterhalten der MGEV-transformierten Zelle und Abkömmlingen davon unter Bedingungen, die zur Genexpression in der Zelle geeignet sind, wobei in MGEV transferierten Zellen kodierte Gene gleichzeitig exprimiert werden, und gegebenenfalls
Regenerieren einer adulten transgenen Pflanze aus der MGEV-transformierten Zelle.

18. Verfahren nach einem der Ansprüche 15 bis 17, wobei die adulte transformierte Pflanze ausgewählt ist aus der Gruppe von Pflanzen, die aus Baumwolle, Sojabohnen, Mais und Reis besteht.

19. Verfahren nach Anspruch 15 für die gleichzeitige Expression von zwei bis acht Proteinen in einer Pflanzenzelle, umfassend die Transformation einer Pflanzenzelle mit einem MGEV nach Anspruch 2, wobei sich das MGEV unter Expressionskontrolle eines einzelnen Promotors befindet.

## Revendications

1. Véhicule d'expression multigénique (MGEV) comprenant un polynucléotide comportant 2 à 8 segments de domaine, D, chaque domaine codant pour une protéine fonctionnelle, chaque domaine étant joint au suivant dans une séquence linéaire par un segment de lieur (L) codant pour un peptide lieur, les segments D et L étant tous dans le même cadre de lecture, où aucun des domaines n'est un inhibiteur de protéinase de type II, et où chaque lieur a la séquence de SEQ ID NO : 5.

2. MGEV selon la revendication 1, dans lequel chaque segment de domaine, D, code indépendamment pour une protéine fonctionnelle choisie parmi un inhibiteur de protéinase de type I de la pomme de terre, une défensine végétale, une défensine animale, une toxine protéinique, une protéine chimérique ou de fusion, une protéine indicateur, une protéine de choc thermique, un inhibiteur de Bowman-Birk de la trypsine, une cystatine, un peptide antimicrobien, une protéine fluorescente verte et un fragment d'anticorps.

3. MGEV selon la revendication 1 ou la revendication 2, comprenant en outre un segment codant pour un peptide signal (S) à l'extrémité N-terminale d'une protéine fonctionnelle, et en outre comprenant éventuellement un segment codant pour un peptide signal de ciblage des vacuoles V.

4. MGEV selon la revendication 1, dans lequel les segments codants D et L sont joints dans l'ordre de la traduction, désignés par (DₖLⱼ), où k est un nombre ordinal pour chaque domaine numéroté de 1 à K et K se situe dans la plage de 2 à 8, et j est un nombre ordinal pour chaque lieur numéroté de 1 à K-1.

5. MGEV selon la revendication 4, comprenant en outre un segment codant, S, codant pour un peptide signal combiné dans l'ordre S-Dₖ-Lⱼ.

6. MGEV selon la revendication 4, comprenant en outre un segment codant V codant pour un peptide de transport des vacuoles,
où V est combiné au segment codant de l'un quelconque de D₁-Dₖ, à l'une ou l'autre extrémité du segment codant pour l'un quelconque de D₁-Dₖ.

7. MGEV selon la revendication 1 ou la revendication 2, comprenant en outre un segment codant pour un peptide pince N-terminal, C2_{N}, et un segment codant pour un peptide pince C-terminal, C2_{C}, les peptides pinces N-terminal et C-terminal étant joints les uns aux autres par des liaisons disulfure après la traduction pour former une seule protéine possédant une activité d'inhibiteur de protéase.

8. MGEV selon la revendication 7, dans lequel les segments C2_{N}, D, L et C2_{C} sont joints dans l'ordre de la traduction, désignés par C2_{N}-Lⱼ-Dₖ₊₁-C2_{C}, où k est un nombre ordinal pour chaque domaine numéroté de 1 à K et K se situe dans la plage de 3 à 8, et j est un nombre ordinal pour chaque lieur, numéroté de 1 à K+1.

9. MGEV selon la revendication 8, comprenant en outre un segment codant, S, codant pour un peptide signal combiné dans l'ordre S-C2_{N}-Lⱼ-Dₖ-Lₖ₊₁-C2_{C}.

10. MGEV selon la revendication 8, comprenant en outre un segment codant V codant pour un peptide de transport des vacuoles, où V est combiné au segment codant de l'un quelconque de D₁-Dₖ₊₁, C2_{N} ou C2_{C}, à l'une ou l'autre extrémité du segment codant pour l'un quelconque de D₁-Dₖ₊₁, C2_{N} ou C2_{C} ; ou bien où V est combiné à l'un quelconque de Dₖ, à l'une ou l'autre extrémité du segment codant pour l'un quelconque de Dₖ, C2_{N} ou C2_{C}.

11. Vecteur d'expression de MGEV comprenant un vecteur de transformation végétale portant et répliquant un MGEV selon l'une quelconque des revendications 1 à 10, le MGEV étant inséré au niveau d'un locus dans le vecteur qui est sous le contrôle d'expression d'un promoteur actif chez les végétaux et d'un terminateur actif chez les végétaux.

12. Vecteur de transformation végétale comprenant un MGEV selon l'une quelconque des revendications 1 à 10 sous le contrôle d'expression d'un promoteur actif chez les végétaux.

13. Cellule végétale contenant le vecteur de transformation végétale selon la revendication 12, et exprimant des protéines codées par un MGEV selon l'une quelconque des revendications 1 à 10.

14. Plante transgénique, transformée par le vecteur de transformation végétale selon la revendication 12, et exprimant simultanément des protéines codées par un MGEV selon l'une quelconque des revendications 1 à 10.

15. Procédé d'expression simultanée de deux à huit protéines souhaitées dans une cellule végétale comprenant l'incorporation d'un MGEV tel que défini dans l'une quelconque des revendications 1 à 10 dans un vecteur de transformation végétale, la transformation dudit vecteur dans ladite cellule végétale et l'expression dudit MGEV dans ladite cellule végétale.

16. Procédé selon la revendication 15 d'expression simultanée de deux à huit protéines souhaitées dans une cellule végétale comprenant les étapes suivantes :
a. l'assemblage d'un véhicule d'expression multigénique (MGEV) tel que défini dans la revendication 4 ;
b. la combinaison du MGEV avec un vecteur de transformation végétale, au niveau d'un locus dans le vecteur qui est sous le contrôle d'expression d'un promoteur actif chez les végétaux et d'un terminateur actif chez les végétaux, fournissant de cette manière un vecteur d'expression de MGEV, et ;
c. la transformation d'une cellule végétale avec le vecteur d'expression de MGEV, fournissant de cette manière une cellule transformée par un MGEV, et ;
d. le maintien de la cellule transformée par un MGEV et de sa descendance dans des conditions appropriées pour l'expression génique au sein de la cellule, grâce à quoi des gènes codés au sein des cellules transformées par un MGEV sont exprimés simultanément ; et éventuellement
la régénération d'une plante transgénique adulte à partir de la cellule transformée par un MGEV.

17. Procédé selon la revendication 15 d'expression simultanée de 3 à 8 protéines dans une cellule végétale comprenant les étapes suivantes :
a. l'assemblage d'un véhicule d'expression multigénique (MGEV) tel que défini dans la revendication 8 ;
b. la combinaison du MGEV avec un vecteur de transformation végétale, au niveau d'un locus dans le vecteur qui est sous le contrôle d'expression d'un promoteur actif chez les végétaux et d'un terminateur actif chez les végétaux, fournissant de cette manière un vecteur d'expression de MGEV, et ;
c. la transformation d'une cellule végétale avec le vecteur d'expression de MGEV, fournissant de cette manière une cellule transformée par un MGEV, et ;
d. le maintien de la cellule transformée par un MGEV et de sa descendance dans des conditions appropriées pour l'expression génique au sein de la cellule, grâce à quoi des gènes codés au sein des cellules transformées par un MGEV sont exprimés simultanément ; et éventuellement
la régénération d'une plante transgénique adulte à partir de la cellule transformée par un MGEV.

18. Procédé selon l'une quelconque des revendications 15 à 17, dans lequel la plante transformée adulte est choisie dans le groupe de plantes constitué de coton, soja, maïs et riz.

19. Procédé selon la revendication 15 pour l'expression simultanée de deux à huit protéines dans une cellule végétale comprenant la transformation d'une cellule végétale avec un MGEV selon la revendication 2, dans lequel le MGEV est sous le contrôle d'expression d'un seul promoteur.


### SEQUENCE LISTING

<110> Hexima Limited
   Anderson, Marilyn
   Heath, Robyn
<120> MULTI-GENE EXPRESSION VEHICLE
<130> 30222500/EJH/AJH
<150> US 60803206
   <151> 2006-05-25
<160> 20
<170> PatentIn version 3.4
<210> 1
   <211> 397
   <212> PRT
   <213> Nicotiana alata
<400> 1
   Met Ala Val His Arg Val Ser Phe Leu Ala Leu Leu Leu Leu Phe Gly
   1               5                   10                  15
   Met Ser Leu Leu Val Ser Asn Val Glu His Ala Asp Ala Lys Ala Cys
               20                  25                  30
   Thr Leu Asn Cys Asp Pro Arg Ile Ala Tyr Gly Val Cys Pro Arg Ser
           35                  40                  45
   Glu Glu Lys Lys Asn Asp Arg Ile Cys Thr Asn Cys Cys Ala Gly Thr
       50                  55                  60
   Lys Gly Cys Lys Tyr Phe Ser Asp Asp Gly Thr Phe Val Cys Glu Gly
   65                  70                  75                  80
   Glu Ser Asp Pro Arg Asn Pro Lys Ala Cys Thr Leu Asn Cys Asp Pro
   85 90 95
   Arg Ile Ala Tyr Gly Val Cys Pro Arg Ser Glu Glu Lys Lys Asn Asp
   100 105 110
   Arg Ile Cys Thr Asn Cys Cys Ala Gly Thr Lys Gly Cys Lys Tyr Phe
   115 120 125
   Ser Asp Asp Gly Thr Phe Val Cys Glu Gly Glu Ser Asp Pro Arg Asn
   130 135 140
   Pro Lys Ala Cys Pro Arg Asn Cys Asp Pro Arg Ile Ala Tyr Gly Ile
   145 150 155 160
   Cys Pro Leu Ala Glu Glu Lys Lys Asn Asp Arg Ile Cys Thr Asn Cys
   165 170 175
   Cys Ala Gly Lys Lys Gly Cys Lys Tyr Phe Ser Asp Asp Gly Thr Phe
   180 185 190
   Val Cys Glu Gly Glu Ser Asp Pro Lys Asn Pro Lys Ala Cys Pro Arg
   195 200 205
   Asn Cys Asp Gly Arg Ile Ala Tyr Gly Ile Cys Pro Leu Ser Glu Glu
   210 215 220
   Lys Lys Asn Asp Arg Ile Cys Thr Asn Cys Cys Ala Gly Lys Lys Gly
   225 230 235 240
   Cys Lys Tyr Phe Ser Asp Asp Gly Thr Phe Val Cys Glu Gly Glu Ser
   245 250 255
   Asp Pro Lys Asn Pro Lys Ala Cys Pro Arg Asn Cys Asp Gly Arg Ile
   260 265 270
   Ala Tyr Gly Ile Cys Pro Leu Ser Glu Glu Lys Lys Asn Asp Arg Ile
   275 280 285
   Cys Thr Asn Cys Cys Ala Gly Lys Lys Gly Cys Lys Tyr Phe Ser Asp
   290 295 300
   Asp Gly Thr Phe Val Cys Glu Gly Glu Ser Asp Pro Arg Asn Pro Lys
   305 310 315 320
   Ala Cys Pro Arg Asn Cys Asp Gly Arg Ile Ala Tyr Gly Ile Cys Pro
   325 330 335
   Leu Ser Glu Glu Lys Lys Asn Asp Arg Ile Cys Thr Asn Cys Cys Ala
   340 345 350
   Gly Lys Lys Gly Cys Lys Tyr Phe Ser Asp Asp Gly Thr Phe Ile Cys
   355 360 365
   Glu Gly Glu Ser Glu Tyr Ala Ser Lys Val Asp Glu Tyr Val Gly Glu
   370 375 380
   Val Glu Asn Asp Leu Gln Lys Ser Lys Val Ala Val Ser
   385 390 395
<210> 2
   <211> 281
   <212> PRT
   <213> Nicotiana alata
<400> 2
   Met Ala Ala His Arg Val Ser Phe Leu Ala Leu Leu Leu Leu Phe Gly
   1 5 10 15
   Met Ser Leu Leu Val Ser Asn Val Glu His Ala Asp Ala Lys Ala Cys
   20 25 30
   Thr Leu Asn Cys Asp Pro Arg Ile Ala Tyr Gly Val Cys Pro Arg Ser
   35 40 45
   Glu Glu Lys Lys Asn Asp Arg Ile Cys Thr Asn Cys Cys Ala Gly Thr
   50 55 60
   Lys Gly Cys Lys Tyr Phe Ser Asp Asp Gly Thr Phe Val Cys Glu Gly
   65 70 75 80
   Glu Ser Asp Pro Arg Asn Pro Lys Ala Cys Thr Leu Asn Cys Asp Pro
   85 90 95
   Arg Ile Ala Tyr Gly Val Cys Pro Arg Ser Glu Glu Lys Lys Asn Asp
   100 105 110
   Arg Ile Cys Thr Asn Cys Cys Ala Gly Thr Lys Gly Cys Lys Tyr Phe
   115 120 125
   Ser Asp Asp Gly Thr Phe Val Cys Glu Gly Glu Ser Asp Pro Lys Asn
   130 135 140
   Pro Lys Ala Cys Pro Arg Asn Cys Asp Pro Arg Ile Ala Tyr Gly Ile
   145 150 155 160
   Cys Pro Leu Ser Glu Glu Lys Lys Asn Asp Arg Ile Cys Thr Asn Cys
   165 170 175
   Cys Ala Gly Lys Lys Gly Cys Lys Tyr Phe Ser Asp Asp Gly Thr Phe
   180 185 190
   Val Cys Glu Gly Glu Ser Asp Pro Arg Asn Pro Lys Ala Cys Pro Arg
   195 200 205
   Asn Cys Asp Gly Arg Ile Ala Tyr Gly Ile Cys Pro Leu Ser Glu Glu
   210 215 220
   Lys Lys Asn Asp Arg Ile Cys Thr Asn Cys Cys Ala Gly Lys Lys Gly
   225 230 235 240
   Cys Lys Tyr Phe Ser Asp Asp Gly Thr Phe Ile Cys Glu Gly Glu Ser
   245 250 255
   Glu Tyr Ala Ser Lys Val Asp Glu Tyr Val Gly Glu Val Glu Asn Asp
   260 265 270
   Leu Gln Lys Ser Lys Val Ala Val Ser
   275 280
<210> 3
   <211> 53
   <212> PRT
   <213> Artificial
<220>
   <223> Synthetic construct: T1 peptide sequence.
<400> 3
   Asp Arg Ile Cys Thr Asn Cys Cys Ala Gly Thr Lys Gly Cys Lys Tyr
   1 5 10 15
   Phe Ser Asp Asp Gly Thr Phe Val Cys Glu Gly Glu Ser Asp Pro Arg
   20 25 30
   Asn Pro Lys Ala Cys Pro Arg Asn Cys Asp Pro Arg Ile Ala Tyr Gly
   35 40 45
   Ile Cys Pro Leu Ala
   50
<210> 4
   <211> 53
   <212> PRT
   <213> Artificial
<220>
   <223> Synthetic construct: T5 peptide sequence.
<400> 4
   Asp Arg Ile Cys Thr Asn Cys Cys Ala Gly Thr Lys Gly Cys Lys Tyr
   1 5 10 15
   Phe Ser Asp Asp Gly Thr Phe Val Cys Glu Gly Glu Ser Asp Pro Lys
   20 25 30
   Asn Pro Lys Ala Cys Pro Arg Asn Cys Asp Pro Arg Ile Ala Tyr Gly
   35 40 45
   Ile Cys Pro Leu Ser
   50
<210> 5
   <211> 5
   <212> PRT
   <213> Artificial
<220>
   <223> Synthetic construct: linker peptide sequence.
<400> 5
   Glu Glu Lys Lys Asn
   1 5
<210> 6
   <211> 948
   <212> DNA
   <213> Artificial
<220>
   <223> Synthetic construct: MGEV-5 sequence
<400> 6
   ggatccatgg ctgctcacag agttagtttc cttgctctcc tcctcttatt tggaatgtct 60
   ctgcttgtaa gcaatgtgga acatgcagat gccaaggctt gtaccttaaa ctgtgatcca 120
   agaattgcct atggagtttg cccgcgttca gaagaaaaga agaatctcga ggatcggata 180
   tgcaccaact gttgtgcagg cacgaagggt tgtaagtact tcagtgatga tggaactttt 240
   gtttgtgaag gagagtctga tcctagaaat ccaaaggctt gtcctcggaa ttgcgatcca 300
   agaattgcct atgggatttg cccactttca gaagaaaaga agaattctag aaaggaatcg 360
   gaatctgaat cttggtgcaa aggaaaacaa ttctggccag aacttattgg tgtaccaaca 420
   aagcttgcta aggaaataat tgagaaggaa aatccatcca taaatgatgt tccaataata 480
   ttgaatggca ctccagtccc agctgatttt agatgtaatc gagttcgtct tttgataaac 540
   attttgggtg atgttgtaca aattcctagg gtggctgaag aaaagaagaa ttctagagat 600
   cggatatgca ccaactgttg cgcaggcacg aagggttgta agtacttcag tgatgatgga 660
   acttttgttt gtgaaggaga gtctgatcct agaaatccaa aggcttgtac cttaaactgt 720
   gatccaagaa ttgcctatgg agtttgcccg cgttcagaag aaaagaagaa tctcgaggat 780
   cggatatgca ccaattgttg cgcaggcaag aagggctgta agtactttag tgatgatgga 840
   acttttattt gtgaaggaga atctgaatat gccagcaaag tggatgaata tgttggtgaa 900
   gtggagaatg atctccagaa gtccaaggtt gctgtttcct aagtcgac 948
<210> 7
   <211> 24
   <212> DNA
   <213> Artificial
<220>
   <223> Synthetic construct: oligonucleotide useful as a primer.
<400> 7
   gtggagaggc tattcggcta tgac 24
<210> 8
   <211> 21
   <212> DNA
   <213> Artificial
<220>
   <223> Synthetic construct: oligonucleotide useful as a primer.
<400> 8
   cgggtagcca acgctatgtc c 21
<210> 9
   <211> 28
   <212> DNA
   <213> Artificial
<220>
   <223> Synthetic construct: oligonucleotide useful as a primer.
<400> 9
   gctctagaaa ggaatcggaa tctgaatc 28
<210> 10
   <211> 41
   <212> DNA
   <213> Artificial
<220>
   <223> Synthetic construct: oligonucleotide useful as a primer.
<400> 10
   gctctagaat tcttcttttc ttcagccacc ctaggaattt g 41
<210> 11
   <211> 67
   <212> PRT
   <213> Solanum tuberosum
<400> 11
   Lys Glu Ser Glu Ser Glu Ser Trp Cys Lys Gly Lys Gln Phe Trp Pro
   1 5 10 15
   Glu Leu Gly Val Pro Thr Lys Leu Ala Lys Glu Glu Lys Glu Asn Pro
   20 25 30
   Ser Asn Asp Val Pro Leu Asn Gly Thr Pro Val Pro Ala Asp Phe Arg
   35 40 45
   Cys Asn Arg Val Arg Leu Phe Asp Asn Leu Gly Asp Val Val Gln Pro
   50 55 60
   Arg Val Ala
   65
<210> 12
   <211> 313
   <212> PRT
   <213> Artificial
<220>
   <223> Synthetic construct: sequence of MGEV-5
<400> 12
   Gly Ser Met Ala Ala His Arg Val Ser Phe Leu Ala Leu Leu Leu Leu
   1 5 10 15
   Phe Gly Met Ser Leu Leu Val Ser Asn Val Glu His Ala Asp Ala Lys
   20 25 30
   Ala Cys Thr Leu Asn Cys Asp Pro Arg Ile Ala Tyr Gly Val Cys Pro
   35 40 45
   Arg Ser Glu Glu Lys Lys Asn Leu Glu Asp Arg Ile Cys Thr Asn Cys
   50 55 60
   Cys Ala Gly Thr Lys Gly Cys Lys Tyr Phe Ser Asp Asp Gly Thr Phe
   65 70 75 80
   Val Cys Glu Gly Glu Ser Asp Pro Arg Asn Pro Lys Ala Cys Pro Arg
   85 90 95
   Asn Cys Asp Pro Arg Ile Ala Tyr Gly Ile Cys Pro Leu Ser Glu Glu
   100 105 110
   Lys Lys Asn Ser Arg Lys Glu Ser Glu Ser Glu Ser Trp Cys Lys Gly
   115 120 125
   Lys Gln Phe Trp Pro Glu Leu Ile Gly Val Pro Thr Lys Leu Ala Lys
   130 135 140
   Glu Ile Ile Glu Lys Glu Asn Pro Ser Ile Asn Asp Val Pro Ile Ile
   145 150 155 160
   Leu Asn Gly Thr Pro Val Pro Ala Asp Phe Arg Cys Asn Arg Val Arg
   165 170 175
   Leu Phe Asp Asn Ile Leu Gly Asp Val Val Gln Ile Pro Arg Val Ala
   180 185 190
   Glu Glu Lys Lys Asn Ser Arg Asp Arg Ile Cys Thr Asn Cys Cys Ala
   195 200 205
   Gly Thr Lys Gly Cys Lys Tyr Phe Ser Asp Asp Gly Thr Phe Val Cys
   210 215 220
   Glu Gly Glu Ser Asp Pro Arg Asn Pro Lys Ala Cys Thr Leu Asn Cys
   225 230 235 240
   Asp Pro Arg Ile Ala Tyr Gly Val Cys Pro Arg Ser Glu Glu Lys Lys
   245 250 255
   Asn Leu Glu Asp Arg Ile Cys Thr Asn Cys Cys Ala Gly Lys Lys Gly
   260 265 270
   Cys Lys Tyr Phe Ser Asp Asp Gly Thr Phe Ile Cys Glu Gly Glu Ser
   275 280 285
   Glu Tyr Ala Ser Lys Val Asp Glu Tyr Val Gly Glu Val Glu Asn Asp
   290 295 300
   Leu Gln Lys Ser Lys Val Ala Val Ser
   305 310
<210> 13
   <211> 238
   <212> PRT
   <213> Artificial
<220>
   <223> Synthetic construct: soluble, modified green fluorescent protein.
<400> 13
   Met Ser Lys Gly Glu Glu Leu Phe Thr Gly Val Val Pro Ile Leu Val
   1 5 10 15
   Glu Leu Asp Gly Asp Val Asn Gly His Lys Phe Ser Val Ser Gly Glu
   20 25 30
   Gly Glu Gly Asp Ala Thr Tyr Gly Lys Leu Thr Leu Lys Phe Ile Cys
   35 40 45
   Thr Thr Gly Lys Leu Pro Val Pro Trp Pro Thr Leu Val Thr Thr Phe
   50 55 60
   Ser Tyr Gly Val Gln Cys Phe Ser Arg Tyr Pro Asp His Met Lys Arg
   65 70 75 80
   His Asp Phe Phe Lys Ser Ala Met Pro Glu Gly Tyr Val Gln Glu Arg
   85 90 95
   Thr Ile Ser Phe Lys Asp Asp Gly Asn Tyr Lys Thr Arg Ala Glu Val
   100 105 110
   Lys Phe Glu Gly Asp Thr Leu Val Asn Arg Ile Glu Leu Lys Gly Ile
   115 120 125
   Asp Phe Lys Glu Asp Gly Asn Ile Leu Gly His Lys Leu Glu Tyr Asn
   130 135 140
   Tyr Asn Ser His Asn Val Tyr Ile Thr Ala Asp Lys Gln Lys Asn Gly
   145 150 155 160
   Ile Lys Ala Asn Phe Lys Ile Arg His Asn Ile Glu Asp Gly Ser Val
   165 170 175
   Gln Leu Ala Asp His Tyr Gln Gln Asn Thr Pro Ile Gly Asp Gly Pro
   180 185 190
   Val Leu Leu Pro Asp Asn His Tyr Leu Ser Thr Gln Ser Ala Leu Ser
   195 200 205
   Lys Asp Pro Asn Glu Lys Arg Asp His Met Val Leu Leu Glu Phe Val
   210 215 220
   Thr Ala Ala Gly Ile Thr His Gly Met Asp Glu Leu Tyr Lys
   225 230 235
<210> 14
   <211> 105
   <212> PRT
   <213> Nicotiana alata
<400> 14
   Met Ala Arg Ser Leu Cys Phe Met Ala Phe Ala Ile Leu Ala Met Met
   1 5 10 15
   Leu Phe Val Ala Tyr Glu Val Gln Ala Arg Glu Cys Lys Thr Glu Ser
   20 25 30
   Asn Thr Phe Pro Gly Ile Cys Ile Thr Lys Pro Pro Cys Arg Lys Ala
   35 40 45
   Cys Ile Ser Glu Lys Phe Thr Asp Gly His Cys Ser Lys Ile Leu Arg
   50 55 60
   Arg Cys Leu Cys Thr Lys Pro Cys Val Phe Asp Glu Lys Met Thr Lys
   65 70 75 80
   Thr Gly Ala Glu Ile Leu Ala Glu Glu Ala Lys Thr Leu Ala Ala Ala
   85 90 95
   Leu Leu Glu Glu Glu Ile Met Asp Asn
   100 105
<210> 15
   <211> 237
   <212> DNA
   <213> Nicotiana alata
<220>
   <221> CDS
   <222> (1)..(234)
<400> 15
   atg gca aac tcc atg cgc ttc ttt gct act gtg tta ctt cta aca ttg        48
   Met Ala Asn Ser Met Arg Phe Phe Ala Thr Val Leu Leu Leu Thr Leu
   1               5                   10                  15
   ctt ttc atg gct aca gag atg gga cca atg aca att gca gag gca aga        96
   Leu Phe Met Ala Thr Glu Met Gly Pro Met Thr Ile Ala Glu Ala Arg
               20                  25                  30
   act tgc gag tct cag agc cac cgt ttc aag gga cca tgc gca aga gat       144
   Thr Cys Glu Ser Gln Ser His Arg Phe Lys Gly Pro Cys Ala Arg Asp
           35                  40                  45
   agc aac tgt gcc acc gtc tgt ttg aca gaa gga ttt tcc ggt ggc gac       192
   Ser Asn Cys Ala Thr Val Cys Leu Thr Glu Gly Phe Ser Gly Gly Asp
       50                  55                  60
   tgc cgt gga ttc cgc cgc cgt tgt ttc tgt acc agc cct tgc taa           237
   Cys Arg Gly Phe Arg Arg Arg Cys Phe Cys Thr Ser Pro Cys
   65                  70                  75
<210> 16
   <211> 78
   <212> PRT
   <213> Nicotiana alata
<400> 16
   Met Ala Asn Ser Met Arg Phe Phe Ala Thr Val Leu Leu Leu Thr Leu
   1               5                   10                  15
   Leu Phe Met Ala Thr Glu Met Gly Pro Met Thr Ile Ala Glu Ala Arg
               20                  25                  30
   Thr Cys Glu Ser Gln Ser His Arg Phe Lys Gly Pro Cys Ala Arg Asp
           35                  40                  45
   Ser Asn Cys Ala Thr Val Cys Leu Thr Glu Gly Phe Ser Gly Gly Asp
       50                  55                  60
   Cys Arg Gly Phe Arg Arg Arg Cys Phe Cys Thr Ser Pro Cys
   65                  70                  75
<210> 17
   <211> 5
   <212> PRT
   <213> Artificial
<220>
   <223> Synthetic construct: consensus sequence for linker peptide in MGEV polypeptide.
<220>
   <221> MISC_FEATURE
   <222> (1) .. (2)
   <223> At positions 1 and 2, Xaa can be Glutamate or Aspartate.
<220>
   <221> MISC_FEATURE
   <222> (3) .. (4)
   <223> At positions 3 and 4, Xaa can be Lysine or Arginine.
<220>
   <221> MISC_FEATURE
   <222> (5) .. (5)
   <223> At position 5, xaa can be Asparagine or Glutamine..
<400> 17
   Xaa Xaa Xaa Xaa Xaa
   1               5
<210> 18
   <211> 1812
   <212> DNA
   <213> Escherichia coli
<400> 18
   atgttacgtc ctgtagaaac cccaacccgt gaaatcaaaa aactcgacgg cctgtgggca      60
   ttcagtctgg atcgcgaaaa ctgtggaatt gatcagcgtt ggtgggaaag cgcgttacaa     120
   gaaagccggg caattgctgt gccaggcagt tttaacgatc agttcgccga tgcagatatt     180
   cgtaattatg cgggcaacgt ctggtatcag cgcgaagtct ttataccgaa aggttgggca     240
   ggccagcgta tcgtgctgcg tttcgatgcg gtcactcatt acggcaaagt gtgggtcaat     300
   aatcaggaag tgatggagca tcagggcggc tatacgccat ttgaagccga tgtcacgccg     360
   tatgttattg ccgggaaaag tgtacgtatc accgtttgtg tgaacaacga actgaactgg     420
   cagactatcc cgccgggaat ggtgattacc gacgaaaacg gcaagaaaaa gcagtcttac     480
   ttccatgatt tctttaacta tgccggaatc catcgcagcg taatgctcta caccacgccg     540
   aacacctggg tggacgatat caccgtggtg acgcatgtcg cgcaagactg taaccacgcg     600
   tctgttgact ggcaggtggt ggccaatggt gatgtcagcg ttgaactgcg tgatgcggat     660
   caacaggtgg ttgcaactgg acaaggcact agcgggactt tgcaagtggt gaatccgcac     720
   ctctggcaac cgggtgaagg ttatctctat gaactgtgcg tcacagccaa aagccagaca 780
   gagtgtgata tctacccgct tcgcgtcggc atccggtcag tggcagtgaa gggcgaacag 840
   ttcctgatta accacaaacc gttctacttt actggctttg gtcgtcatga agatgcggac 900
   ttgcgtggca aaggattcga taacgtgctg atggtgcacg accacgcatt aatggactgg 960
   attggggcca actcctaccg tacctcgcat tacccttacg ctgaagagat gctcgactgg 1020
   gcagatgaac atggcatcgt ggtgattgat gaaactgctg ctgtcggctt taacctctct 1080
   ttaggcattg gtttcgaagc gggcaacaag ccgaaagaac tgtacagcga agaggcagtc 1140
   aacggggaaa ctcagcaagc gcacttacag gcgattaaag agctgatagc gcgtgacaaa 1200
   aaccacccaa gcgtggtgat gtggagtatt gccaacgaac cggatacccg tccgcaaggt 1260
   gcacgggaat atttcgcgcc actggcggaa gcaacgcgta aactcgaccc gacgcgtccg 1320
   atcacctgcg tcaatgtaat gttctgcgac gctcacaccg ataccatcag cgatctcttt 1380
   gatgtgctgt gcctgaaccg ttattacgga tggtatgtcc aaagcggcga tttggaaacg 1440
   gcagagaagg tactggaaaa agaacttctg gcctggcagg agaaactgca tcagccgatt 1500
   atcatcaccg aatacggcgt ggatacgtta gccgggctgc actcaatgta caccgacatg 1560
   tggagtgaag agtatcagtg tgcatggctg gatatgtatc accgcgtctt tgatcgcgtc 1620
   agcgccgtcg tcggtgaaca ggtatggaat ttcgccgatt ttgcgacctc gcaaggcata 1680
   ttgcgcgttg gcggtaacaa gaaagggatc ttcactcgcg accgcaaacc gaagtcggcg 1740
   gcttttctgc tgcaaaaacg ctggactggc atgaacttcg gtgaaaaacc gcagcaggga 1800
   ggcaaacaat ga 1812
<210> 19
   <211> 603
   <212> PRT
   <213> Escherichia coli
<400> 19
   Met Leu Arg Pro Val Glu Thr Pro Thr Arg Glu Ile Lys Lys Leu Asp
   1 5 10 15
   Gly Leu Trp Ala Phe Ser Leu Asp Arg Glu Asn Cys Gly Ile Asp Gln
   20 25 30
   Arg Trp Trp Glu Ser Ala Leu Gln Glu Ser Arg Ala Ile Ala Val Pro
   35 40 45
   Gly Ser Phe Asn Asp Gln Phe Ala Asp Ala Asp Ile Arg Asn Tyr Ala
   50 55 60
   Gly Asn Val Trp Tyr Gln Arg Glu Val Phe Ile Pro Lys Gly Trp Ala
   65 70 75 80
   Gly Gln Arg Ile Val Leu Arg Phe Asp Ala Val Thr His Tyr Gly Lys
   85 90 95
   Val Trp Val Asn Asn Gln Glu Val Met Glu His Gln Gly Gly Tyr Thr
   100 105 110
   Pro Phe Glu Ala Asp Val Thr Pro Tyr Val Ile Ala Gly Lys Ser Val
   115 120 125
   Arg Ile Thr Val Cys Val Asn Asn Glu Leu Asn Trp Gln Thr Ile Pro
   130 135 140
   Pro Gly Met Val Ile Thr Asp Glu Asn Gly Lys Lys Lys Gln Ser Tyr
   145 150 155 160
   Phe His Asp Phe Phe Asn Tyr Ala Gly Ile His Arg Ser Val Met Leu
   165 170 175
   Tyr Thr Thr Pro Asn Thr Trp Val Asp Asp Ile Thr Val Val Thr His
   180 185 190
   Val Ala Gln Asp Cys Asn His Ala Ser Val Asp Trp Gln Val Val Ala
   195 200 205
   Asn Gly Asp Val Ser Val Glu Leu Arg Asp Ala Asp Gln Gln Val Val
   210 215 220
   Ala Thr Gly Gln Gly Thr Ser Gly Thr Leu Gln Val Val Asn Pro His
   225 230 235 240
   Leu Trp Gln Pro Gly Glu Gly Tyr Leu Tyr Glu Leu Cys Val Thr Ala
   245 250 255
   Lys Ser Gln Thr Glu Cys Asp Ile Tyr Pro Leu Arg Val Gly Ile Arg
   260 265 270
   Ser Val Ala Val Lys Gly Glu Gln Phe Leu Ile Asn His Lys Pro Phe
   275 280 285
   Tyr Phe Thr Gly Phe Gly Arg His Glu Asp Ala Asp Leu Arg Gly Lys
   290 295 300
   Gly Phe Asp Asn Val Leu Met Val His Asp His Ala Leu Met Asp Trp
   305 310 315 320
   Ile Gly Ala Asn Ser Tyr Arg Thr Ser His Tyr Pro Tyr Ala Glu Glu
   325 330 335
   Met Leu Asp Trp Ala Asp Glu His Gly Ile Val Val Ile Asp Glu Thr
   340 345 350
   Ala Ala Val Gly Phe Asn Leu Ser Leu Gly Ile Gly Phe Glu Ala Gly
   355 360 365
   Asn Lys Pro Lys Glu Leu Tyr Ser Glu Glu Ala Val Asn Gly Glu Thr
   370 375 380
   Gln Gln Ala His Leu Gln Ala Ile Lys Glu Leu Ile Ala Arg Asp Lys
   385 390 395 400
   Asn His Pro Ser Val Val Met Trp Ser Ile Ala Asn Glu Pro Asp Thr
   405 410 415
   Arg Pro Gln Gly Ala Arg Glu Tyr Phe Ala Pro Leu Ala Glu Ala Thr
   420 425 430
   Arg Lys Leu Asp Pro Thr Arg Pro Ile Thr Cys Val Asn Val Met Phe
   435 440 445
   Cys Asp Ala His Thr Asp Thr Ile Ser Asp Leu Phe Asp Val Leu Cys
   450 455 460
   Leu Asn Arg Tyr Tyr Gly Trp Tyr Val Gln Ser Gly Asp Leu Glu Thr
   465 470 475 480
   Ala Glu Lys Val Leu Glu Lys Glu Leu Leu Ala Trp Gln Glu Lys Leu
   485 490 495
   His Gln Pro Ile Ile Ile Thr Glu Tyr Gly Val Asp Thr Leu Ala Gly
   500 505 510
   Leu His Ser Met Tyr Thr Asp Met Trp Ser Glu Glu Tyr Gln Cys Ala
   515 520 525
   Trp Leu Asp Met Tyr His Arg Val Phe Asp Arg Val Ser Ala Val Val
   530 535 540
   Gly Glu Gln Val Trp Asn Phe Ala Asp Phe Ala Thr Ser Gln Gly Ile
   545 550 555 560
   Leu Arg Val Gly Gly Asn Lys Lys Gly Ile Phe Thr Arg Asp Arg Lys
   565 570 575
   Pro Lys Ser Ala Ala Phe Leu Leu Gln Lys Arg Trp Thr Gly Met Asn
   580 585 590
   Phe Gly Glu Lys Pro Gln Gln Gly Gly Lys Gln
   595 600
<210> 20
   <211> 36
   <212> PRT
   <213> Solanum tuberosum
<400> 20
   Met Glu Ser Lys Phe Ala His Ile Ile Val Phe Phe Leu Leu Ala Thr
   1 5 10 15
   Ser Phe Glu Thr Leu Met Ala Arg Lys Glu Gly Asp Gly Ser Glu Val
   20 25 30
   Ile Lys Leu Leu
   35